# EUROPEAN PATENT SPECIFICATION

(11) **EP 3 886 852 B1**
(45) Date of publication and mention of the grant of the patent: **31.01.2024**
(21) Application number: 18942199.3
(22) Date of filing: 03.12.2018
(51) Int. Cl.: A61K 31/216, A61K 31/192, A61K 31/41, A61K 31/7048, A61P 27/02, A61K 31/59, A61K 31/439, A61K 31/56

(54) **OCTYL GALLATE AND ESTERS THEREOF OF FOR USE IN THE TREATMENT AND PREVENTION OF AGE-RELATED MACULAR DEGENERATION CAUSED BY BACILLUS MEGATERIUM**
OCTYLGALLAT UND ESTER DAVON ZUR VERWENDUNG BEI DER BEHANDLUNG UND PRÄVENTION VON DURCH BACILLUS MEGATERIUM VERURSACHTER ALTERSBEDINGTER MAKULADEGENERATION
GALLATE D'OCTYLE ET ESTERS DE CELUI-CI DESTINÉS À ÊTRE UTILISÉS DANS LE TRAITEMENT ET LA PRÉVENTION DE LA DÉGÉNÉRESCENCE MACULAIRE LIÉE À L'ÂGE PROVOQUÉE PAR BACILLUS MEGATERIUM

(43) Date of publication of application: 06.10.2021
(73) Proprietor: Smilebiotek Zhuhai Limited, Guangdong Province (CN)
(72) Inventor: WEI, Lai, Zhuhai, Guangdong 519000 (CN); OUYANG, Hui, Zhuhai, Guangdong 519000 (CN); ZHANG, Yan, Zhuhai, Guangdong 519000 (CN)
(74) Representative: Murgitroyd & Company
(86) International application number: PCT/CN2018/118929
(87) International publication number: WO 2020/113373

(56) References cited:
- WO-A1-2016/090005
- WO-A1-2020/098630
- WO-A2-2007/029008
- CN-A- 1 436 074
- CN-A- 1 731 997
- CN-A- 101 137 370
- CN-A- 102 558 178
- CN-A- 108 349 867
- CN-A- 109 706 238
- JP-A- 2014 094 917
- JP-B2- 6 036 193
- KR-A- 20120 041 390
- KR-B1- 101 706 389
- US-A1- 2009 082 470
- US-A1- 2014 328 951
- US-B1- 6 218 368
- XIAOFENG WEN ET AL: "Epigenetics, microbiota, and intraocular inflammation: New paradigms of immune regulation in the eye", PROGRESS IN RETINAL AND EYE RESEARCH, vol. 64, 1 May 2018 (2018-05-01), pages 84-95, XP055707633, GB ISSN: 1350-9462, DOI: 10.1016/j.preteyeres.2018.01.001
- KANG JUNG-HWAN ET AL: "Protective effects of resveratrol and its analogs on age-related macular degeneration in vitro", ARCHIVES OF PHARMACAL RESEARCH, NATL. FISHERIES UNIVERSITY , PUSAN, KR, vol. 39, no. 12, 22 September 2016 (2016-09-22), pages 1703-1715, XP036121196, ISSN: 0253-6269, DOI: 10.1007/S12272-016-0839-0 [retrieved on 2016-09-22]
- Yu Feifei; Sun He: "Recent research status of traditional Chinese medicine on age-related macular degeneration", National Medical Frontiers of China, Chinese Hospital Association, vol. 3, no. 16, 31 August 2008 (2008-08-31), pages 13-14,41, XP009521638, CN ISSN: 1673-5552

## Description

### BACKGROUND OF THE INVENTION

### Field of the Invention

In various embodiments, the present disclosure generally relates to
a compound or a pharmaceutically acceptable salt or ester thereof, or a pharmaceutical compo sition comprising the compound or pharmaceutically acceptable salt or ester thereof for the use in treating or preventing age-related macular degeneration (AMD).

### Background Art

In the elderly population, age-related macular degeneration (AMD) is the leading cause of irreversible vision loss worldwide. It is characterized by confluent soft drusen deposited between retinal pigment epithelium (RPE) and the Bruch's membrane and/or retinal pigmentary changes in the macula at the early stage (intermediate AMD). At later stages, advanced AMD is characterized by two major subtypes, geographic atrophy (dry AMD) or choroidal neovascularization (wet AMD) in the macula. While anti-VEGF therapies have been used to control wet AMD, currently there is no approved therapy for dry AMD.

Xiaofeng Wen ET AL: "Epigenetics, microbiota, and intraocular inflammation: New paradigms of immune regulation in the eye",PROGRESS IN RETINAL AND EYE RESEARCH, vol. 64, 1 May 2018 (2018-05-01), pages 84-95 reveals that dysbiosis of human microbiota leads to rapid epigenomic reprogramming of host cells and results in the onset of many diseases.

WO2019/080916 discloses the methods of treatment of AMD by administration of an agents that kills or inhibits Bacillus megaterium.

WO2017/031161 reveals rhein and diacerein as NLPR3 inhibitors for use in the treatment of inflammasome-related diseases such as AMD.

None of the available prior artteaches that octyl gallate may be useful in the treatment of AMD caused by an infection by *Bacillus megaterium.*

The pathogenesis of AMD involves both genetic and environmental factors. Numerous studies have identified variations at the loci of genes that are associated with AMD susceptibility, including complement factor H (*CFH*), age-related maculopathy susceptibility *2 (ARMS2),* HtrA serine peptidase 1 (*HTRA1*), indicating that AMD is possible an inflammatory disease. Currently, the environmental factors triggering the local inflammation and leading to the early soft drusen in AMD pathology are not clear.

### BRIEF SUMMARY OF THE INVENTION

The invention is as defined in claim 1.

In various embodiments, the present disclosure is based in part on the unexpected discovery that the intraocular environment is not sterile and certain intraocular microbiota such as *Bacillus megaterium* can be a pathogenic cause of AMD.

Accordingly, in some embodiments, the present disclosure is directed to various compounds and/or compositions comprising the compounds that can kill or inhibit the growth of microorganisms related to AMD, such as *Bacillus megaterium.* In some embodiments, the present disclosure provides a compound according to any of Formula I, II, III, IV-1, IV-2, V, and any of the sub-formulae thereof, as defined herein, or a pharmaceutically acceptable salt or ester thereof. In some embodiments, the present disclosure provides a compound according to any of compounds 1-13, or a pharmaceutically acceptable salt or ester thereof. In some embodiments, the compounds of the present disclosure can be derived from synthetic sources. In some embodiments, the compounds of the present disclosure can be an isolated compound or a substantially pure compound.

Certain embodiments are directed to a pharmaceutical composition comprising one or more of the compounds of the present disclosure, and optionally a pharmaceutically acceptable excipient. For example, in some embodiments, the pharmaceutical composition comprises a compound of Formula I, II, III, IV-1, IV-2, V, any sub-formulae thereof, or any one or more of compounds 1-13, or a pharmaceutically acceptable salt or ester thereof, for example, in an amount effective to kill or inhibit the growth of a microorganism herein, such as *B. megaterium,* for example, in the eye (e.g., intraocular space), blood, and/or GI tract, such as intestine of the subject. The pharmaceutical composition described herein can be formulated for delivery via any of the known routes of delivery, such as for oral, topical, intravitreous, intramuscular, subcutaneous, or intravenous administration. In some embodiments, the pharmaceutical composition described herein can further include an antibiotic and/or an anti-VEGF medication, e.g., as described herein.

In various embodiments, the present disclosure also provides a method of using the compounds of the present disclosure or the pharmaceutical compositions herein for treating infections (e.g., ocular infections, such as in the intraocular space) with a microorganism herein, such as *Bacillus megaterium,* and for treating or preventing diseases or disorders associated with such infections, such as AMD.

In some embodiments, the present disclosure provides a method for killing or inhibiting the growth of a microorganism herein, such as *Bacillus megaterium,* in a subject in need thereof. In some embodiments, the method comprises administering to the subject a therapeutically effective amount of a compound of the present disclosure (e.g., compound of Formula I, II, III, IV-1, IV-2, V, any sub-formulae thereof, or any one or more of compounds 1-13, or a pharmaceutically acceptable salt or ester thereof, or a pharmaceutical composition herein. In some embodiments, the subject suffers from AMD. In some embodiments, the subject does not suffer from AMD. In some embodiments, the subject is at risk of developing AMD. In some embodiments, the subject has ocular infection with the microorganism, such as *Bacillus megaterium.* In some embodiments, the method further comprises identifying, or having identified, the subject as being infected with, e.g., in the intraocular space, the microorganism, such as *Bacillus megaterium.* In some embodiments, the subject is further administered an antibiotic and/or an anti-VEGF medication, e.g., as described herein.

In some embodiments, the present disclosure provides a method of treating or preventing AMD in a subject in need thereof. In some embodiments, the method comprises administering to the subject a therapeutically effective amount of a compound of the present disclosure (e.g., a compound of Formula I, II, III, IV-1, IV-2, V, any sub-formulae thereof, or any one or more of compounds 1-13, or a pharmaceutically acceptable salt or ester thereof). In some embodiments, the method further comprises administering to the subject an antibiotic and/or an anti-VEGF medication, e.g., as described herein. In some embodiments, the AMD can be dry or wet age-related macular degeneration with drusen symptoms, including a hard drusen, a soft drusen, a mixed drusen and/or a degraded drusen, for example, dry or wet age-related macular degeneration with soft drusen symptoms. In some embodiments, the method further comprises identifying, or having identified, the subject as being infected with, e.g., in the intraocular space, a microorganism herein, such as *Bacillus megaterium.* In some embodiments, the subject is infected with, e.g., in the intraocular space, a microorganism herein, such as *Bacillus megaterium.*

In some embodiments, the present disclosure provides a method of using extracts of Traditional Chinese Medicine(s) (TCMs) that have antibacterial activities. In some embodiments, the method is for killing or inhibiting the growth of a microorganism herein, a method of treating an infection (e.g., ocular infection, such as in the intraocular space) with a microorganism herein, such as *Bacillus megaterium,* or for treating or preventing AMD in a subject in need thereof. In some embodiments, the method comprises administering to the subject an extract from one or more TCMs selected from Licorice (*e.g*., *Glycyrrhiza uralensis),* Rhubarb (*e.g*., *Rheum palmatum*), White Peony Root (*e.g*., *Cynanchum otophyllum*), Forsythia (*e.g*., *Forsythia suspense*), Fructus Aurantii (*e.g*., *Citrus aurantium L*.)*,* Rehmannia glutinosa (*e.g*., *Rehmannia glutinosa Libosch*), Tangerine Peel (*e.g*., *Citrus reticulata Blanco*), and Notoginseng (*e.g*., *Panax notoginseng*)*.* In some embodiments, the method further comprises identifying, or having identified, the subject as being infected with, e.g., in the intraocular space, a microorganism herein, such as *Bacillus megaterium.* In some embodiments, the subject is infected with, e.g., in the intraocular space, a microorganism herein, such as *Bacillus megaterium.* The extract can be an extract of a single TCM or an extract of more than one TCMs. Typically, the extract is an aqueous extract. In some embodiments, the extracts can exist in liquid, semisolid, or solid form or any other form. In some embodiments, the subject is further administered an antibiotic and/or anti-VEGF medication, e.g., as described herein.

In some embodiments, the present disclosure provides a method of using an antibiotic, for example, for killing or inhibiting the growth of a microorganism herein, treating an infection (e.g., ocular infection, such as in the intraocular space) with a microorganism herein, such as *Bacillus megaterium,* or for treating or preventing AMD, in a subject in need thereof. In some embodiments, the method comprises administering to the subject an effective amount of an antibiotic, e.g., as described herein. In some embodiments, any of the commercially available antibiotics, e.g., those approved by the U.S. FDA, can be used. In some embodiments, the method further comprises identifying, or having identified, the subject as being infected with, e.g., in the intraocular space, a microorganism herein, such as *Bacillus megaterium.* In some embodiments, the subject is infected with, e.g., in the intraocular space, a microorganism herein, such as *Bacillus megaterium.* In some embodiments, the subject is further administered an anti-VEGF medication, e.g., as described herein.

The administering herein is not limited to any particular route of administration. For example, in some embodiments, the administering can be orally, topically, intravitreously, intramuscularly, subcutaneously, or intravenously.

It is to be understood that both the foregoing summary and the following detailed description are exemplary and explanatory only, and are not restrictive of the invention herein.

### BRIEF DESCRIPTION OF THE DRAWINGS/FIGURES

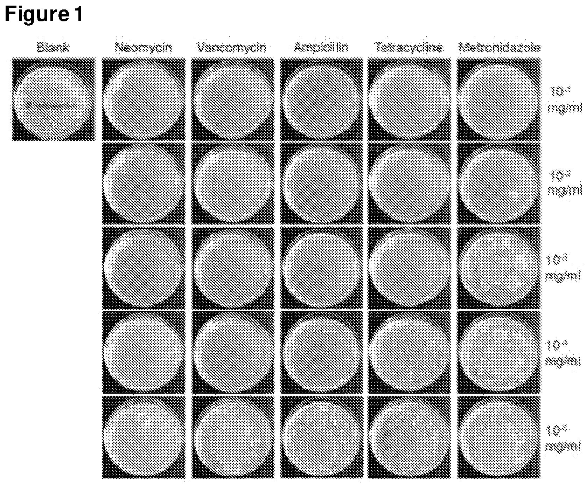

Figure 1 illustrates the sensitivity of *Bacillus megaterium* to several antimicrobial agents.

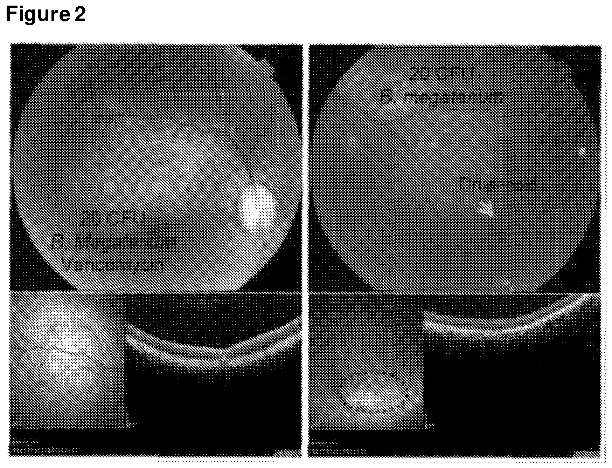

Figure 2 illustrates that an antibiotic treatment is able to change the bacteria-induced drusenoid pathology in monkey retinal tissues.
Figure 3 shows that compound 9 and reference compounds 1-8, 10-13 are effective in controlling growth of *Bacillus megaterium.* Testing condition: 1 mg compound, *B. megaterium,* at 1 × 10⁵/100ul in 15 ml medium.

### DETAILED DESCRIPTION OF THE INVENTION

In various embodiments, the present disclosure is based in part on the unexpected discovery that the intraocular environment is not sterile and certain intraocular microbiota can be pathogenic causes of AMD. From this initial discovery, which is detailed in WO2019080916 filed as PCT Application No. PCT/CN2018/112022, filed October 26, 2018, entitled METHODS AND COMPOSITIONS FOR ASSESSING AND TREATING INTRAOCULAR DISEASES AND DISORDERS, it was also found that such microorganisms, e.g., *Bacillus megaterium* (*B. megaterium*), when administered alive, can activate complement system and induce drusenoid lesions in macaque *in vivo.* Further, killing or inhibiting the growth of such microorganisms, such as by intravitreous administration of an antibiotic, vancomycin, can result in a reduction in the size of drusenoid lesion in retinal tissue of macaque as compared to control. *See also* Example 3 herein. These data and results establish that agents capable of killing or inhibiting the growth of such microorganisms, such as *Bacillus megaterium,* are useful in treating age-related macular degeneration.

As detailed in WO2019080916 filed as PCT Application No. PCT/CN2018/112022, metagenomic sequencing analysis were carried out on aqueous humor (AH) specimens from 41 cataract (Cat), 20 AMD, 18 glaucoma (GLA), 9 Betch's disease (BD), 9 Vogt-Koyanagi-Harada Syndrome (VKH), and 8 endophthalmitis (EOS) patients. 14 bacterial species were identified as highly enriched in the AH of AMD patients using metagenomic analysis. While *P. acnes* was the most abundant microorganism in the AH of AMD patients, *Bacillus licheniformis* (*B. licheniformis*) and *Bacillus megaterium* (*B. megaterium*) were the most enriched species, among the 14 AMD-specific ones, in AMD AH specimens. The present inventors then carried out PCR analysis to investigate whether the 14 AMD-specific bacteria could be detected in the hard or soft drusen tissues, as compared to the non-drusen retinal tissues from 6 archived ocular slides of AMD patients. The results showed only 8 bacteria could be detected, among which *P. acnes* was the most abundant species and *B. megaterium* was the only species enriched in soft drusen. The relative abundance of *P. acnes* was comparable in hard drusen, soft drusen, and dry AMD lesion tissues as compared to the non-drusen non-lesion retinal tissues. The relative abundance of *B. megaterium* was elevated by ~18 fold in soft drusen but not the AMD lesions when compared to the non-drusen/non-lesion tissues. These data suggest a possible role of *B. megaterium* in drusen formation and AMD pathogenesis.

Previous studies demonstrate that drusen contains a variety of complement components and polysaccharides in addition to many other proteins. In addition, the drusen components activate inflammasomes and promote expression of IL-1β and IL-18. The present inventors therefore first examined whether *B. megaterium,* as a component of drusen, was able to induce the activation of complement system and promote the secretion of IL-1β and IL-18, by acute retinal pigment epitheliitis-19(ARPE19) cells in vitro. The present inventors found *B. megaterium* but not *P. acnes* significantly increased the pyroptosis of RPE cells in a time dependent manner. The activation of complement system was confirmed by the production of active form of C5A protein. Both bacteria induced secretion of CFH proteins secreted by ARPE19 cell, while the induction of CFH was more profound by *B. megaterium* than by *P*. *acnes.* As the result of pyroptosis, in vitro infection of *B. megaterium,* but not *P. acnes,* led to secretion of active IL-1β and IL-18 by RPE cells. These results indicate that infection of *B. megaterium* can lead to inflammation similarly found in soft drusen.

The present inventors next tested whether *B. megaterium* was able to induce inflammation *in vivo.* The non-human primate macaque (Macaca fascicularis) as a model system considering the ocular anatomy and intraocular environment shared by human and macaque. Infection of live *P. acnes* bacterium or inoculation of its sonication-inactivated proteins into the eye, as well as live *B. licheniformis* bacterium or inoculation of its sonication-inactivated proteins into the eye did not induce significant intraocular inflammation. However, infection of live *B. megaterium* but not its proteins into the eye led to a profound intraocular inflammation. The intraocular inflammation induced by live *B*. *megaterium* was characterized by the elevation of TNFA and IL6 but not IFNG and IL17A expression. Importantly, only live *B. megaterium* was able to activate complement system including C5A and CFH and induce pyroptotic cytokines IL-1β and IL-18 *in vivo.* The bacteria remained alive in the eyes after inflammation was initiated, suggesting the intraocular inflammation can be long lasting in nature. Taken together, our data demonstrate that infection of *B. megaterium* can activate complement system and induce pyroptosis of ocular cells *in vitro* and *in vivo.*

Without wishing to be bound by theories, the fact that bacteria such as *B. megaterium* located in drusen and activated local complement-mediated immune response can explain the formation of diversified drusen between RPE and Bruch's membrane. The major proteins found in drusen including complement components such as C1Q and immunoglobulin are all first line of anti-infectious agents. Other drusen proteins such as vitronectin and Apolipoprotein E are all recently proved as anti-infectious agents. Therefore, the formation of drusen is very possible the key response of the aging retina in controlling infiltrated bacterial pathogens. Due to the diversity of bacteria, the shape and size of drusen could vary. In the case of hard drusen, where the infection may be cleared, drusen will disappear. However, certain pathogens such as *B. megaterium* will induce long term activation of immune responses in soft drusen and result in the damage of RPE cells and photoreceptors. Activation of the inflammation of macrophage and pyroptosis of RPE cells are protective responses against local infection, which is consistent with the previous finding that NLRP3 mediated inflammasome activation and IL-18 production protect the retina from neovascularization.

Without wishing to be bound by theories, the infectious etiology of AMD is also consistent with the conclusions reached by all genetic studies. For example, a defective CFH, the negative regulator of complement activation induced by *B. megaterium* infection, will result in uncontrolled complement activation. A defective HTRA1, the protease producing the active form of immunosuppressive cytokine TGF-β, will result in decrease of local TGF-β family proteins. Both of these genetic variations can lead to dysregulation of local anti-infectious responses that damages RPE cells and photoreceptors.

In addition, the potential difference in pathogenic microbiota found in drusen may explain the association of varied genetic risk factors with different ethnic groups (e.g. Caucasian vs Asian). Therefore, evidence shows that the infectious etiology of AMD is one mechanism by which early AMD pathology is initiated in the elderly.

In summary, in various embodiments, the present inventors show that killing and/or inhibiting growth of microorganisms can treat and/or prevent AMD, such as dry or wet age-related macular degeneration with drusen symptoms, including a hard drusen, a soft drusen, a mixed drusen and/or a degraded drusen, for example, dry or wet age-related macular degeneration with soft drusen symptoms.

### Compounds

In some embodiments, the present disclosure is directed to various compounds and/or compositions comprising the compounds that can kill or inhibit the growth of microorganisms related to AMD, such as *Bacillus megaterium.*

The compounds herein typically have antibacterial activity by themselves or in combination with another agent. The compounds herein can be bactericidal or bacteriostatic. Various compounds known to have antibacterial activities can be used for embodiments of the present invention. For example, in some embodiments, the compounds herein can include any of the alcohols, phenolic compounds, amines, sulfonamides, quinolones, anthraquinone, and/or benzoic acid related compounds that are known to have antibacterial activities. Nonlimiting examples of useful compounds include benzoid acid, benzyl alcohol, coumarins, catechols, polyphenols, chalconoids (including licochalcones), etc., stilbenes such as resveratrol, isoresveratrol, etc., phenolic acids, such as p-hydroxbenzoic acid, 2,4-dihydroxbenzoid acid, protocatechuic acid, gallic acid, vanillic acid, syringic acid, cinnamic acid, coumaric acids, caffeic acids, ferulic acids, chlorogenic acid, sinapic acids etc., flavonoids such as catechin, narigenin, quercetin, rutin, chrysin, etc., tannins, such as ellagic acid, and esters thereof and glycosides thereof.

The compounds herein are typically characterized by certain functional groups present in their molecular structures. For example, in some embodiments, the compounds herein are characterized by having alcoholic hydroxyl group, phenolic hydroxyl group, and/or carboxylic acid group, or derivatives thereof such as esters, amides, carbonates, carbamates, sulfonates, glycosides, etc. In some embodiments, compounds with an amino group, a sulfonamide group, a thiol group, and/or a sulfoxide or sulfone group can also be useful for the compositions and methods herein.

The compounds herein can have a polycyclic core structure, a bicyclic core structure, or a monocyclic core structure, each of which can be substituted with various groups as described herein.

In some embodiments, the compounds herein can be characterized by having a Formula I, or a pharmaceutically acceptable salt or ester thereof: 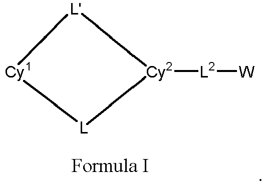 For the avoidance of doubt, in Formula I, a cyclic structure Cy¹ is connected with another cyclic structure Cy², which can be the same or different, through two linkers, L and L , which form an additional ring structure between Cy¹ and Cy². It should be understood that both Cy¹ and Cy² are separately a ring structure, which is independent of L and L .

In Formula I, Cy¹ and Cy² are each independently an optionally substituted cycloalkyl ring (e.g., C₃₋₇ cycloalkyl ring), an optionally substituted heterocyclic ring, such as an optionally substituted 4-7 membered heterocyclic ring (e.g., having one or two ring heteroatoms independently selected from N, O, and S), an optionally substituted aryl ring (e.g., C₆₋₁₀ aryl ring (e.g., Phenyl)), or an optionally substituted heteroaryl ring, such as an optionally substituted 5-10 membered heteroaryl ring (e.g., 5, or 6-membered heteroaryl ring with one or two ring heteroatoms independently selected from N, O, and S);
L and L' are each independently null or a linker (e.g., described herein); as used herein, the term "linker" is not restricted to any particular types of linking groups. For example, in some embodiments, the linker can also form a ring structure with one of the moieties that it is attached to, for example, L and Cy¹ can form a ring structure independent of Cy²;
L² is null, an optionally substituted C₁₋₆ alkylene, an optionally substituted C₁₋₆ heteroalkylene, an optionally substituted C₂₋₆ alkenylene, an optionally substituted C₂₋₆ alkynylene, an optionally substituted C₃₋₆ cycloalkylene, an optionally substituted arylene, an optionally substituted heteroarylene, or an optionally substituted 4-7 membered heterocyclylene;
W is -OR¹; -COR²; -COOR^{1a}; -OCOOR^{1a}; -NR³R⁴; -CONR^{3a}R^{4a}; -CONR^{3b}R^{4b}; - SO₂NR^{3c}R^{4c}; -OSO₂NR^{3d}R^{4d}; -SR⁵; -SO₂R^{5a}; -OCOR^{2a}; -OSO₂R^{5a} or 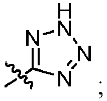
wherein:
   R¹ and R^{1a} are each independently hydrogen, optionally substituted alkyl, optionally substituted alkenyl, optionally substituted alkynyl, optionally substituted cycloalkyl, optionally substituted aryl, optionally substituted heteroaryl, or optionally substituted heterocyclyl;
   R³ and R⁴ are each independently hydrogen, -COR^{2b}, -SO₂R^{5b}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl, or R³ and R⁴ together with the atoms they are bound to form an optionally substituted 4-7 membered heterocyclyl;
   R², R^{2a}, R^{2b}, R⁵, R^{5a}, and R^{5b} are each independently hydrogen, -OH, -NR^{3e}R^{4e}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; and
   R^{3a}, R^{3b}, R^{3c}, R^{3d}, R^{3e}, R^{4a}, R^{4b}, R^{4c}, R^{4d}, and R^{4e} are each independently hydrogen, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or R^{3a} and R^{4a}, R^{3b} and R^{4b}, R^{3c} and R^{4c}, R^{3d} and R^{4d}, or R^{3e} and R^{4e}, together with the atoms they are bound to form an optionally substituted 4-7 membered heterocyclyl.

The Cy¹ and Cy² in Formula I can be either an aromatic or non-aromatic ring system, and can in some cases include heteroatoms. In preferred embodiments, at least one of Cy¹ and Cy² in Formula I is an aryl or heteroaryl ring, such as an optionally substituted C₆₋₁₀ aryl ring, or an optionally substituted 5-10 membered heteroaryl ring. For example, in some embodiments, the Cy¹ and Cy² are such that the core structure of Formula I, the structure of 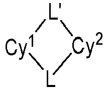 without showing optional substituents, can be any of the following: 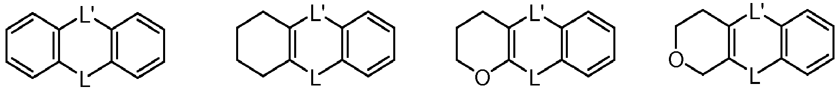 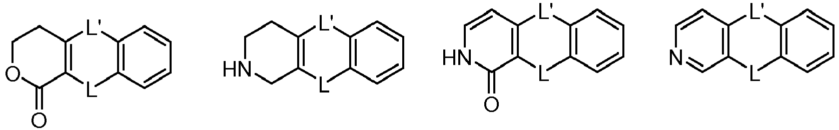 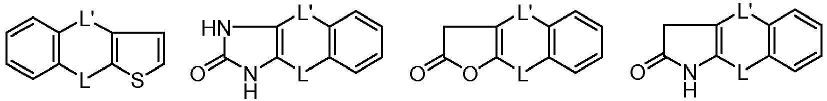 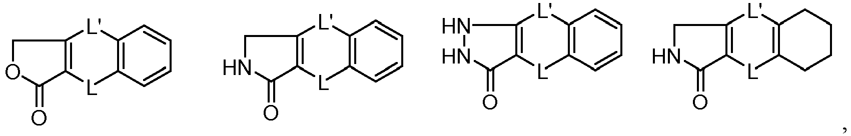 wherein L²-W can be attached to either the left or the right ring, wherein L and L' can be any of those described herein and suitable substituents for the rings are described herein.

In some embodiments, both Cy¹ and Cy² in Formula I can be an aryl or heteroaryl ring. For example, in some embodiments, the compound of Formula I can have a Formula I-1:

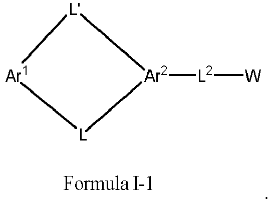

In some embodiments, Ar¹ and Ar² in Formula I-1 are each independently an optionally substituted C₆₋₁₀ aryl ring, or an optionally substituted 5-10 membered heteroaryl ring. In some embodiments, Ar¹ and Ar² in Formula I-1 are each independently an optionally substituted phenyl ring or a 5 or 6 membered heteroaryl ring. For example, in some embodiments, Ar¹ and Ar² in Formula I-1 are each independently an optionally substituted phenyl ring, an optionally substituted thienyl ring, an optionally substituted furanyl ring, an optionally substituted pyridyl ring, or an optionally substituted pyrimidinyl ring.

Formula I-1 typically has a polycyclic core structure. For example, in some embodiments, the Ar¹ and Ar² are such that the core structure of Formula I-1, 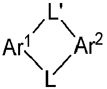 without showing optional substituents, can be any of the following: 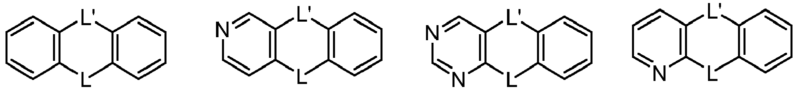 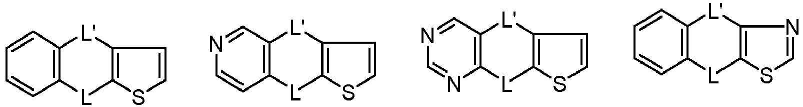 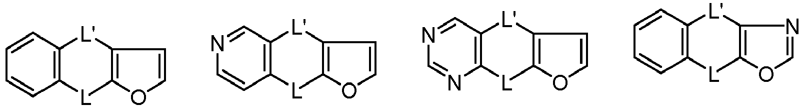 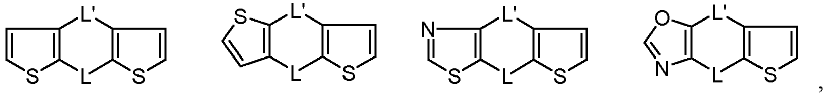 wherein L²-W can be attached to either the left or the right ring, wherein L and L' are defined herein and suitable substituents for the rings are described herein.

In some embodiments, the compound of Formula I can have a Formula I-2: 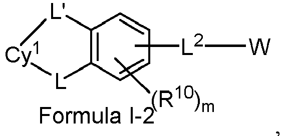 wherein:
m is 0, 1, 2, or 3,
R¹⁰ at each occurrence is independently halogen, L^{2'}-W^{'}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or two adjacent R¹⁰, or one R¹⁰ and L or L^{'}, together with the atoms they are bound to, form an optionally substituted cycloalkyl, heterocyclyl, aryl, or heteroaryl ring;
wherein -L^{2'}-W^{'} at each occurrence is independently selected; and
L^{2'} at each occurrence is independently null, an optionally substituted C₁₋₆ alkylene, an optionally substituted C₁₋₆ heteroalkylene, an optionally substituted C₂₋₆ alkenylene, an optionally substituted C₂₋₆ alkynylene, an optionally substituted C₃₋₆ cycloalkylene, an optionally substituted arylene, an optionally substituted heteroarylene, or an optionally substituted 4-7 membered heterocyclylene; and W' at each occurrence is independently -OR¹; -COR²; -COOR^{1a}; -OCOOR^{1a}; -NR³R⁴; -CONR^{3a}R^{4a}; -OCONR^{3b}R^{4b}; -SO₂NR^{3c}R^{4c}; - OSO₂NR^{3d}R^{4d}; -SR⁵; -SO₂R^{5a}; -OCOR^{2a}; -OSO₂R^{5a} or 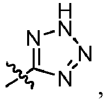 wherein R¹, R^{1a}, R², R^{2a}, R^{2b}, R³, R⁴, R^{3a}, R^{3b}, R^{3c}, R^{3d}, R^{3e}, R^{4a}, R^{4b}, R^{4c}, R^{4d}, R^{4e}, R⁵, R^{5a}, and R^{5b} are defined herein, see e.g., Formula I.
It should be noted that each instance of the structural unit -L^{2'}-W^{'} and -L²-W are independently selected and can be the same or different.

In some embodiments, Cy¹ in Formula I-2 is an optionally substituted phenyl ring, an optionally substituted thienyl ring, an optionally substituted furanyl ring, an optionally substituted pyridyl ring, or an optionally substituted pyrimidinyl ring. In some embodiments, Cy¹ in Formula I-2 is an optionally substituted C₃₋₆ cycloalkyl ring or an optionally substituted 4-7 heterocyclic ring with 1 or 2 ring heteroatoms independently selected from N, O, and S.

In some embodiments, the Cy¹ is such that the core structure of Formula I-2 can be any of the following: 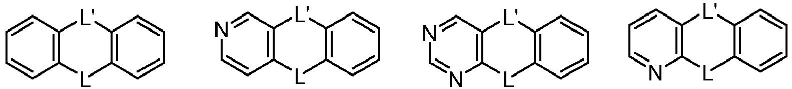 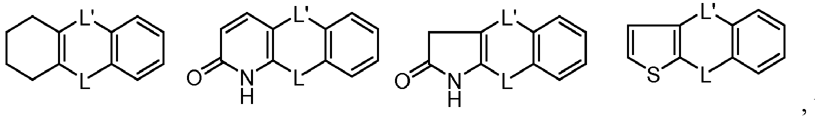 wherein -L²-W is attached to the right phenyl ring, L and L' are defined herein and suitable substituents for the rings are described herein.

In more preferred embodiments, both Cy¹ and Cy² in Formula I are phenyl rings. For example, in some embodiments, the compound of Formula I-2 can have a Formula I-3: 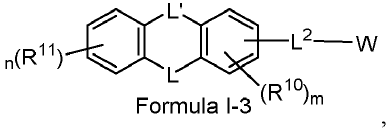
wherein: L, L^{'}, L², W, R¹⁰, and m are defined herein, see e.g., Formula I-2,
n is 0, 1, 2, or 3,
R¹¹ at each occurrence is independently halogen, -L^{2'}-W^{'}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or two adjacent R¹¹, or one R¹¹ and L or L^{'}, together with the atoms they are bound to form an optionally substituted cycloalkyl, heterocyclyl, aryl, or heteroaryl ring; wherein L^{2'} and W^{'} are defined herein, see e.g., for Formula I-2, and -L^{2'}-W^{'} at each occurrence is independently selected.

L and L' in Formula I (e.g., any of the Formula I-1 to I-3) can be independently null or a linker. In some embodiments, L and L' in Formula I are each independently null, -C(O)-, optionally substituted C₁₋₄ alkylene, optionally substituted C₂₋₄ alkenylene, -O-, -S-, -NR¹⁰⁰-, - S(O)-, -SO₂-, -X¹-G¹-, -X²-G²-X^{2a}-, or -CR¹⁰¹R¹⁰²-,
wherein:
X¹, X², and X^{2a} are independently optionally substituted C₁₋₄ alkylene, optionally substituted C₂₋₄ alkenylene, -O-, -C(O)-, -S-, -NR^{100a}-, -S(O)-, -SO₂-, or -CR^{101a}R^{102a}-;
G¹ and G² are independently optionally substituted C₁₋₄ alkylene, optionally substituted C₂₋₄ alkenylene, -C(O)-, -NR^{100a}-, -S(O)-, -SO₂-, or -CR^{101a}R^{102a}-;
preferably, in some embodiments, -X¹-G¹- or -X²-G²-X^{2a}- does not contain an O-N, S-S, S-N (other than SO₂-N), or -C(O)-S bond;
R¹⁰⁰ and R^{100a} are each independently lone pair (as applicable), hydrogen, COR^{2c}, - SO₂R^{5c}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl; or R¹⁰⁰ or R^{100a} forms an optionally substituted heterocyclic or heteroaryl ring with a R¹⁰ or R¹¹ group;
R¹⁰¹, R^{101a}, R¹⁰², and R^{102a}, when present, are each independently hydrogen, -OH, halogen, optionally substituted C₁₋₆ alkyl, optionally substituted C₂₋₆ alkenyl, optionally substituted C₂₋₆ alkynyl, optionally substituted C₃₋₆ cycloalkyl, optionally substituted C₁₋₆ alkoxy, optionally substituted C₃₋₆ cycloalkoxy, optionally substituted amino group, optionally substituted phenyl, optionally substituted 5 or 6 membered heteroaryl, or optionally substituted 4-7 membered heterocyclyl, or R¹⁰¹ and R¹⁰², or R^{101a} and R^{102a}, together with the atoms they are bound to form an optionally substituted 3-7 membered cycloalkyl or heterocyclyl ring; or one of R¹⁰¹ and R¹⁰², or one of R^{101a} and R^{102a} forms an optionally substituted cycloalkyl or heterocyclyl ring together with a R¹⁰ or R¹¹ group; and
R^{2c} and R^{5c} are each independently hydrogen, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl.

When the linker L or L' forms a double bond with one of the ring carbons, it cannot be CR¹⁰¹R¹⁰² with both R¹⁰¹ and R¹⁰² present, as the valence of the carbon will exceed 4. In such cases, it should be understood that one of R¹⁰¹ and R¹⁰² is absent and L or L' is CR¹⁰¹ or CR¹⁰² as defined herein. When L or L' forms a double bond with one of the ring carbons, it can be NR¹⁰⁰ with R¹⁰⁰ typically being a lone pair. Other similar situations in the present disclosure should be understood similarly.

In some embodiments, L and L' in Formula I are each independently null, -O-, -C(O)-, -S-, -NR¹⁰⁰-, -S(O)-, -SO₂-, or -CR¹⁰¹R¹⁰²-. In some embodiments, the compound of Formula I has a formula according to any one of I-4 to I-6: 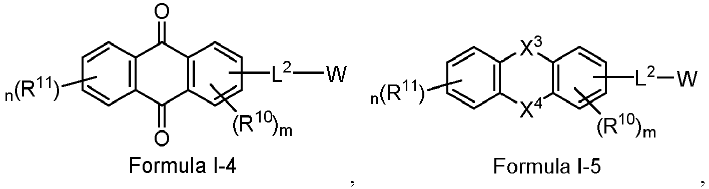 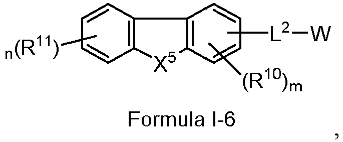 wherein:
X³, X⁴, and X⁵ are each independently null, -O-, -C(O)-, -S-, -NR^{100a}-, -S(O)-, -SO₂-, or - CR^{101a}R^{102a}-; and
R¹⁰, R¹¹, R^{100a}, R^{101a}, R^{102a}, W, L², m and n are defined herein.

In some embodiments, the compound has a Formula I-5, wherein X³ and X⁴ are each independently -O-, -C(O)-, -S-, -NR^{100a}-, or -SO₂-. In some embodiments, the compound has a Formula I-6, wherein X⁵ is -O-, -C(O)-, -S-, -NR^{100a}-, or -SO₂-. In some embodiments, R^{100a} is hydrogen or an optionally substituted C₁₋₄ alkyl.

In some embodiments, the compound of Formula I can have a structure of any one of the following: 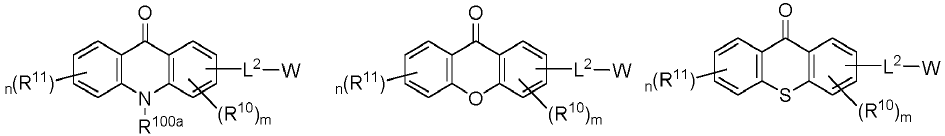 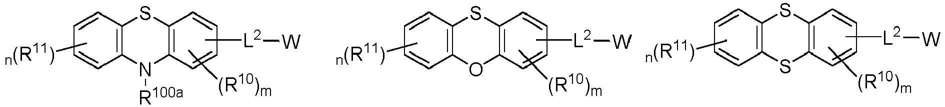 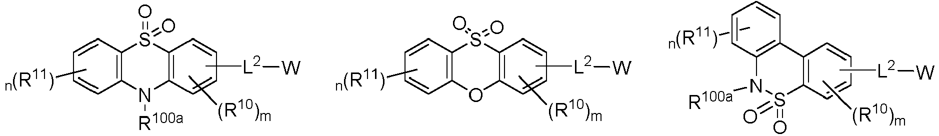 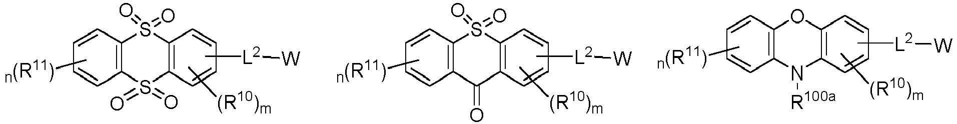 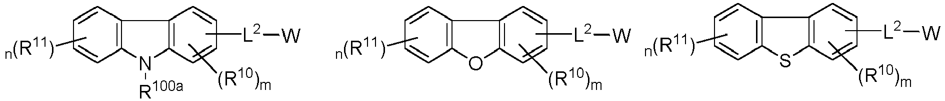 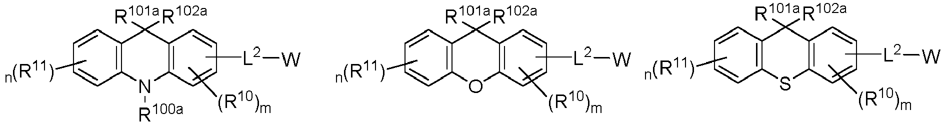 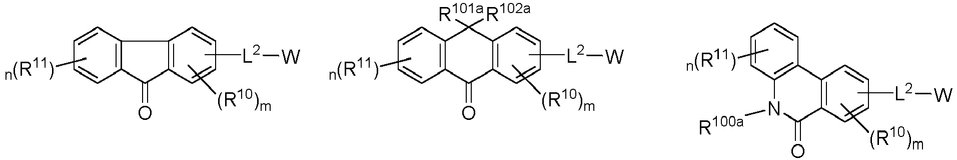 wherein R¹⁰, R¹¹, R^{100a}, R^{101a}, R^{102a}, W, L², m, and n are defined herein, for example, in connection with any of Formula I, any of the sub-formulae described herein, such as Formula I-1 to I-6, as applicable.

L² in Formula I (e.g., any of the sub-formulae described herein, such as Formula I-1 to I-6) is typically null, i.e., the W group is directly attached to Cy². In some embodiments, L² in Formula I can also be a C₁₋₄ alkylene, C₂₋₄ alkenylene, C₂₋₄ alkynylene or C₁₋₄ heteroalkylene. For example, the W group can be attached to Cy², through a methylene or vinyl group.

Various W groups are suitable for compounds of Formula I (e.g., any of the sub-formulae described herein, such as Formula I-1 to I-6). In preferred embodiments, W group at each occurrence is independently -OH, -NH₂, -SO₂NH₂, -SO₂NH(C₁₋₄ alkyl), -SO₂NH(C₁₋₄ alkanoyl), -COOH, 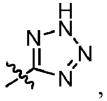 -C(O)(O-C₁₋₁₀ alkyl), -C(O)(O-C₂₋₁₀ alkenyl), -OC(O)NH₂, - OC(O)NH(C₁₋₄ alkyl)-, -O-(CO)-(C₁₋₄ alkyl), -O-(C₁₋₄ alkyl), wherein each of the C₁₋₄ alkyl is independently optionally substituted with 1-3 substituents independently selected from C₁₋₄ alkyl, C₁₋₄ alkoxy, -OH, -NH₂, and fluorine. In some embodiments, W in Formula I is -OH, - NH₂, -SO₂NH₂, -SO₂NH(Acetyl), -COOH, 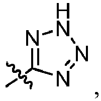 or -O-C(O)-CH₃.

As described herein, L^{2'}-W^{'} can in some embodiments be selected as a substituent for Cy¹ or Cy², such as for Ar¹ or Ar². When applicable, L^{2'} in Formula I, including any of the sub-formulae described herein, such as Formula I-1 to I-6, at each occurrence can be independently null, i.e., the W' group is directly attached to Cy¹ or Cy², such as for Ar¹ or Ar², as applicable, or a C₁₋₄ alkylene, C₂₋₄ alkenylene, C₂₋₄ alkynylene or C₁₋₄ heteroalkylene. For example, the W^{'} group can be attached to Cy¹ or Cy², such as for Ar¹ or Ar², as applicable, through a methylene or vinyl group. When applicable, W^{'} in Formula I, including any of the sub-formulae described herein, such as Formula I-1 to I-6, at each occurrence can be independently -OH, -NH₂, -SO₂NH₂, -SO₂NH(C₁₋₄ alkyl), -SO₂NH(C₁₋₄ alkanoyl), - COOH, 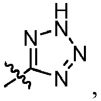 -C(O)(O-C₁₋₁₀ alkyl), -C(O)(O-C₂₋₁₀ alkenyl), -OC(O)NH₂, -OC(O)NH(C₁₋₄ alkyl)-, -O-(CO)-(C₁₋₄ alkyl), -O-(C₁₋₄ alkyl), wherein each of the C₁₋₄ alkyl is independently optionally substituted with 1-3 substituents independently selected from C₁₋₄ alkyl, C₁₋₄ alkoxy, -OH, -NH₂, and fluorine. In some embodiments, each instance of W^{'} in Formula I, when applicable, can be -OH, -NH₂, -SO₂NH₂, -SOzNH(Acetyl), -COOH, 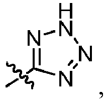 or -O-C(O)-CH₃.

Various groups can be suitable for R¹⁰ and R¹¹ in any of the applicable Formula I (e.g., any of the sub-formulae described herein, such as Formula I-2 to I-6, as applicable). In some embodiments, each of R¹⁰ and R¹¹ at each occurrence can be independently F; Cl; -OH; - NH₂; -SO₂NH₂; -SO₂NH(C₁₋₄ alkyl); -SO₂NH(C₁₋₄ alkanoyl); -COOH; 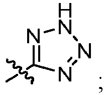 -C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine. In some embodiments, each of R¹⁰ and R¹¹ at each occurrence can be independently -OH; -NH₂; -SO₂NH₂; -SO₂NH(C₁₋₄ alkyl); -SO₂NH(C₁₋₄ alkanoyl); -COOH; 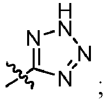 -C(O)(O-C₁₋₁₀ alkyl), -C(O)(O-C₂₋₁₀ alkenyl), -OC(O)NH₂; - OC(O)NH(C₁₋₄ alkyl)-; -O-(CO)-(C₁₋₄ alkyl); C₁₋₄ alkyl; or C₁₋₄ alkoxy. In some embodiments, one or more instances of R¹⁰ and/or one or more instances of R¹¹ can be independently selected L^{2'}-W^{'} as described herein.

Typically, m, as applicable, is 0, 1, or 2; preferably, 1.

Typically, n, as applicable, is 0, 1, 2, or 3; preferably, 1 or 2.

In some preferred embodiments, the compound of Formula I has a formula I-7, I-8, or I-9: 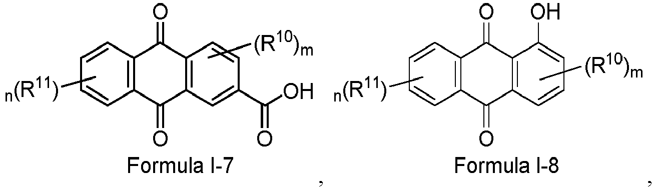 or 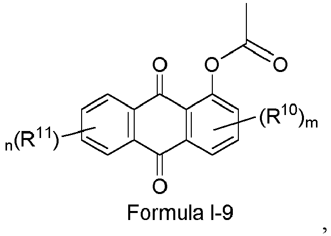 wherein R¹⁰, R¹¹, m, and n are defined herein.

In some embodiments, in Formula I-7 to I-9, each of R¹⁰ and R¹¹ at each occurrence can be independently F; Cl; -OH; -NH₂; -SO₂NH₂; -SO₂NH(C₁₋₄ alkyl); -SO₂NH(C₁₋₄ alkanoyl); - COOH; 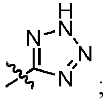 -C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine. In some embodiments, each of R¹⁰ and R¹¹ at each occurrence can be independently -OH; -NH₂; - SO₂NH₂; -SO₂NH(C₁₋₄ alkyl); -SO₂NH(C₁₋₄ alkanoyl); -COOH; 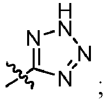 -C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); C₁₋₄ alkyl; or C₁₋₄ alkoxy. In some embodiments, one or more instances of R¹⁰ and/or one or more instances of R¹¹ can be independently selected L^{2'}-W^{'} as described herein. In some embodiments, m in Formula I-7 to I-9 is 1, and n in Formula I-7 to I-9 is 1 or 2.

In some preferred embodiments, the compound of Formula I has a Formula I-10 or I-11: 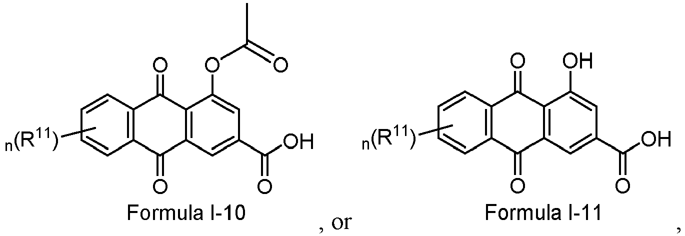 wherein R¹⁰, R¹¹, m, and n are defined herein.

In some embodiments, R¹¹ at each occurrence can be independently F; Cl; -OH; -NH₂; - SO₂NH₂; -SO₂NH(C₁₋₄ alkyl); -SO₂NH(C₁₋₄ alkanoyl); -COOH; 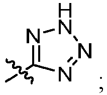 -C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine. In some embodiments, R¹¹ at each occurrence is independently -OH; -NH₂; -SO₂NH₂; - SO₂NH(C₁₋₄ alkyl); -SO₂NH(C₁₋₄ alkanoyl); -COOH; 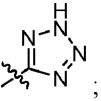 -C(O)(O-C₁₋₁₀ alkyl), - C(O)(O-C₂₋₁₀ alkenyl), -OC(O)NH₂; -OC(O)NH(C₁₋₄ alkyl)-; -O-(CO)-(C₁₋₄ alkyl); C₁₋₄ alkyl; or C₁₋₄ alkoxy. In some embodiments, n in Formula I-10 to I-11 is 0, 1 or 2, preferably, 1, or 2.

In some specific embodiments, the compound of Formula I can be 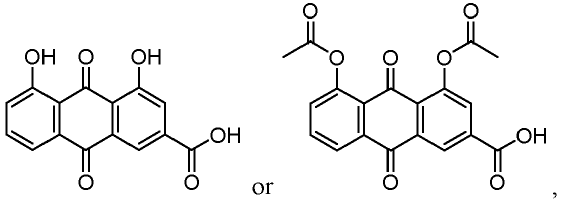 or a pharmaceutically acceptable salt or ester thereof.

In some embodiments, the compounds herein can be characterized by having a Formula II, or a pharmaceutically acceptable salt or ester thereof:

Cy¹⁰-L¹⁰-Cy¹¹-L¹¹-W¹⁰ Formula II

wherein:
Cy¹⁰ and Cy¹¹ are each independently an optionally substituted cycloalkyl ring (e.g., C₃₋₇ cycloalkyl ring), an optionally substituted heterocyclic ring (e.g., 4-7 membered heterocyclic ring), an optionally substituted aryl ring (e.g., C₆₋₁₀ aryl ring), an optionally substituted heteroaryl ring (e.g., 5-10 membered heteroaryl ring), or an optionally substituted ring structure comprising a cycloalkyl ring or heterocyclic ring, and an aryl or heteroaryl ring, wherein the ring structure can be a fused ring or otherwise connected;
L¹⁰ is null or a linker;
L¹¹ is null, an optionally substituted C₁₋₆ alkylene, an optionally substituted C₁₋₆ heteroalkylene, an optionally substituted C₂₋₆ alkenylene, an optionally substituted C₂₋₆ alkynylene, an optionally substituted C₃₋₆ cycloalkylene, an optionally substituted arylene, an optionally substituted heteroarylene, or an optionally substituted 4-7 membered heterocyclylene,
W¹⁰ is -OR¹; -COOR^{1a}; -OCOOR^{1a}; -COR²; -NR³R⁴; -CONR^{3a}R^{4a}; -OCONR^{3b}R^{4b}; - SO₂NR^{3c}R^{4c}; -OSO₂NR^{3d}R^{4d}; -SR⁵; -SO₂R^{5a}; -OCOR^{2a}; -OSO₂R^{5a}; or 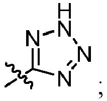
wherein:
   R¹ and R^{1a} are each independently hydrogen, optionally substituted alkyl, optionally substituted alkenyl, optionally substituted alkynyl, optionally substituted cycloalkyl, optionally substituted aryl, optionally substituted heteroaryl, or optionally substituted heterocyclyl;
   R³ and R⁴ are each independently hydrogen, -COR^{2b}, -SO₂R^{5b}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl, or R³ and R⁴ together with the atoms they are bound to form an optionally substituted 4-7 membered heterocyclyl;
   R², R^{2a}, R^{2b}, R⁵, R^{5a}, and R^{5b} are each independently hydrogen, -OH, -NR^{3e}R^{4e}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; and
   R^{3a}, R^{3b}, R^{3c}, R^{3d}, R^{3e}, R^{4a}, R^{4b}, R^{4c}, R^{4d}, and R^{4e} are each independently hydrogen, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or R^{3a} and R^{4a}, R^{3b} and R^{4b}, R^{3c} and R^{4c}, R^{3d} and R^{4d}, or R^{3e} and R^{4e}, together with the atoms they are bound to form an optionally substituted 4-7 membered heterocyclyl.

In some embodiments, in Formula II, at least one of Cy¹⁰ and Cy¹¹ is an optionally substituted C₆₋₁₀ aryl ring, or an optionally substituted 5-10 membered heteroaryl ring. In some embodiments, Cy¹¹ is an optionally substituted C₆₋₁₀ aryl ring, or an optionally substituted 5-10 membered heteroaryl ring. When Cy¹¹ is a bicyclic or polycyclic aryl or heteroaryl ring, the L¹⁰-Cy¹⁰ and L¹¹-W¹⁰ can be independently connected to Cy¹¹through any of the rings. In some embodiments, Cy¹¹ can have a fused ring structure comprising an aryl or heteroaryl ring and a cycloalkyl or heterocyclic ring structure. In such embodiments, Cy¹¹ can be connected to L¹⁰-Cy¹⁰ and L¹¹-W¹⁰ through either of the aryl or heteroaryl ring and cycloalkyl or heterocyclic ring structure; or alternatively, one of L¹⁰-Cy¹⁰ and L¹¹-W¹⁰ is connected to Cy¹¹through the aryl or heteroaryl ring and the other of L¹⁰-Cy¹⁰ and L¹¹-W¹⁰ is connected to Cy¹¹through the cycloalkyl or heterocyclic ring structure.

In some embodiments, the compound of Formula II has at least one phenyl ring, which can have the following core structure as Cy¹⁰-L¹⁰-Cy¹¹: 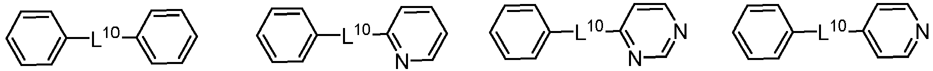 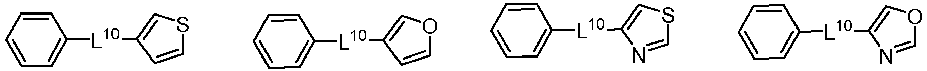 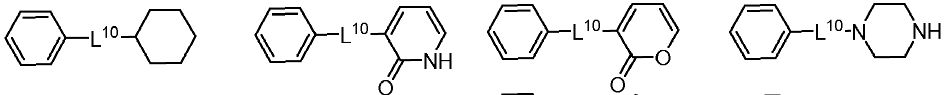 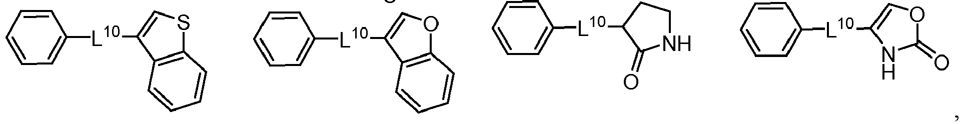 wherein Cy¹⁰ can be the left ring or the right ring in the above drawings, i.e., the drawings are not limited to a particular direction, wherein L¹¹-W¹⁰ can connect to either the left or the right ring, both of which can be optionally substituted.

In some embodiments, the compound of Formula II can have the following core structure as Cy¹⁰-L¹⁰-Cy¹¹: 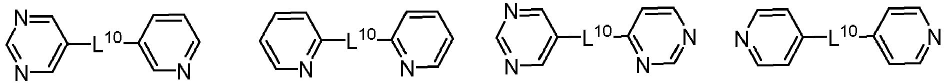 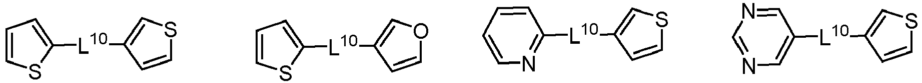 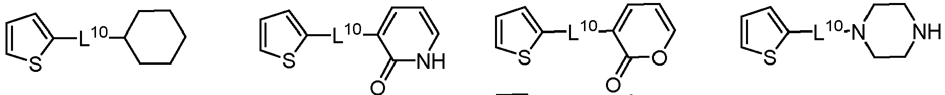 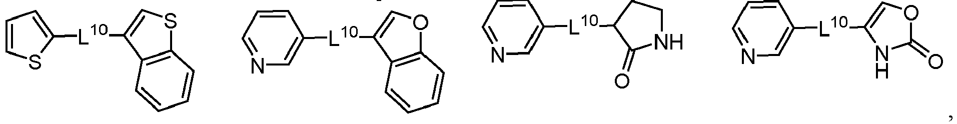 wherein Cy¹⁰ can be the left ring or the right ring in the above drawings, i.e., the drawings are not limited to a particular direction, wherein L¹¹-W¹⁰ can connect to either the left or the right ring, both of which can be optionally substituted.

In some embodiments, both of Cy¹⁰ and Cy¹¹ in Formula II are an aryl or heteroaryl ring. In some embodiments, the compound of Formula II has a Formula II-1:

Ar¹⁰-L¹⁰-Ar¹¹-L¹¹-W¹⁰ Formula II-1 ,

wherein Ar¹⁰ and Ar¹¹ are each independently an optionally substituted C₆₋₁₀ aryl ring, or an optionally substituted 5-10 membered heteroaryl ring. In some embodiments, Ar¹⁰ and Ar¹¹ in Formula II-1 are each independently an optionally substituted phenyl ring or an optionally substituted 5 or 6 membered heteroaryl ring. In some embodiments, Ar¹⁰ and Ar¹¹ in Formula II-1 are each independently an optionally substituted phenyl ring, an optionally substituted thienyl ring, an optionally substituted furanyl ring, an optionally substituted pyridyl ring, or an optionally substituted pyrimidinyl ring. In some embodiments, one of Ar¹⁰ and Ar¹¹ in Formula II-1 is a bicyclic aryl or bicyclic heteroaryl ring, each of which is optionally substituted, for example, in some embodiments, Ar¹¹ can be optionally substituted bicyclic aryl or bicyclic heteroaryl ring.

In some embodiments, Cy¹¹ in Formula II is a phenyl ring. In some embodiments, the compound of Formula II has a Formula II-2: 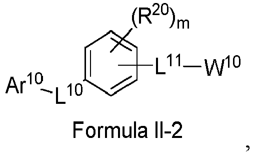
wherein Ar¹⁰, L¹⁰, L¹¹, and W¹⁰ are defined herein, see e.g., Formula II-1,
m is 0, 1, 2, or 3,
R²⁰ at each occurrence is independently halogen, -L^{11'}-W^{10'}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or two adjacent R²⁰, or one R²⁰ and L¹⁰ or L¹¹, together with the atoms they are bound to form an optionally substituted cycloalkyl, heterocyclyl, aryl, or heteroaryl ring;
wherein -L^{11'}-W^{10'} at each occurrence is independently selected;
wherein L^{11'} at each occurrence is independently null, an optionally substituted C₁₋₆ alkylene, an optionally substituted C₁₋₆ heteroalkylene, an optionally substituted C₂₋₆ alkenylene, an optionally substituted C₂₋₆ alkynylene, an optionally substituted C₃₋₆ cycloalkylene, an optionally substituted arylene, an optionally substituted heteroarylene, or an optionally substituted 4-7 membered heterocyclylene; and W^{10'} at each occurrence is independently -OR¹; -COR²; -COOR^{1a}; -OCOOR^{1a}; -NR³R⁴; -CONR^{3a}R^{4a}; -OCONR^{3b}R^{4b}; - SO₂NR^{3c}R^{4c}; -OSO₂NR^{3d}R^{4d}; -SR⁵; -SO₂R^{5a}; -OCOR^{2a}; -OSO₂R^{5a} or 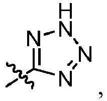 wherein R¹, R^{1a}, R², R^{2a}, R^{2b}, R³, R⁴, R^{3a}, R^{3b}, R^{3c}, R^{3d}, R^{3e}, R^{4a}, R^{4b}, R^{4c}, R^{4d}, R^{4e}, R⁵, R^{5a}, and R^{5b} are defined herein, see e.g., Formula II. It should be noted that each instance of the structural unit -L^{11'}-W^{10'} and -L¹¹-W¹⁰ are independently selected and can be the same or different.

In some embodiments, Cy¹¹ in Formula II is a benzofused ring. In some embodiments, the compound of Formula II has a Formula II-3: 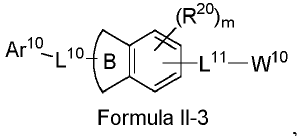
wherein Ar¹⁰, L¹⁰, L¹¹, and W¹⁰ are defined herein, see e.g., Formula II-1,
m is 0, 1, 2, or 3,
R²⁰ at each occurrence is independently halogen, -L^{11'}-W^{10'}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or two adjacent R²⁰, or one R²⁰ and L¹⁰ or L¹¹, together with the atoms they are bound to form an optionally substituted cycloalkyl, heterocyclyl, aryl, or heteroaryl ring;
wherein L^{11'} and W^{10'} are defined herein, see e.g., Formula II-2, and -L^{11'}-W^{10'} at each occurrence is independently selected; and
ring B is a 4-7 membered cycloalkyl ring, 4-7 membered heterocyclic ring, phenyl ring, 5 or 6 membered heteroaryl ring, each of which is optionally substituted.

In some embodiments, Cy¹¹ in Formula II is a benzofused bicyclic aryl or heteroaryl ring. For example, in some embodiments, Cy¹¹ in Formula II can have the following core structure: 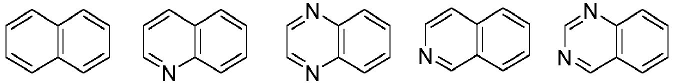 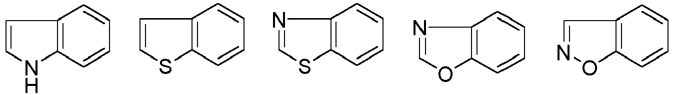 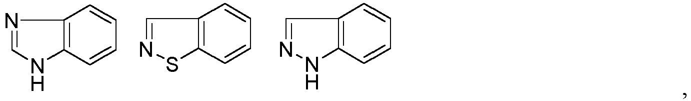 wherein the L¹⁰-Cy¹⁰ and L¹¹-W¹⁰ can be independently connected to Cy¹¹through any of the two rings, wherein the phenyl ring can be optionally substituted with 1-3 R²⁰ groups defined herein. For example, in the case of benzothiophene ring, in some embodiments, L¹⁰-Cy¹⁰ can be attached to the thiophene ring whereas L¹¹-W¹⁰ can be attached to the phenyl ring, or vice versa, and in some cases, both L¹⁰-Cy¹⁰ and L¹¹-W¹⁰ can be attached to the same ring, such as the phenyl ring.

In some embodiments, the compound of Formula II can have a structure of any of the following: 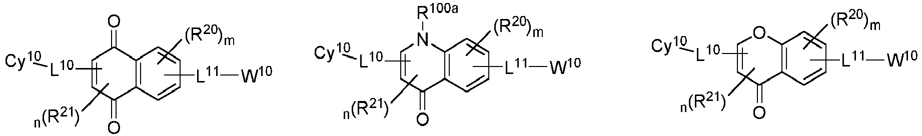 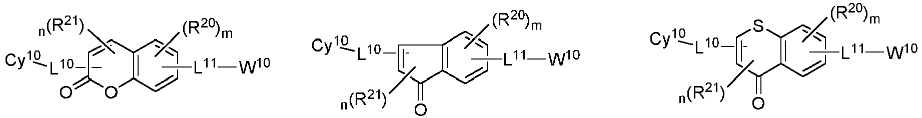 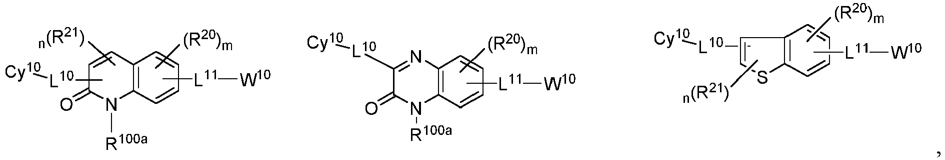 wherein: Cy¹⁰, L¹⁰, R²⁰, m, R²¹, n, R^{100a}, L¹¹, and W¹⁰ are defined herein, see e.g., Formula II and sub-formulae herein, such as Formula II-3. In some embodiments, Cy¹⁰ is Ar¹⁰ as defined for Formula II-3.

In some embodiments, the compound of Formula II-3 can have a Formula II-4: 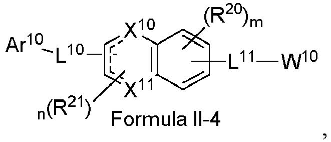
wherein: Ar¹⁰, L¹⁰, R²⁰, m, L¹¹, and W¹⁰ are defined herein, see e.g., Formula II-3,
n is 0 or 1,
R²¹ at each occurrence is independently halogen, oxo, -L^{11'}-W^{10'}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; wherein L^{11'} and W^{10'} are defined herein, see e.g., Formula II-2, and -L^{11'}-W^{10'} at each occurrence is independently selected;
X¹⁰ and X¹¹ are each independently null, -O-, -C(O)-, -S-, -NR^{100a}-, -S(O)-, -SO₂-, or - CR^{101a}R^{102a}- , as valence permits;
wherein R^{100a} is lone pair (as applicable), hydrogen, COR^{2c}, -SO₂R^{5c}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl; or R^{100a} forms an optionally substituted heterocyclic or heteroaryl ring with a R²⁰ or R²¹ group;
R^{101a} and R^{102a}, when present, are each independently hydrogen, -OH, halogen; optionally substituted C₁₋₆ alkyl, optionally substituted C₂₋₆ alkenyl, optionally substituted C₂₋₆ alkynyl, optionally substituted C₃₋₆ cycloalkyl, optionally substituted C₁₋₆ alkoxy, optionally substituted C₃₋₆ cycloalkoxy, optionally substituted amino group, optionally substituted phenyl, optionally substituted 5 or 6 membered heteroaryl, or optionally substituted 4-7 membered heterocyclyl; or R^{101a} and R^{102a}, together with the atoms they are bound to form an optionally substituted 3-7 membered cycloalkyl or heterocyclyl ring; or one of R^{101a} and R^{102a} forms an optionally substituted cycloalkyl or heterocyclyl ring together with a R²⁰ or R²¹ group; and
R^{2c} and R^{5c} are each independently hydrogen, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl;
or R²⁰ or R²¹ and L¹⁰, X¹⁰ or X¹¹, together with the atoms they are bound to form an optionally substituted cycloalkyl, heterocyclyl, aryl, or heteroaryl ring.

When X¹⁰ or X¹¹ forms a double bond with one of the ring carbons, it cannot be CR^{101a}R^{102a} with both R^{101a} and R^{102a} present, as the valence of the carbon will exceed 4. In such cases, it should be understood that one of R^{101a} and R^{102a} is absent and X¹⁰ or X¹¹ is CR^{101a} or CR^{102a} as defined herein. When X¹⁰ or X¹¹ forms a double bond with one of the ring carbons, it can be NR^{100a} with R^{100a}typically being a lone pair.

In some embodiments, the compound of Formula II has a Formula II-5: 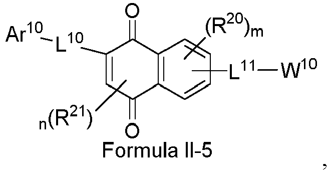 wherein: Ar¹⁰, L¹⁰, R²⁰, m, R²¹, n, L¹¹, and W¹⁰ are defined herein, see e.g., Formula II-4.

The Cy¹⁰ and Cy¹¹ in Formula II (e.g., sub-formulae described herein, such as Formula II-1 to II-4) can be connected directly or via various groups. For example, in some embodiments, L¹⁰ in Formula II (e.g., Formula 11-1 to II-5) is null, -C(O)-, optionally substituted C₁₋₄ alkylene, optionally substituted C₂₋₄ alkenylene, optionally substituted C₃₋₆ cycloalkylene, optionally substituted 4-7 membered heterocyclylene, optionally substituted phenylene, optionally substituted 5 or 6 membered heteroarylene, -O-, -S-, -NR¹⁰⁰-, -S(O)-, - SO₂-, -X¹-G¹-, -X²-G²-X^{2a}-, -X¹²-G¹⁰-, -X¹³-G¹¹-X^{13a}-, or -CR¹⁰¹R¹⁰²-,
wherein:
X¹, X², and X^{2a} are independently optionally substituted C₁₋₄ alkylene, optionally substituted C₂₋₄ alkenylene, optionally substituted C₃₋₆ cycloalkylene, optionally substituted 4-7 membered heterocyclylene, optionally substituted phenylene, optionally substituted 5 or 6 membered heteroarylene, -O-, -C(O)-, -S-, -NR^{100a}-, -S(O)-, -SO₂-, or -CR^{101a}R^{102a}-;
G¹ and G² are independently optionally substituted C₁₋₄ alkylene, optionally substituted C₂₋₄ alkenylene, optionally substituted C₃₋₆ cycloalkylene, optionally substituted 4-7 membered heterocyclylene, optionally substituted phenylene, optionally substituted 5 or 6 membered heteroarylene, -C(O)-, -NR^{100a}-, -S(O)-, -SO₂-, or -CR^{101a}R^{102a}-;
preferably, in some embodiments, -X¹-G¹- or -X²-G²-X^{2a}- does not contain an O-N, S-S, S-N (except SO₂-N bond), or -C(O)-S bond;
X¹², X¹³, and X^{13a} are independently optionally substituted C₁₋₄ alkylene, optionally substituted C₂₋₄ alkenylene, optionally substituted C₃₋₆ cycloalkylene, optionally substituted 4-7 membered heterocyclylene, optionally substituted phenylene, optionally substituted 5 or 6 membered heteroarylene, -O-, -C(O)-, -S-, -NR^{100a}-, -S(O)-, -SO₂-, or -CR^{101a}R^{102a}-; and G¹⁰ and G¹¹ are independently -X¹-G¹- or -X²-G²-X^{2a}-;
in some embodiments, preferably, -X¹²-G¹⁰- or -X¹³-G¹¹-X^{13a}- does not contain an O-O, O-N, S-S, S-N (except SO₂-N bond), or -C(O)-S bond or three (or more) consecutive heteroatoms, with the exception of O-SO₂-O, O-SO₂-N, and N-SO₂-N;
R¹⁰⁰ and R^{100a} are each independently lone pair (as applicable), hydrogen, COR^{2c}, - SO₂R^{5c}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl;
R¹⁰¹, R^{101a}, R¹⁰², and R^{102a} are each independently hydrogen, -OH, halogen; optionally substituted C₁₋₆ alkyl, optionally substituted C₂₋₆ alkenyl, optionally substituted C₂₋₆ alkynyl, optionally substituted C₃₋₆ cycloalkyl, optionally substituted C₁₋₆ alkoxy, optionally substituted C₃₋₆ cycloalkoxy, optionally substituted amino group, optionally substituted phenyl, optionally substituted 5 or 6 membered heteroaryl, or optionally substituted 4-7 membered heterocyclyl; or R¹⁰¹ and R¹⁰², or R^{101a} and R^{102a}, together with the atoms they are bound to form an optionally substituted 3-7 membered cycloalkyl or heterocyclyl ring.

In some embodiments, L¹⁰ in Formula II can be null, and Cy¹⁰ is directly linked with Cy¹¹. In some embodiments, L¹⁰ in Formula II can be null, -O-, -C(O)-, -S-, -NR¹⁰⁰-, -S(O)-, -SO₂-, or -CR¹⁰¹R¹⁰²-. In some embodiments, L¹⁰ in Formula II can be -X¹-G¹- or -X²-G²-X^{2a}-, wherein: X¹, X², and X^{2a} are independently -O-, -C(O)-, -S-, -NR^{100a}-, -S(O)-, -SO₂-, or -CR^{101a}R^{102a}-; and G¹ and G² are independently -C(O)-, -NR^{100a}-, -S(O)-, -SO₂-, or - CR^{101a}R^{102a}-.

In some embodiments, L¹⁰ in Formula II can be -X¹²-G¹⁰-. In some embodiments, X¹² is optionally substituted C₂₋₄ alkenylene, preferably, 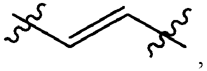 and G¹⁰ is -X¹-G¹- or - X²-G²-X^{2a}-; wherein: X¹, X², and X^{2a} are independently -O-, -C(O)-, -S-, -NR^{100a}-, _S(O)-,-SO₂-, or -CR^{101a}R^{102a}-; and G¹ and G² are independently -C(O)-, -NR^{100a}-, -S(O)-, -SO₂-, or - CR^{101a}R^{102a}-.

In some preferred embodiments, L¹⁰ in Formula II can be

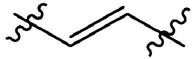

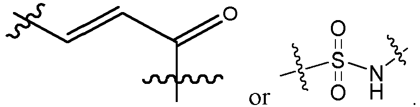

In some embodiments, the compound of Formula II can have the following core structure: 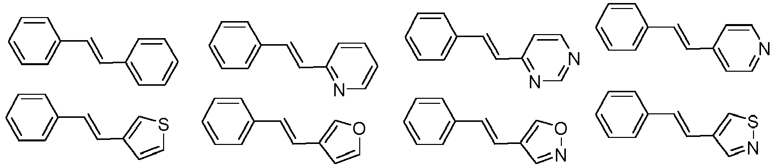 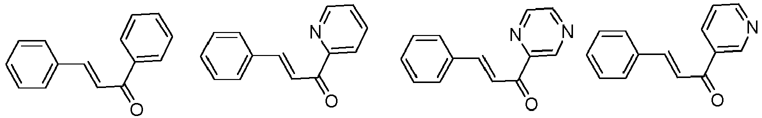 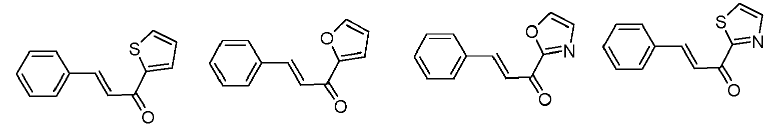 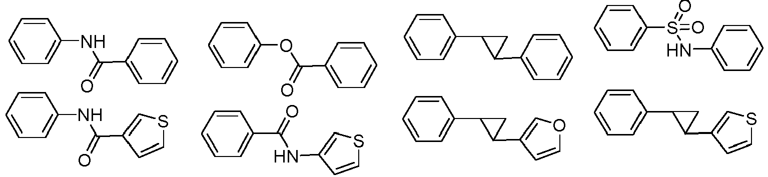 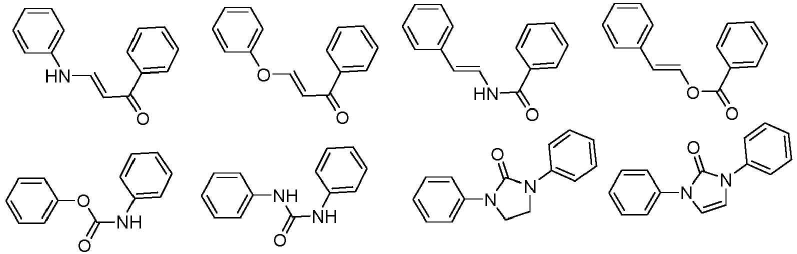 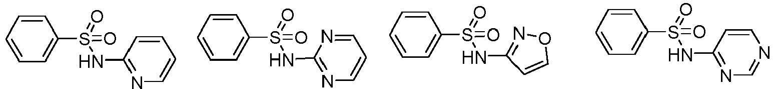 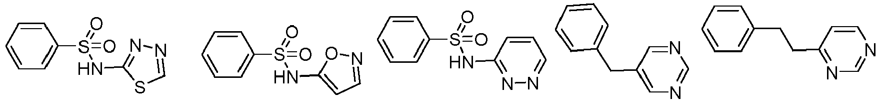 wherein L¹¹-W¹⁰ can be attached to either of the rings, preferably to one of the two pheny rings or the sole phenyl ring, wherein each of the rings can be optionally substituted with one or more suitable substituents described herein, for example, each substituent can be independently selected from F; Cl; -OH; -NH₂; -SO₂NH₂; -SO₂NH(C₁₋₄ alkyl); - SO₂NH(C₁₋₄ alkanoyl); -COOH; 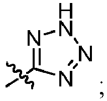 -C(O)(O-C₁₋₁₀ alkyl), -C(O)(O-C₂₋₁₀ alkenyl), - OC(O)NH₂; -OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine; optionally substituted C₃₋₆ cycloalkyl; optionally substituted 4-10 membered heterocyclyl; optionally substituted 5-10 membered heteroaryl; or optionally substituted C₆₋₁₀ aryl. For example, in some embodiments, the L¹¹-W¹⁰ is NH₂ or NH(C₁₋₄ alkanoyl), which is connected to one of the two phenyl rings or the sole phenyl ring, whereas the other ring is optionally substituted with 1 or 2 substituents selected from methyl and methoxy.

In some particular embodiments, the compound of Formula II has a formula according to Formula II-6 or II-7: 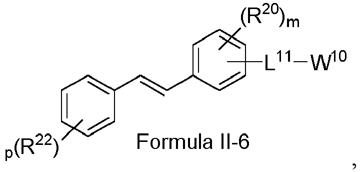 or 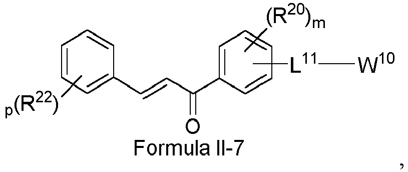
wherein: L¹¹, W¹⁰, R²⁰, and m are defined herein, see e.g., Formula II-3,
p is 0, 1, 2, 3, or 4,
R²² at each occurrence is independently halogen, -L^{11'}-W^{10'}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or two adjacent R²² together with the atoms they are bound to form an optionally substituted cycloalkyl, heterocyclyl, aryl, or heteroaryl ring;
wherein L^{11'} and W^{10'} are defined herein, see e.g., Formula II-2, and -L^{11'}-W^{10'} at each occurrence is independently selected.

L¹¹ in Formula II (e.g., any of the sub-formulae, such as Formula II-1 to II-7) is typically null, i.e., the W¹⁰ group is directly attached to Cy¹¹, as applicable. In some embodiments, L¹¹ in Formula II can also be a C₁₋₄ alkylene, C₂₋₄ alkenylene, C₂₋₄ alkynylene or C₁₋₄ heteroalkylene. For example, the W¹⁰ group can be attached to Cy¹¹ through a methylene or vinyl group.

Various W¹⁰ groups are suitable for compounds of Formula II (e.g., Formula II-1 to II-7). In preferred embodiments, W¹⁰ group at each occurrence is independently -OH, -NH₂, - SO₂NH₂, -SO₂NH(C₁₋₄ alkyl); -SO₂NH(C₁₋₄ alkanoyl), -COOH, 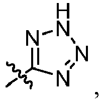 -C(O)(O-C₁₋₁₀ alkyl), -C(O)(O-C₂₋₁₀ alkenyl), -OC(O)NH₂, -OC(O)NH(C₁₋₄ alkyl)-, -O-(CO)-(C₁₋₄ alkyl), - O-(C₁₋₄ alkyl), wherein each of the C₁₋₄ alkyl is independently optionally substituted with 1-3 substituents independently selected from C₁₋₄ alkyl, C₁₋₄ alkoxy, -OH, -NH₂, and fluorine. In some embodiments, W¹⁰ group in Formula II is -OH, -OMe, -NH₂, -SO₂NH₂, - SO₂NH(Acetyl), -COOH, 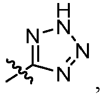 or -O-C(O)-CH₃.

As described herein, L^{11'}-W^{10'} can in some embodiments be selected as a substituent for Cy¹⁰ or Cy¹¹, such as for Ar¹⁰ or Ar¹¹. When applicable, L^{11'} in Formula II, including any of the sub-formulae described herein, such as Formula II-1 to I-7, at each occurrence can be independently null, i.e., the W^{10'} group is directly attached to Cy¹⁰ or Cy¹¹, such as for Ar¹⁰ or Ar¹¹, as applicable, or a C₁₋₄ alkylene, C₂₋₄ alkenylene, C₂₋₄ alkynylene or C₁₋₄ heteroalkylene. For example, the W^{10'} group can be attached to Cy¹⁰ or Cy¹¹, such as for Ar¹⁰ or Ar¹¹, as applicable, through a methylene or vinyl group. When applicable, W^{10'} in Formula II, including any of the sub-formulae described herein, such as Formula II-1 to II-7, at each occurrence can be independently -OH, -NH₂, -SO₂NH₂, -SO₂NH(C₁₋₄ alkyl), -SO₂NH(C₁₋₄ alkanoyl), -COOH, 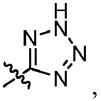 -C(O)(O-C₁₋₁₀ alkyl), -C(O)(O-C₂₋₁₀ alkenyl), -OC(O)NH₂, - OC(O)NH(C₁₋₄ alkyl)-, -O-(CO)-(C₁₋₄ alkyl), -O-(C₁₋₄ alkyl), wherein each of the C₁₋₄ alkyl is independently optionally substituted with 1-3 substituents independently selected from C₁₋₄ alkyl, C₁₋₄ alkoxy, -OH, -NH₂, and fluorine. In some embodiments, each instance of W^{10'} in Formula II, when applicable, can be -OH, -OMe, -NH₂, -SO₂NH₂, -SO₂NH(Acetyl), -COOH, or -O-C(O)-CH₃.

Various groups can be suitable for R²⁰, R²¹, and R²² in any of the applicable Formula II (e.g., Formula II-1 to II-7, as applicable). In some embodiments, each of R²⁰, R²¹, and R²² at each occurrence can be independently F; Cl; -OH; -NH₂; -SO₂NH₂; -SO₂NH(C₁₋₄ alkyl); - SO₂NH(C₁₋₄ alkanoyl); -COOH; 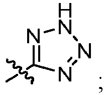 -C(O)(O-C₁₋₁₀ alkyl), -C(O)(O-C₂₋₁₀ alkenyl), - OC(O)NH₂; -OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine. In some embodiments, each of R²⁰, R²¹, and R²² at each occurrence can be independently F; Cl; -OH; -NH₂, -SO₂NH₂, -SO₂NH(C₁₋₄ alkyl), -SO₂NH(C₁₋₄ alkanoyl), -COOH; 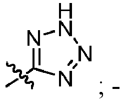 C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); -O-(C₁₋₆ alkyl); -O-(C₂₋₆ alkenyl); C₁₋₆ alkyl optionally substituted with 1-3 substituents independently selected from C₁₋₄ alkyl, C₁₋₆ alkoxy, -OH, -NH₂, and fluorine; or C₂₋₆ alkenyl optionally substituted with 1-3 substituents independently selected from C₁₋₄ alkyl, C₁₋₆ alkoxy, -OH, -NH₂, and fluorine. In some embodiments, each of R²⁰, R²¹, and R²² at each occurrence can be independently -OH, C₁₋₄ alkyl, C₂₋₆ alkenyl, or -O-(C₁₋₄ alkyl). In some embodiments, each of R²⁰, R²¹, and R²² at each occurrence can be independently -OH, - OMe, or 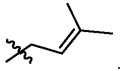 In some embodiments, one or more instances of R²⁰, one or more instances of R²¹, and/or one or more instances of R²² can be independently selected L^{11'}-W^{10'} as described herein.

Typically, m and p, as applicable, is 0, 1, 2, or 3; preferably, 1 or 2.

Typically, n, as applicable, is 0, 1, or 2; preferably, 0 or 1.

In some embodiments, the compound of Formula II can have a formula according to any of Formula 11-8 to II-10: 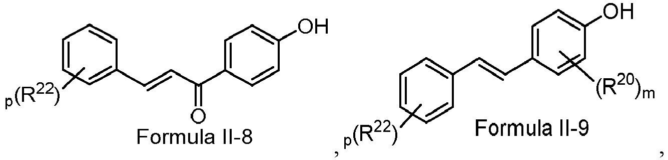 or 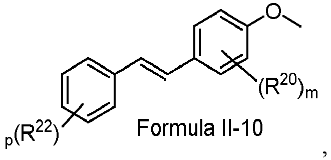 wherein R²⁰, R²², m, and p are defined herein. In some embodiments, m is 1 or 2, p is 1, 2, or 3. In some embodiments, each of R²⁰ and R²² at each occurrence is independently F; Cl; - OH; -NH₂, -SO₂NH₂, -SO₂NH(C₁₋₄ alkyl), -SO₂NH(C₁₋₄ alkanoyl), -COOH; 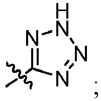 -C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); - O-(C₁₋₆ alkyl); -O-(C₂₋₆ alkenyl); C₁₋₆ alkyl optionally substituted with 1-3 substituents independently selected from C₁₋₄ alkyl, C₁₋₆ alkoxy, -OH, -NH₂, and fluorine; or C₂₋₆ alkenyl optionally substituted with 1-3 substituents independently selected from C₁₋₄ alkyl, C₁₋₆ alkoxy, - OH, -NH₂, and fluorine.

In some embodiments, the structural unit 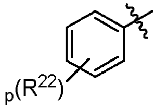 in any of the applicable Formula II can be selected from

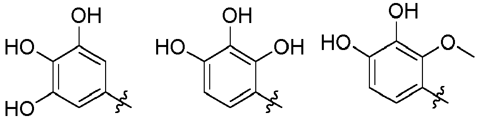

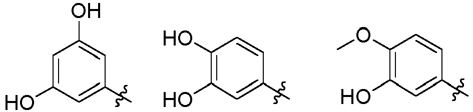

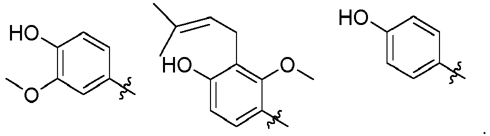

In some embodiments, the compound of Formula II can also have a formula according to any of Formula II-11 to Formula 11-14: 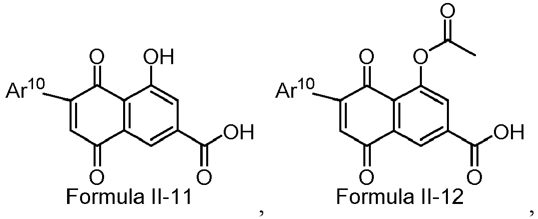 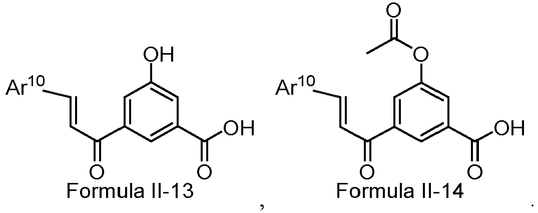 wherein Ar¹⁰ is defined herein. For example, in some embodiments, Ar¹⁰ is a phenyl optionally substituted with 1-4 substituents independently selected from F; Cl; -OH; -NH₂, -SO₂NH₂, - SO₂NH(C₁₋₄ alkyl), -SO₂NH(C₁₋₄ alkanoyl), -COOH; 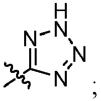 -C(O)(O-C₁₋₁₀ alkyl), -C(O)(O-C₂₋₁₀ alkenyl), -OC(O)NH₂; -OC(O)NH(C₁₋₄ alkyl)-; -O-(CO)-(C₁₋₄ alkyl); -O-(C₁₋₆ alkyl); -O-(C₂₋₆ alkenyl); C₁₋₆ alkyl optionally substituted with 1-3 substituents independently selected from C₁₋₄ alkyl, C₁₋₆ alkoxy, -OH, -NH₂, and fluorine; or C₂₋₆ alkenyl optionally substituted with 1-3 substituents independently selected from C₁₋₄ alkyl, C₁₋₆ alkoxy, -OH, -NH₂, and fluorine.

In some specific embodiments, the compound of Formula II can be: 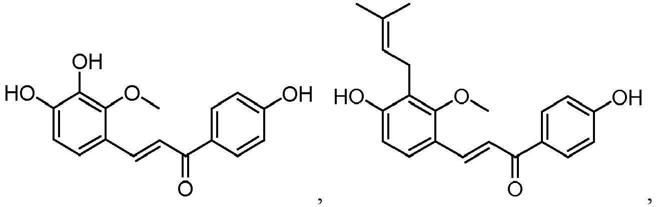 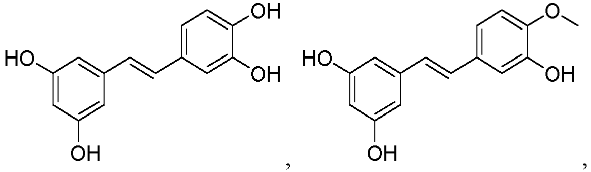 or 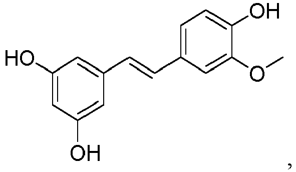 or a pharmaceutically acceptable salt or ester thereof.

In some embodiments, the compounds herein can be characterized by having a Formula III, or a pharmaceutically acceptable salt or ester thereof:

Ar²⁰-L²⁰-W²⁰ Formula III

wherein Ar²⁰ is an optionally substituted aryl ring (e.g., C₆₋₁₀ aryl ring), or an optionally substituted heteroaryl ring (e.g., 5-10 membered heteroaryl ring);
L²⁰ is null, an optionally substituted C₁₋₆ alkylene, an optionally substituted C₁₋₆ heteroalkylene, an optionally substituted C₂₋₆ alkenylene, an optionally substituted C₂₋₆ alkynylene, an optionally substituted C₃₋₆ cycloalkylene, an optionally substituted arylene, an optionally substituted heteroarylene, or an optionally substituted 4-7 membered heterocyclylene,
W²⁰ is -OR¹; -COR²; -COOR^{1a}; -OCOOR^{1a}; -NR³R⁴; -CONR^{3a}R^{4a}; -OCONR^{3b}R^{4b}; - SO₂NR^{3c}R^{4c}; -OSO₂NR^{3d}R^{4d}; -SR⁵; -SO₂R^{5a}; -OCOR^{2a}; -OSO₂R^{5a}; or 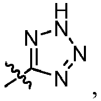
wherein:
   R¹ and R^{1a} are each independently hydrogen, optionally substituted alkyl, optionally substituted alkenyl, optionally substituted alkynyl, optionally substituted cycloalkyl, optionally substituted aryl, optionally substituted heteroaryl, or optionally substituted heterocyclyl;
   R³ and R⁴ are each independently hydrogen, -COR^{2b}, -SO₂R^{5b}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl, or R³ and R⁴ together with the atoms they are bound to form an optionally substituted 4-7 membered heterocyclyl;
   R², R^{2a}, R^{2b}, R⁵, R^{5a}, and R^{5b} are each independently hydrogen, -OH, -NR^{3e}R^{4e}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; and
   R^{3a}, R^{3b}, R^{3c}, R^{3d}, R^{3e}, R^{4a}, R^{4b}, R^{4c}, R^{4d}, and R^{4e} are each independently hydrogen, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or R^{3a} and R^{4a}, R^{3b} and R^{4b}, R^{3c} and R^{4c}, R^{3d} and R^{4d}, or R^{3e} and R^{4e}, together with the atoms they are bound to form an optionally substituted 4-7 membered heterocyclyl.

In some embodiments, Ar²⁰ in Formula III is an optionally substituted phenyl ring or an optionally substituted 5 or 6 membered heteroaryl ring. For example, in some embodiments, Ar²⁰ in Formula III can be an optionally substituted phenyl ring, an optionally substituted thienyl ring, an optionally substituted furanyl ring, an optionally substituted pyridyl ring, or an optionally substituted pyrimidinyl ring. In some embodiments, Ar²⁰ in Formula III can also be an optionally substituted bicyclic aryl or bicyclic heteroaryl ring, each of which is optionally substituted. In such embodiments, L²⁰-W²⁰ can be attached to either of the bicyclic rings.

In some embodiments, Ar²⁰ in Formula III can be an optionally substituted phenyl ring, wherein two adjacent substituents together with the carbon they are attached to form an optionally substituted cycloalkyl, heterocyclyl, aryl, or heteroaryl ring.

For example, in some embodiments, Ar²⁰ in Formula III can be a benzofused bicyclic aryl or heteroaryl ring. For example, in some embodiments, Ar²⁰ in Formula III can have the following structure: 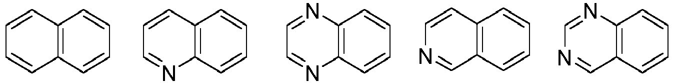 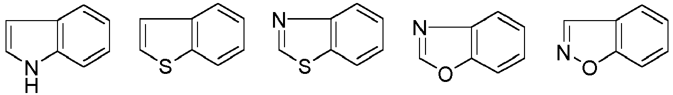 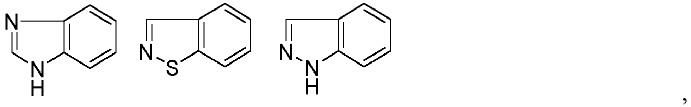 wherein-L²⁰-W²⁰ can be attached at either of the two rings, wherein either or both of the rings can be optionally substituted.

In some embodiments, the compound of Formula III can have a Formula III-1, III-2, or III-3: 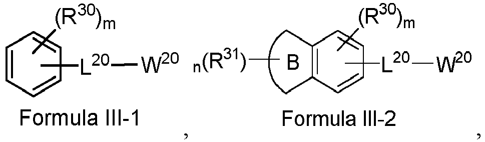 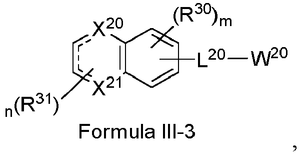
wherein L²⁰ and W²⁰ are defined herein,
m is 0, 1, 2, or 3; n is 0, 1, 2, or 3;
each of R³⁰ and R³¹ at each occurrence is independently halogen, -L^{20'}-W^{20'}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; wherein -L^{20'}-W^{20'} at each occurrence is independently selected; wherein L^{20'} at each occurrence is independently null, an optionally substituted C₁₋₆ alkylene, an optionally substituted C₁₋₆ heteroalkylene, an optionally substituted C₂₋₆ alkenylene, an optionally substituted C₂₋₆ alkynylene, an optionally substituted C₃₋₆ cycloalkylene, an optionally substituted arylene, an optionally substituted heteroarylene, or an optionally substituted 4-7 membered heterocyclylene; and W^{20'} at each occurrence is independently -OR¹; -COR²; - COOR^{1a}; -OCOOR^{1a}; -NR³R⁴; -CONR^{3a}R^{4a}; -OCONR^{3b}R^{4b}; -SO₂NR^{3c}R^{4c}; -OSO₂NR^{3d}R^{4d}; - SR⁵; -SO₂R^{5a}; -OCOR^{2a}; -OSO₂R^{5a} or 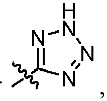 wherein R¹, R^{1a}, R², R^{2a}, R^{2b}, R³, R⁴, R^{3a}, R^{3b}, R^{3c}, R^{3d}, R^{3e}, R^{4a}, R^{4b}, R^{4c}, R^{4d}, R^{4e}, R⁵, R^{5a}, and R^{5b} are defined herein, see e.g., Formula III,
ring B is a 4-7 membered cycloalkyl ring, 4-7 membered heterocyclic ring, phenyl ring, 5 or 6 membered heteroaryl ring, each of which is optionally substituted 1-3 independently selected R³¹;
X²⁰ and X²¹ are each independently null, -O-, -C(O)-, -S-, -NR^{100a}-, -S(O)-, -SO₂-, or - CR^{101a}R^{102a}-, as valence permits;
wherein R^{100a} is lone pair (as applicable), hydrogen, COR^{2c}, -SO₂R^{5c}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl; or R^{100a} and one of R³⁰ or R³¹, together with the atoms they are bound to, form an optionally substituted heterocyclic or heteroaryl ring, e.g., optionally substituted 5 or 6 membered heteroaryl, or optionally substituted 4-7 membered heterocyclyl;
R^{101a} and R^{102a}, when present, are each independently hydrogen, -OH, halogen; optionally substituted C₁₋₆ alkyl, optionally substituted C₂₋₆ alkenyl, optionally substituted C₂₋₆ alkynyl, optionally substituted C₃₋₆ cycloalkyl, optionally substituted C₁₋₆ alkoxy, optionally substituted C₃₋₆ cycloalkoxy, optionally substituted amino group, optionally substituted phenyl, optionally substituted 5 or 6 membered heteroaryl, or optionally substituted 4-7 membered heterocyclyl, or R^{101a} and R^{102a}, together with the atoms they are bound to form an optionally substituted 3-7 membered cycloalkyl or heterocyclyl ring; or one of R^{101a} and R^{102a} forms an optionally substituted cycloalkyl or heterocyclyl ring together with a R³⁰ or R³¹ group; and
R^{2c} and R^{5c} are each independently hydrogen, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl;
or two adjacent R³⁰ or two adjacent R³¹, or R³⁰ or R³¹ and X²⁰ or X²¹, together with the atoms they are bound to form an optionally substituted cycloalkyl, heterocyclyl, aryl, or heteroaryl ring.

When X²⁰ or X²¹ forms a double bond with one of the ring carbons, it cannot be CR^{101a}R^{102a} with both R^{101a} and R^{102a} present, as the valence of the carbon will exceed 4. In such cases, it should be understood that one of R^{101a} and R^{102a} is absent and X²⁰ or X²¹ is CR^{101a} or CR^{102a} as defined herein. When X²⁰ or X²¹ forms a double bond with one of the ring carbons, it can be N^{R100a} with R^{100a} typically being a lone pair.

It should be noted that each instance of the structural unit -L^{20'}-W^{20'} and -L²⁰-W²⁰ are independently selected and can be the same or different.

In some embodiments, the compound of Formula III can have a structure of any of the following: 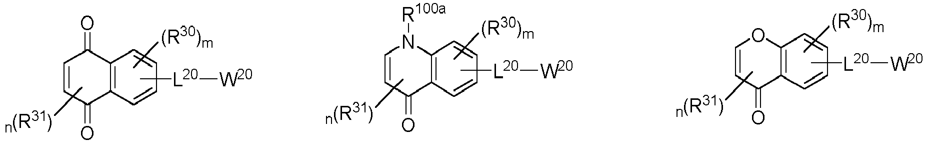 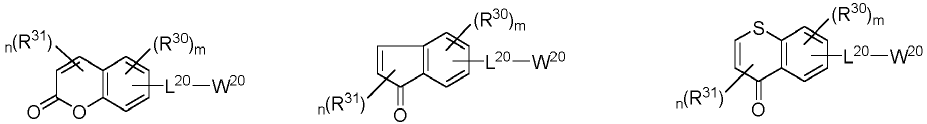 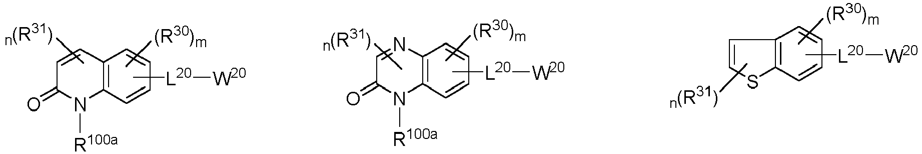 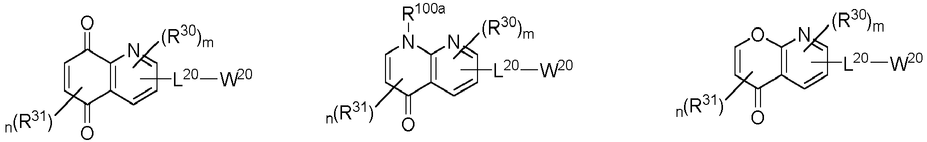 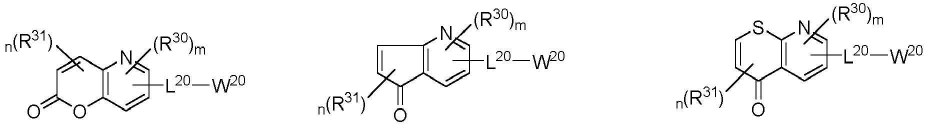 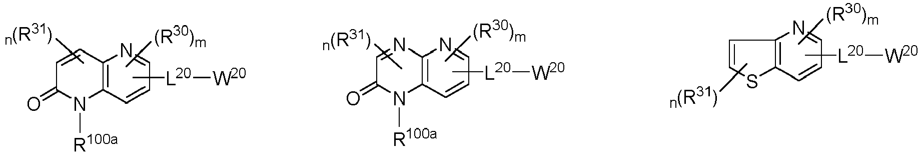 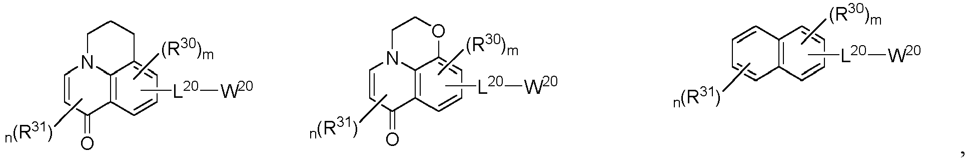 wherein: R³⁰, m, R³¹, n, R^{100a}, L²⁰, and W²⁰ are defined herein, see e.g., Formula III and sub-formulae herein, such as Formula III-1 to III-3, wherein for the tricyclic structures, the piperidine ring or the morpholine ring can be optionally substituted.

L²⁰ in Formula III (e.g., any of the sub-formulae such as Formula III-1 to III-3) is typically null, i.e., the W²⁰ group is directly attached to Ar²⁰. In some embodiments, L²⁰ in Formula III can also be a C₁₋₄ alkylene, C₂₋₄ alkenylene, C₂₋₄ alkynylene or C₁₋₄ heteroalkylene. For example, the W²⁰ group can be attached to Ar²⁰, through a methylene or a vinyl group.

Various W²⁰ groups are suitable for compounds of Formula III (e.g., any of the sub-formulae such as Formula III-1 to III-3). In preferred embodiments, W²⁰ in Formula III can be -OH, -COOH, 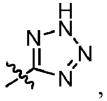 -C(O)(O-C₁₋₁₀ alkyl), -C(O)(O-C₂₋₁₀ alkenyl),-OC(O)NH₂, -NH₂, -SO₂NH₂, -SO₂NH(C₁₋₄ alkyl); -SO₂NH(C₁₋₄ alkanoyl), -OC(O)NH(C₁₋₄ alkyl)-, -O-(CO)-(C₁₋₄ alkyl), -O-(C₁₋₄ alkyl), wherein each of the C₁₋₄ alkyl is independently optionally substituted with 1-3 substituents independently selected from C₁₋₄ alkyl, C₁₋₄ alkoxy, -OH, -NH₂, and fluorine. In some embodiments, W²⁰ group in Formula III (e.g., any of the sub-formulae such as Formula III-1 to III-3) is -OH, -NH₂, -SO₂NH₂, -SO₂NH(Acetyl), 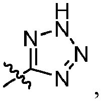 -C(O)-(O-C₈ alkyl), -COOH, or -O-C(O)-CH₃.

As described herein, L^{20'}-W^{20'} can in some embodiments be selected as a substituent for Ar²⁰. When applicable, L^{20'} in Formula III, including any of the sub-formulae described herein, such as Formula III-1 to III-3, at each occurrence can be independently null, i.e., the W^{20'} group is directly attached to Ar²⁰, as applicable, or a C₁₋₄ alkylene, C₂₋₄ alkenylene, C₂₋₄ alkynylene or C₁₋₄ heteroalkylene. For example, the W^{20'} group can be attached to Ar²⁰, as applicable, through a methylene or vinyl group. When applicable, W^{20'} in Formula III, including any of the sub-formulae described herein, such as Formula III-1 to III-3, at each occurrence can be independently -OH, -COOH, 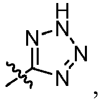 -C(O)(O-C₁₋₁₀ alkyl), -C(O)(O-C₂₋₁₀ alkenyl),-OC(O)NH₂, -NH₂, -SO₂NH₂, -SO₂NH(C₁₋₄ alkyl); -SO₂NH(C₁₋₄ alkanoyl), - OC(O)NH(C₁₋₄ alkyl)-, -O-(CO)-(C₁₋₄ alkyl), -O-(C₁₋₄ alkyl), wherein each of the C₁₋₄ alkyl is independently optionally substituted with 1-3 substituents independently selected from C₁₋₄ alkyl, C₁₋₄ alkoxy, -OH, -NH₂, and fluorine. In some embodiments, each instance of W^{20'} in Formula III, when applicable, can be -OH, -NH₂, -SO₂NH₂, -SO₂NH(Acetyl), 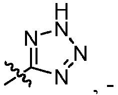 COOH, -C(O)(O-Cs alkyl) or -O-C(O)-CH₃.

Various groups can be suitable for R³⁰ and R³¹ in any of the applicable Formula III (e.g., any of the sub-formulae such as Formula III-1 to III-3). In some embodiments, each of R³⁰ and R³¹ at each occurrence can be independently F; Cl; -OH; -COOH; -OC(O)NH₂; - OC(O)NH(C₁₋₄ alkyl)-; -O-(CO)-(C₁₋₄ alkyl); C₁₋₄ alkyl optionally substituted with 1-3 substituents independently selected from C₁₋₄ alkyl, C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine. In some embodiments, each of R³⁰ and R³¹ at each occurrence can be independently -OH, C₂₋₆ alkenyl, -O-(C₁₋₄ alkyl), -COOH, or -C(O)(O-C₁₋₁₀ alkyl). In some embodiments, each of R³⁰ and R³¹ at each occurrence can be -OH or -OMe. In some embodiments, one or more instances of R³⁰ and/or one or more instances of R³¹ can be independently selected L^{20'}-W^{20'} as described herein.

Typically, m is 0, 1, 2, or 3; preferably, 2 or 3. Typically, n is 1, 2 or 3.

The present invention provides the following compound, 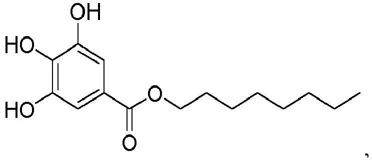 a pharmaceutically acceptable salt or ester thereof.

In some embodiments, the present disclosure also provides the following compound, 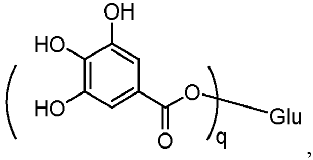 a pharmaceutically acceptable salt or ester thereof, wherein q is 1, 2, 3, 4, or 5, and Glu is a residue of glucose. In some specific embodiments, the present disclosure also provides 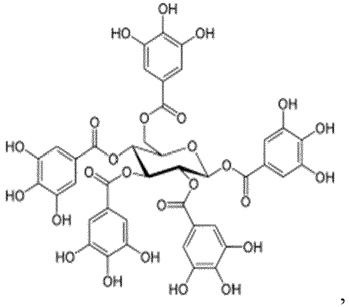 a pharmaceutically acceptable salt or ester thereof.

In some embodiments, the compounds herein can also be an alkaloid having antibacterial activity. As shown herein, certain indole alkaloids, such as *Vinca* alkaloids, tabersonine, vindoline, vinblastine, vincristine, etc., are shown to be effective in killing the microorganisms such as *B. megaterium.* In some embodiments, the compounds herein are characterized by Formula IV-1 or IV-2, which are tabersonine or vindoline and derivatives: 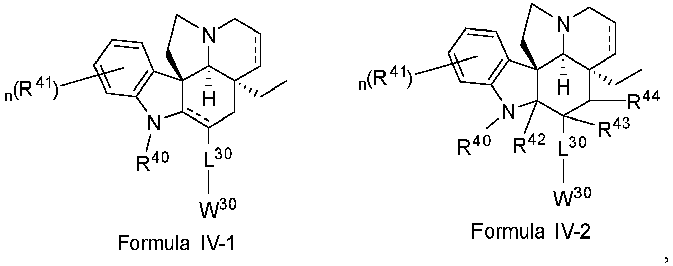 wherein:
R⁴⁰ is hydrogen; -COR²; -COOR^{1a}; -SO₂R^{5a}; optionally substituted alkyl, optionally substituted alkenyl, optionally substituted alkynyl, optionally substituted cycloalkyl, optionally substituted aryl, optionally substituted heteroaryl, or optionally substituted heterocyclyl;
R⁴¹ is -OR¹; -OCOOR^{1a}; -OCONR^{3b}R^{4b}; -OCOR^{2a}; or -OSO₂R^{5a}; n is 0 or 1;
R⁴², R⁴³, and R⁴⁴ are each independently hydrogen, -OR¹, OCOR^{2a}; or -OSO₂R^{5a} ;
L³⁰ is null or methylene,
W³⁰ is -OR¹; -COR²; -COOR^{1a}; -OCOOR^{1a}; -NR³R⁴; -CONR^{3a}R^{4a}; -OCONR^{3b}R^{4b}; - OSO₂NR^{3d}R^{4d}; -OCOR^{2a}; or -OSO₂R^{5a}
wherein:
   R¹ and R^{1a} are each independently hydrogen, optionally substituted alkyl, optionally substituted alkenyl, optionally substituted alkynyl, optionally substituted cycloalkyl, optionally substituted aryl, optionally substituted heteroaryl, or optionally substituted heterocyclyl;
   R³ and R⁴ are each independently hydrogen, -COR^{2b}, -SO₂R^{5b}, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl, or R³ and R⁴ together with the atoms they are bound to form an optionally substituted 4-7 membered heterocyclyl;
   R², R^{2a}, R^{2b}, R⁵, R^{5a}, and R^{5b} are each independently hydrogen, -OH, -NR^{3e}R^{4e}, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; and
   R^{3a}, R^{3b}, R^{3c}, R^{3d}, R^{3e}, R^{4a}, R^{4b}, R^{4c}, R^{4d}, and R^{4e} are each independently hydrogen, an optionally substituted C₁₋₆ alkyl, an optionally substituted C₂₋₆ alkenyl, an optionally substituted C₂₋₆ alkynyl, an optionally substituted C₁₋₆ alkoxy, an optionally substituted C₃₋₆ cycloalkyl, an optionally substituted C₃₋₆ cycloalkoxy, an optionally substituted phenyl; an optionally substituted 5 or 6 membered heteroaryl; or an optionally substituted 4-7 membered heterocyclyl; or R^{3a} and R^{4a}, R^{3b} and R^{4b}, R^{3c} and R^{4c}, R^{3d} and R^{4d}, or R^{3e} and R^{4e}, together with the atoms they are bound to form an optionally substituted 4-7 membered heterocyclyl.

In some embodiments, the compound of Formula IV-1 or IV-2 has a formula according to one of Formula IV-3 to IV-6: 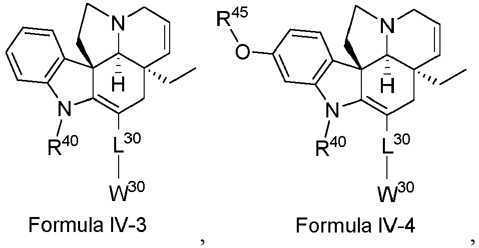 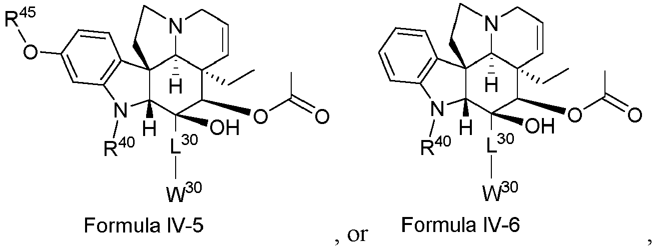 wherein R⁴⁵ is hydrogen or methyl.

In some embodiments, R⁴⁰ in any of the Formula IV-1 to IV-6 can be hydrogen, C₁₋₄ alkyl, or C₁₋₄ alkanoyl.

L³⁰ in Formula IV-1 to IV-6 is typically null. However, in some embodiments, L³⁰ in Formula IV-1 to IV-6 can also be CH₂.

W³⁰ in Formula IV-1 to IV-6 is typically a carboxylic acid derivative, an amine derivative or an alcohol derivative, which are useful for the compositions and methods herein. The naturally occurring indole alkaloid tabersonine contains a CO₂Me group as W³⁰, with L³⁰ being null. The CO₂Me group can be transformed into the corresponding acid, amide etc. via routine transformations, or it can be reduced or transformed into an amine through a rearrangement such as Curtius rearrangement. In some embodiments, W³⁰ in Formula IV-1 to IV-6 can be -OH, -NH₂, -OSO₂NH₂, -COOH, -C(O)(O-C₁₋₁₀ alkyl), -C(O)(O-C₂₋₁₀ alkenyl), -OC(O)NH₂, -OC(O)NH(C₁₋₄ alkyl)-, -O-(CO)-(C₁₋₄ alkyl), -O-(C₁₋₄ alkyl), wherein each of the C₁₋₄ alkyl is independently optionally substituted with 1-3 substituents independently selected from C₁₋₄ alkyl, C₁₋₄ alkoxy, -OH, -NH₂, and fluorine. In some embodiments, W³⁰ in Formula IV-1 to IV-6 can be -OH, -NH₂, -OSO₂NH₂, -C(O)-(O-Cs alkyl), -COOH, or -OC(O)NH₂.

In some specific embodiments, the compound can have the following structure:

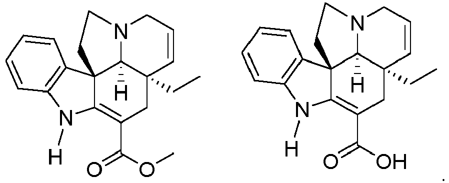

In some embodiments, the compounds herein can also be a glycoside having antibacterial activity, or a pharmaceutically acceptable salt or ester thereof. As shown herein, certain glycosides, such as ginsenosides, and gallic acid glycosides, are shown to be effective in killing the microorganisms such as *B. megaterium.* Other useful glycosides include any of those known in the art to have antibacterial activities, which can for example, include glycosides characterized by its corresponding aglyone being a phenolic compound, a flavonoid, a coumarin, a benzoic acid, or a sterol. Typically, the glycoside is a glucoside, although other glycosides can also be useful. In some embodiments, the glycosides can be characterized as amphiphilic, which can destroy biological membranes and confer antimicrobial activity to the glycosides. In some embodiments, the glycosides can also be characterized as a saponin, which for example, include various plant derived glycosides that can act as "surfactants" and can help to kill bacteria.

In some embodiments, the glycosides herein can be characterized by a Formula V: 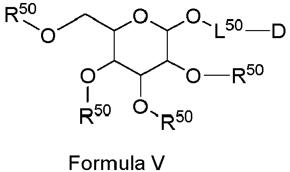
wherein each R⁵⁰ is independently hydrogen, -L⁵⁰-D, an oxygen protecting group, or a sugar residue;
L⁵⁰ is null or -C(O)-;
D is an optionally substituted aryl (e.g., C₆₋₁₀ aryl), optionally substituted heteroaryl (e.g., 5 to 14 membered heteroaryl), optionally substituted fused ring comprising two or more rings independently selected from aryl, heteroaryl, cycloalkyl and heterocyclyl (e.g., 8-14 membered, e.g., benzofused cycloalkyl/heterocyclyl, pyridofused cycloalkyl/heterocyclyl), or a steroid residue having a formula V-A:

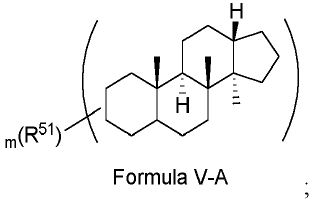

wherein 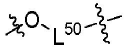 can connect to Formula V-A via the steroid backbone or any of the R⁵¹ group(s), as valence permits,
wherein R⁵¹ at each occurrence is independently optionally substituted alkyl, optionally substituted alkenyl, optionally substituted alkynyl, -OH optionally substituted with an oxygen protecting group, oxo, halogen, optionally substituted cycloalkyl, optionally substituted alkoxy, optionally substituted cycloalkoxy, optionally substituted amino group, optionally substituted phenyl, optionally substituted heteroaryl, or optionally substituted heterocyclyl, or two R⁵¹ groups together with the atoms they are bound to form an optionally substituted cycloalkyl, heterocyclyl, aryl, or heteroaryl ring;
m is an integer of 1-8; and
   wherein -L⁵⁰-D at each occurrence is independently selected.

In some embodiments, each R⁵⁰ is hydrogen.

In some embodiments, one to four R⁵⁰ can be -L⁵⁰-D which are independently selected. When two or more -L⁵⁰-D units are linked to the pyranose unit in Formula V, they are preferably the same. In some embodiments, one or more (e.g., 1 or 2) R⁵⁰ can be a sugar residue which connects to the remainder of Formula V via a glycoside bond. In some embodiments, the sugar residue is a glucose residue or a rhamnose residue.

L⁵⁰ in Formula V can be null or a carbonyl group, -C(O)-, depending on whether the linking group is a phenolic -OH or a COOH group from a benzoic acid or a heteraryl counterpart.

Various residues can be used as D, which is typically residue from a phenolic compound, a coumarin, a flavonoid, or a sterol, which in some embodiments can have antibacterial activity without the glycoside unit.

In some embodiments, D can be an optionally substituted ring selected from 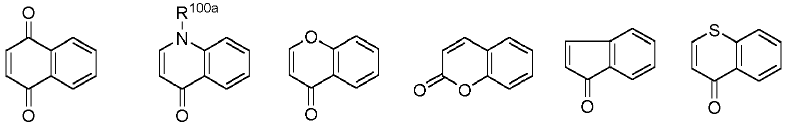 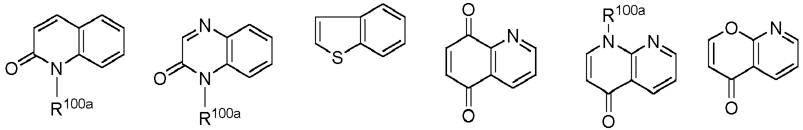 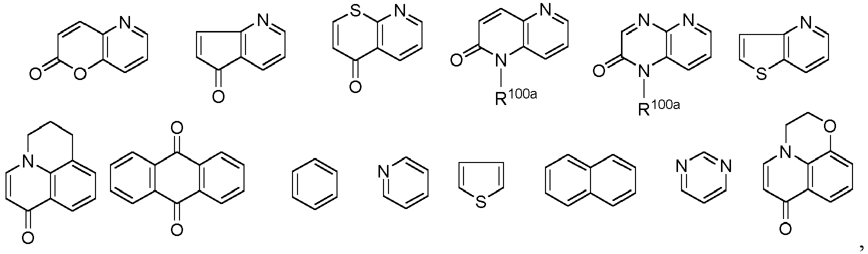 wherein
R^{100a} is lone pair (as applicable), hydrogen, nitrogen protecting group, optionally substituted C₁₋₆ alkyl, optionally substituted C₂₋₆ alkenyl, optionally substituted C₂₋₆ alkynyl, optionally substituted C₃₋₆ cycloalkyl, optionally substituted phenyl, optionally substituted 5 or 6 membered heteroaryl, or optionally substituted 4-7 membered heterocyclyl; or R^{100a} forms an optionally substituted heterocyclic or heteroaryl ring with the pheny or pyridyl ring;
wherein 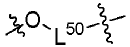 can connect to D via any of the available positions, and
each of the ring systems of D is optionally substituted with 1-5 (e.g., 1, 2, or 3) substituents each independently selected from -OH; -COOH; -C(O)(O-C₁₋₁₀ alkyl); -C(O)(O-C₂₋₁₀ alkenyl); -OC(O)NH₂; -OC(O)NH(C₁₋₄ alkyl)-; -O-(CO)-(C₁₋₄ alkyl); -NH₂; -SO₂NH₂; - SO₂NH(C₁₋₄ alkyl); -SO₂NH(C₁₋₄ alkanoyl); halogen; optionally substituted C₁₋₆ alkyl;

optionally substituted C₂₋₆ alkenyl; optionally substituted C₂₋₆ alkynyl; optionally substituted C₃₋₆ cycloalkyl; optionally substituted C₁₋₆ alkoxy; optionally substituted C₃₋₆ cycloalkoxy;
optionally substituted amino group; optionally substituted phenyl; optionally substituted 5 or 6 membered heteroaryl; or optionally substituted 4-7 membered heterocyclyl.

In some embodiments, each of the ring systems of D as shown above can be optionally substituted with 1-5 substituents each independently selected from F; Cl; -OH; -COOH; - C(O)(O-C₁₋₁₀ alkyl); -C(O)(O-C₂₋₁₀ alkenyl); -OC(O)NH₂; -OC(O)NH(C₁₋₄ alkyl)-; -O-(CO)-(C₁₋₄ alkyl); -NH₂; -SO₂NH₂; -SO₂NH(C₁₋₄ alkyl); -SO₂NH(C₁₋₄ alkanoyl); C₁₋₄ alkyl optionally substituted with 1-3 substituents independently selected from C₁₋₄ alkyl; C₁₋₄ alkoxy, -OH, -NH₂, and fluorine; C₂₋₆ alkenyl optionally substituted with 1-3 substituents independently selected from C₁₋₄ alkyl, C₁₋₄ alkoxy, -OH, -NH₂, and fluorine; C₂₋₆ alkynyl optionally substituted with 1-3 substituents independently selected from C₁₋₄ alkyl, C₁₋₄ alkoxy, -OH, -NH₂, and fluorine; C₃₋₆ cycloalkyl optionally substituted with 1-3 substituents independently selected from C₁₋₄ alkyl and fluorine; C₃₋₆ cycloalkoxy optionally substituted with 1-3 substituents independently selected from C₁₋₄ alkyl and fluorine; or C₁₋₄ alkoxy optionally substituted with 1-3 substituents independently selected from C₁₋₄ alkyl, C₁₋₄ alkoxy, -OH, -NH₂, and fluorine.

In some embodiments, D can be selected from: 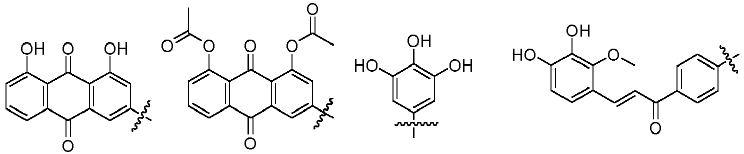 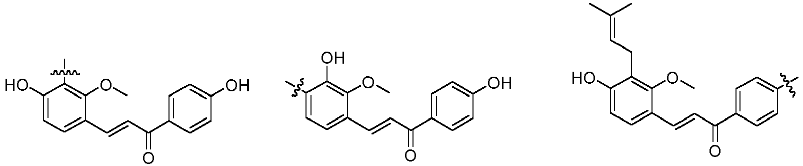 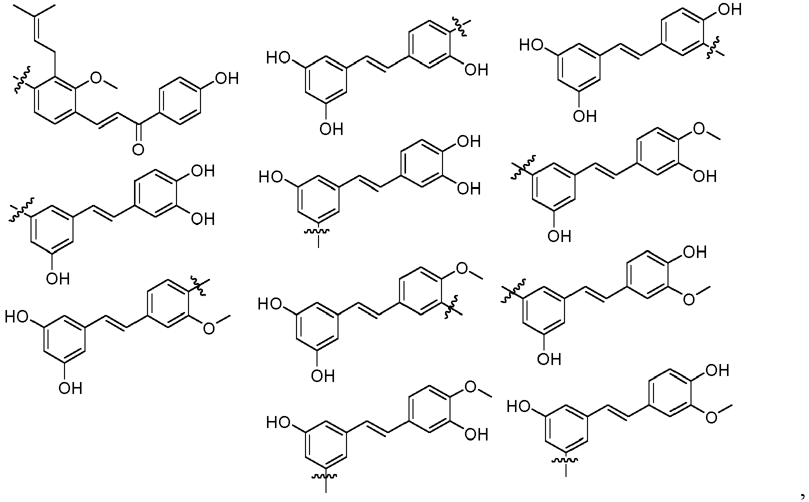 wherein each of the phenolic OH group is optionally linked to a sugar (such as glucose) via a glycoside bond.

In some embodiments, D is derived from a sterol. For example, in some embodiments, D is selected from 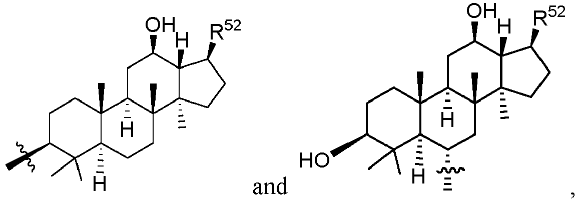
wherein R⁵² is an optionally substituted alkyl or an optionally substituted alkenyl,
wherein each of the remaining -OH groups in D is optionally linked to a sugar via a glycoside bond.

Preferably, R⁵² can be

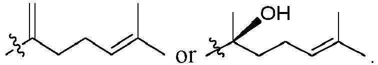

In any of the embodiments described hereinabove, the glycoside can have Formula V-1 or V-2:

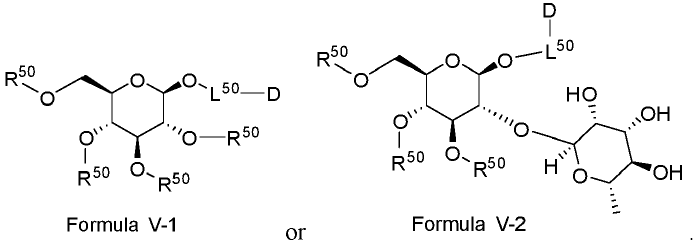

In some embodiments, the glycoside can be a compound selected from: 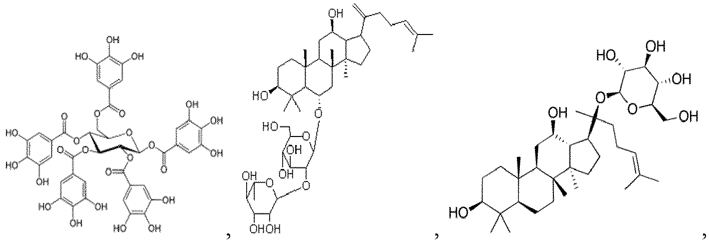 and

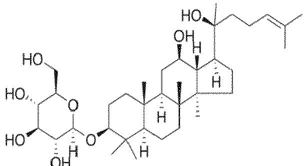

In some embodiments, the compounds herein can be any one or more of compounds selected from benzoid acid, benzyl alcohol, coumarins, catechols, polyphenols, chalconoids (including licochalcones), etc., stilbenes such as resveratrol, isoresveratrol, etc., phenolic acids, such as p-hydroxbenzoic acid, 2,4-dihydroxbenzoid acid, protocatechuic acid, gallic acid, vanillic acid, syringic acid, cinnamic acid, coumaric acids, caffeic acids, ferulic acids, chlorogenic acid, sinapic acids etc., flavonoids such as catechin, narigenin, quercetin, rutin, chrysin, etc., tannins, such as ellagic acid, and pharmaceutically acceptable salts or esters thereof and glycosides thereof.

In some embodiments, the compounds herein can be any one or more of compounds 1-13, or a pharmaceutically acceptable salt or ester thereof. 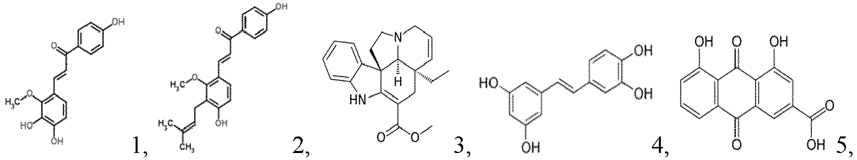 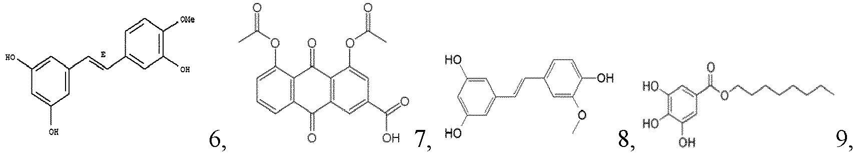 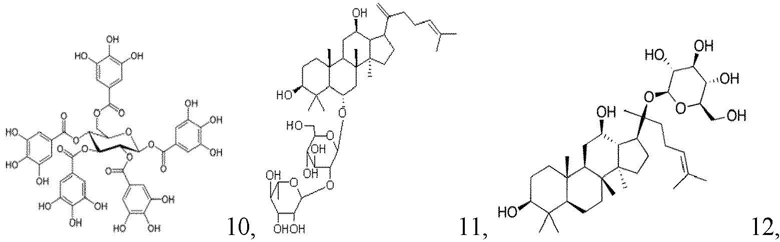 and

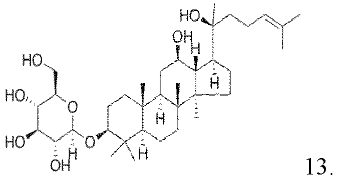

The compounds herein can be typically isolated from a natural source, or alternatively be prepared via routine chemical synthesis. For example, each of the compounds 1-13 is commercially available and has been identified as a component in a plant. Unless indicated to the contrary, in any of the embodiments described herein, the compounds can be derived from a synthetic source. Unless indicated to the contrary, in any of the embodiments described herein, the compounds can exist in an isolated form or in a substantially pure form. It should be understood that the term "isolated form" refers to a compound that has been isolated and/or enriched from its sources, such as a synthetic reaction mixture or natural sources. Typically, such isolated compounds are also substantially pure, for example, greater than 80%, 85%, 90%, 95%, or more, purity by weight. It should also be understood that a composition such as a pharmaceutical composition comprising the compound in an isolated or substantially pure form means that the compound has been isolated or purified, i.e., in an isolated or substantially pure form, prior to mixing with other ingredients of the composition.

Synthetic chemistry transformations and protecting group methodologies (protection and deprotection) useful in synthesizing applicable compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

### Pharmaceutical Compositions

Certain embodiments are directed to a pharmaceutical composition comprising one or more of the compounds of the present disclosure, and optionally a pharmaceutically acceptable excipient. In some embodiments, the pharmaceutical composition comprises a compound of the present disclosure and a pharmaceutically acceptable excipient. Pharmaceutically acceptable excipients are known in the art. Non-limiting suitable excipients include, for example, encapsulating materials or additives such as absorption accelerators, antioxidants, binders, buffers, carriers, coating agents, coloring agents, diluents, disintegrating agents, emulsifiers, extenders, fillers, flavoring agents, humectants, lubricants, perfumes, preservatives, propellants, releasing agents, sterilizing agents, sweeteners, solubilizers, wetting agents and mixtures thereof. See also Remington's The Science and Practice of Pharmacy, 21st Edition, A. R. Gennaro (Lippincott, Williams & Wilkins, Baltimore, Md., 2005), which discloses various excipients used in formulating pharmaceutical compositions and known techniques for the preparation thereof.

The pharmaceutical composition can include any one or more of the compounds of the present disclosure. For example, in some embodiments, the pharmaceutical composition comprises a compound of Formula I, II, III, IV-1, IV-2, V, any sub-formulae thereof, or any one or more of compounds 1-13, or a pharmaceutically acceptable salt or ester thereof. Unless indicated to the contrary, in any of the embodiments described herein, the pharmaceutical composition can comprise a compound selected from compounds 1-13, or a pharmaceutically acceptable salt or ester thereof. Unless indicated to the contrary, in any of the embodiments described herein, the pharmaceutical composition can also be free or substantially free of a compound selected from compounds 1-13, or a pharmaceutically acceptable salt or ester thereof.

The pharmaceutical composition can include various amounts of the compounds of the present disclosure, depending on various factors such as the intended use and potency of the compounds. In some embodiments, the pharmaceutical composition comprises a therapeutically effective amount of a compound of the present disclosure and a pharmaceutically acceptable excipient. In some embodiments, a therapeutically effective amount of a compound of the present disclosure can be an amount effective to treat AMD (e.g., wet AMD, dry AMD) as described herein, which can depend on the recipient of the treatment, the stage and the severity of the AMD, the composition containing the compound, the time of administration, the route of administration, the duration of treatment, the compound potency, its rate of clearance and whether or not another drug is co-administered. In some embodiments, a therapeutically effective amount of a compound of the present disclosure can be an amount effective to kill or inhibit the growth of a microorganism, for example, *B. megaterium,* for example, in the eye (e.g., intraocular space), blood, and/or GI tract, such as intestine of the subject. In some embodiments, a therapeutically effective amount of a compound of the present disclosure can be an amount effective to kill or inhibit the growth of a microorganism, for example, one or more selected from *Staphylococcus epidermidis*, *Pseudomonas aeruginosa*, *Staphylococcus aureus*, *Staphylococcus haemolyticus*, *Pseudomonas putida*, *Stenotrophomonas maltophilia, Bacillus cereus, Bacillus megaterium*, *Lactobacillus reuteri*, *Gardnerella vaginalis*, *Enterococcus faecium*, *Cytophaga hutchinsonii*, *Bacillus licheniformis*, or *Xanthomonas oryzae*, for example, in the eye (e.g., intraocular space), blood, and/or GI tract, such as intestine of the subject. In some embodiments, a therapeutically effective amount of a compound of the present disclosure can be an amount effective to treat soft drusen symptoms such as reduce soft drusenoid lesion.

In various embodiments, the pharmaceutical compositions described herein are useful in treating AMD and/or in killing or inhibiting the growth of a microorganism herein, for example, *B. megaterium.* The microorganisms herein are not particularly limited and are generally related to microorganisms such as bacteria found in the intraocular space in the eye of a subject, more preferably, microorganisms related to AMD such as those enriched in an AMD patient. Unless otherwise specified, in any of the embodiments described herein, the microorganism can comprise *B. megaterium.* In some embodiments, the microorganism can comprise one or more selected from *Staphylococcus epidermidis, Pseudomonas aeruginosa*, *Staphylococcus aureus*, *Staphylococcus haemolyticus*, *Pseudomonas putida*, *Stenotrophomonas maltophilia, Bacillus cereus*, *Bacillus megaterium*, *Lactobacillus reuteri*, *Gardnerella vaginalis*, *Enterococcus faecium*, *Cytophaga hutchinsonii*, *Bacillus licheniformis*, or *Xanthomonas oryzae.*

Relative amounts of the active ingredient(s), the pharmaceutically acceptable excipient, and/or any additional ingredients in a pharmaceutical composition described herein will vary, depending upon the identity, size, and/or condition of the subject treated and further depending upon the route by which the composition is to be administered.

The pharmaceutical composition described herein can be formulated for delivery via any of the known routes of delivery, which include but are not limited to oral, injectable or infusable, topical, intraocular, inhalation, etc.

In some embodiments, the pharmaceutical composition can be formulated for oral administration. The oral formulations can be presented in discrete units, such as capsules, pills, cachets, lozenges, or tablets, each containing a predetermined amount of the active compound; as a powder or granules; as a solution or a suspension in an aqueous or non-aqueous liquid; or as an oil-in-water or water-in-oil emulsion. Excipients for the preparation of compositions for oral administration are known in the art. Non-limiting suitable excipients include, for example, agar, alginic acid, aluminum hydroxide, benzyl alcohol, benzyl benzoate, 1,3-butylene glycol, carbomers, castor oil, cellulose, cellulose acetate, cocoa butter, corn starch, corn oil, cottonseed oil, cross-povidone, diglycerides, ethanol, ethyl cellulose, ethyl laureate, ethyl oleate, fatty acid esters, gelatin, germ oil, glucose, glycerol, groundnut oil, hydroxypropylmethyl cellulose, isopropanol, isotonic saline, lactose, magnesium hydroxide, magnesium stearate, malt, mannitol, monoglycerides, olive oil, peanut oil, potassium phosphate salts, potato starch, povidone, propylene glycol, Ringer's solution, safflower oil, sesame oil, sodium carboxymethyl cellulose, sodium phosphate salts, sodium lauryl sulfate, sodium sorbitol, soybean oil, stearic acids, stearyl fumarate, sucrose, surfactants, talc, tragacanth, tetrahydrofurfuryl alcohol, triglycerides, water, and mixtures thereof.

In some embodiments, the pharmaceutical composition is formulated for injection or infusion, such as intravenous injection or infusion, subcutaneous or intramuscular injection, or intraocular such as intravitreous injection. The injectable/infusable formulations can be, for example, an aqueous solution, a suspension, a depot, an implant, or an emulsion. Excipients for the preparation of inj ectable/infusable formulations are known in the art. Non-limiting suitable excipients include, for example, 1,3-butanediol, castor oil, corn oil, cottonseed oil, dextrose, germ oil, groundnut oil, liposomes, oleic acid, olive oil, peanut oil, Ringer's solution, safflower oil, sesame oil, soybean oil, U.S.P. or isotonic sodium chloride solution, water and mixtures thereof. In some embodiments, the pharmaceutical composition is formulated for intraocular administration, such as intravitreous injection.

In some embodiments, the pharmaceutical composition is formulated for topical use. Topical formulations and excipients for topical formulations are well known in the art.

Compounds of the present disclosure can be used as a monotherapy, in combination with each other, or in a combination treatment. For example, in certain embodiments, the pharmaceutical composition described herein can further include anothher antibiotic and/or an anti-VEGF medication. In some embodiments, such antibiotic and/or anti-VEGF medication can be included in a separate dosage form. In some embodiments, any of the commercially available, e.g., FDA approved, antibiotics and anti-VEGF medications can be used in combination with the compounds and compositions herein. In some embodiments, the antibiotic can be a β-lactam antibiotic, an aminoglycoside antibiotic, a tetracycline antibiotic, a chloramphenicol antibiotic, a macrolide antibiotic, a glycopeptide antibiotic, a quinolone antibiotic, a nitroimidazole antibiotic, a rifamycin antibiotic, an echinocandins antibiotic, a polyene antibiotic, a pyrimidine antibiotic, an allylamines antibiotic, or an azoles antibiotic, or a combination thereof. For example, in some embodiments, the antibiotics can include one or more of the following: β-lactam antibiotics, including penicillins (e.g., penicillin V), amoxicillin, ampicillin, bacampicillin, carbenicillin, cloxacillin, dicloxacillin, flucloxacillin, mezlocillin, nafcillin, oxacillin, penicillin G, piperacillin, pivampicillin, pivmecillinam, ticarcillin, cephalosporins such as cefacetrile, cefadroxil, cefalexin, cefaloglycin, cefalonium, cefaloridine, cefalotin, cefapirin, cefatrizine, cefazaflur, cefazedone, cefazolin, cefradine, cefroxadine, ceftezole, cefaclor, cefamandole, cefmetazole, cefonicid, cefotetan, cefoxitin, cefprozil, cefuroxime, cefuzonam, cefcapene, cefdaloxime, cefdinir, cefditoren, cefetamet, cefixime, cefmenoxime, cefodizime, cefotaxime, cefpimizole, cefpodoxime, cefteram, ceftibuten, ceftiofur, ceftiolene, ceftizoxime, ceftriaxone, cefoperazone, ceftazidime, cefclidine, cefepime, cefluprenam, cefoselis, cefozopran, cefpirome, cefquinome, ceftobiprole, ceftaroline, cefaclomezine, cefaloram, cefaparole, cefcanel, cefedrolor, cefempidone, cefetrizole, cefivitril, cefmatilen, cefmepidium, cefovecin, cefoxazole, cefrotil, cefsumide, cefuracetime, ceftioxide, thienamycins, monobactams, β-lactamase inhibitors, methoxypenicillins, etc.; Aminoglycoside antibiotics: including streptomycin, gentamicin, kanamycin (e.g., kanamycin A), tobramycin, amikacin, neomycin (e.g., neomycin B, neomycin C, neomycin E), ribomycin, micronomicin, azithromycin, dibekacin, sisomicin, netilmicin, paramomycin, bramycin, etc.; Tetracycline antibiotics: including tetracycline, oxytetracycline, chlortetracycline and doxycycline; chloramphenicol antibiotics: including chloramphenicol, thiamphenicol, etc.; macrolide antibiotics: including erythromycin, leucomycin, odorless erythromycin, acetylspiramycin, medimycin, josamycin, azithromycin, clarithromycin, dirithromycin, oxithromycin, telithromycin, etc.; glycopeptide antibiotics: including vancomycin, norvancomycin, teicoplanin, etc.; quinolone antibiotics : including norfloxacin, ofloxacin, ciprofloxacin, pefloxacin, gatifloxacin, enoxacin, lomefloxacin, nalidixic acid, levofloxacin, moxifloxacin, besifloxacin,; nitroimidazole antibiotics: including metronidazole, tinidazole, ornidazole, etc.; rifamycinoid antibiotics: including rifampicin; echinocandin antibiotics; polyene antibiotics; pyrimidines antibiotics; allylamine antibiotics; azole antibiotics; other antibiotics: fosfomycin, capreomycin, cycloserine, lincomycin, clindamycin, mitomycin, actinomycin D, bleomycin, doxorubicin, isoniazid, pyrazinamide, cyclosporine, polymyxin B combinations such as polymyxin B/trimethoprim, polymyxin B/bacitracin, polymyxin B/neomycin/gramicidin, etc.

In some embodiments, the antibiotic can be selected from Amikacin, Amoxicillin, Ampicillin, Arsphenamine, Azithromycin, Azlocillin, Aztreonam, Bacitracin, Capreomycin, Carbenicillin, Cefaclor, Cefadroxil, Cefalexin, Cefalotin, Cefamandole, Cefazolin, Cefdinir, Cefditoren, Cefixime, Cefoperazone, Cefotaxime, Cefoxitin, Cefpodoxime, Cefprozil, Ceftazidime, Ceftibuten, Ceftizoxime, Ceftriaxone, Cefuroxime, Chloramphenicol, Cilastatin, Clarithromycin, Clavulanate, Clindamycin, Clofazimine, Cloxacillin, Colistin, Cycloserine, Dalfopristin, Dapsone, Daptomycin, Dicloxacillin, Dirithromycin, Doripenem, Doxycycline, Erythromycin, Ethambutol, Ethionamide, Flucloxacillin, Fosfomycin, Furazolidone, Fusidic acid, Gentamicin, Imipenem, Isoniazid, Kanamycin, Lincomycin, Linezolid, Loracarbef, Mafenide, Meropenem, Methicillin, Metronidazole, Mezlocillin, Minocycline, Mupirocin, Nafcillin, Neomycin, Netilmicin, Nitrofurantoin, Oxacillin, Oxytetracycline, Paromomycin, Penicillin G, Penicillin V, Piperacillin, Platensimycin, Polymyxin B, Pyrazinamide, Quinupristin, Rapamycin, Rifabutin, Rifampicin, Rifampin, Rifapentine, Rifaximin, Roxithromycin, Silver sulfadiazine, Spectinomycin, Streptomycin, Sulbactam, Sulfacetamide, Sulfadiazine, Sulfamethizole, Sulfamethoxazole, Sulfanilimide, Sulfasalazine, Sulfisoxazole, Tazobactam, Teicoplanin, Telavancin, Telithromycin, Temocillin, Tetracycline, Thiamphenicol, Ticarcillin, Tigecycline, Tinidazole, Tobramycin, Trimethoprim, Troleandomycin Vancomycin, enoxacin, lomefloxacin, nalidixic acid, ciprofloxacin, levofloxacin, gatifloxacin, moxifloxacin, ofloxacin, norfloxacin, Cefotetan, Cefonicid, Cephradine, Cephapirin, Cephalothin, Cefmetazole, Cefotaxime, Moxalactam, Cefepime, Ceftaroline fosamil, Ceftobiprole, Dalbavancin, Demeclocycline, Metacycline, Ertapenem, Fidaxomicin, geldanamycin, herbimycin, Posizolid, Radezolid, Torezolid, Oritavancin, Spiramycin, Sulfadimethoxine, Sulfonamidochrysoidine, Gemifloxacin Nadifloxacin Trovafloxacin Grepafloxacin Sparfloxacin Temafloxacin, Teixobactin, Malacidins, and combinations thereof. The antibiotics can be in any form such as in the form of or in a mixture with their respective pharmaceutically acceptable salts. The antibiotics can be formulated and administered according to its known route of administration and are not particularly limited.

Anti-VEGF medications typically include biological drugs targeting VEGF, such as Ranibizumab, Aflibercept, Bevacizumab, Conbercept, etc.

### Method of Treatment

Compounds of the present disclosure are useful as therapeutic active substances for the treatment and/or prophylaxis of diseases or disorders that are associated with infections (e.g., ocular infections, such as in the intraocular space) with the microorganisms herein, such as *Bacillus megaterium.* As shown in the Examples section, representative compounds of the present disclosure show potent effect in killing or inhibiting a representative microorganism, *Bacillus megaterium* in an *in vitro* test. Further, examples show that by killing or inhibiting *Bacillus megaterium in vivo*, for example, in the macaque model described herein, antibiotics such as vancomycin were able to reduce drusenoid lesion induced by *Bacillus megaterium.*

Accordingly, in various embodiments, the present disclosure also provides a method of using the compounds of the present disclosure or the pharmaceutical compositions herein for treating infections a microorganism herein, such as *Bacillus megaterium,* and for treating or preventing diseases or disorders associated with such infections, such as AMD.

Unless otherwise specified, in any of the embodiments described herein, the infection can comprise ocular infections, such as in the intraocular space. Unless otherwise specified, in any of the embodiments described herein, the microorganism can comprise *B. megaterium.* In some embodiments, the microorganism can comprise one or more selected from *Staphylococcus epidermidis*, *Pseudomonas aeruginosa*, *Staphylococcus aureus*, *Staphylococcus haemolyticus*, *Pseudomonas putida*, *Stenotrophomonas maltophilia*, *Bacillus cereus*, *Bacillus megaterium*, *Lactobacillus reuteri*, *Gardnerella vaginalis*, *Enterococcus faecium*, *Cytophaga hutchinsonii*, *Bacillus licheniformis*, or *Xanthomonas oryzae.*

In various embodiments, compounds of the present disclosure can be used for killing or inhibiting the growth of microorganisms herein, such as *Bacillus megaterium.* In some embodiments, compounds of the present disclosure can be used for treating or preventing AMD, such as dry or wet age-related macular degeneration with drusen symptoms, including a hard drusen, a soft drusen, a mixed drusen and/or a degraded drusen, for example, dry or wet age-related macular degeneration with soft drusen symptoms. Compounds of the present disclosure can be used either alone, in combination with each other, or in combination with another antibiotic and/or an anti-VEGF medication, e.g., as described herein.

In some embodiments, the present disclosure provides a method of killing or inhibiting the growth of a microorganism herein, such as *Bacillus megaterium.* In some embodiments, the method comprises contacting the microorganism with an effective amount of a compound of the present disclosure or a pharmaceutical composition described herein. In some embodiments, the contacting can be *in vitro*, *ex vivo*, or *in vivo*.

In some embodiments, the present disclosure also provides a method for killing or inhibiting the growth of a microorganism herein, such as *Bacillus megaterium,* in a subject in need thereof. In some embodiments, the method comprises administering to the subject a compound of the present disclosure (e.g., compound of Formula I, II, III, IV-1, IV-2, V, any sub-formulae thereof, or any one or more of compounds 1-13, or a pharmaceutically acceptable salt or ester thereof. Unless indicated to the contrary, in any of the embodiments described herein, the method can comprise administering to the subject a compound selected from compounds 1-13, or a pharmaceutically acceptable salt or ester thereof. Unless indicated to the contrary, in any of the embodiments described herein, the method can also comprise administering to the subject a pharmaceutical composition that is free or substantially free of a compound selected from compounds 1-13, or a pharmaceutically acceptable salt or ester thereof. In some embodiments, the compound or pharmaceutical composition is administered in an amount effective for killing or inhibiting the growth of the microorganism in the subject, for example, in the eye (e.g., intraocular space), blood, and/or GI tract, such as intestine of the subject. In some embodiments, the subject suffers from AMD. In some embodiments, the subject does not suffer from AMD. In some embodiments, the subject is at risk of developing AMD. In some embodiments, the subject has ocular infection with the microorganism herein, such as *Bacillus megaterium.* In some embodiments, the method further comprises identifying, or having identified, the subject as being infected with, e.g., in the intraocular space, the microorganism, such as *Bacillus megaterium.* In some embodiments, the subject is further administered an antibiotic and/or an anti-VEGF medication, e.g., as described herein. In such embodiments, the antibiotic and/or anti-VEGF medication can be administered to the subject either concurrently or sequentially in any order with the compounds of the present disclosure or pharmaceutical compositions herein.

In some embodiments, the present disclosure provides a method of treating or preventing AMD in a subject in need thereof. In some embodiments, the method comprises administering to the subject a therapeutically effective amount of a compound of the present disclosure (e.g., compound of Formula I, II, III, IV-1, IV-2, V), any sub-formulae thereof, or any one or more of compounds 1-13, or a pharmaceutically acceptable salt or ester thereof. Unless indicated to the contrary, in any of the embodiments described herein, the method can comprise administering to the subject a compound selected from compounds 1-13, or a pharmaceutically acceptable salt or ester thereof. Unless indicated to the contrary, in any of the embodiments described herein, the method can also comprise administering to the subject a pharmaceutical composition that is free or substantially free of a compound selected from compounds 1-13, or a pharmaceutically acceptable salt or ester thereof. In some embodiments, the method further comprises administering to the subject an antibiotic and/or an anti-VEGF medication, e.g., as described herein. In some embodiments, the AMD can be dry or wet age-related macular degeneration with drusen symptoms, including a hard drusen, a soft drusen, a mixed drusen and/or a degraded drusen, for example, dry or wet age-related macular degeneration with soft drusen symptoms. In some embodiments, the method further comprises identifying, or having identified, the subject as being infected with, e.g., in the intraocular space, a microorganism herein, such as *Bacillus megaterium.* In some embodiments, the subject is infected with, e.g., in the intraocular space, a microorganism herein, such as *Bacillus megaterium.* In some embodiments, the method comprises administered to the subject the compound or pharmaceutical composition in an amount effective for killing or inhibiting the growth of a microorganism herein, such as *Bacillus megaterium* in the subject, for example, in the eye (e.g., intraocular space), blood, and/or GI tract, such as intestine of the subject.

The administering herein is not limited to any particular route of administration. For example, in some embodiments, the administering can be orally, nasally, topically, intraocularly, intravitreously, transdermally, pulmonary, inhalationally, buccally, sublingually, intraperintoneally, subcutaneously, intramuscularly, intravenously, rectally, intrapleurally, intrathecally and parenterally. In some embodiments, the administering can be orally, topically, intravitreously, intramuscularly, subcutaneously, or intravenously. In some embodiments, the administering is orally. In some embodiments, the administering is intravitreously.

The dosing regimen such as amounts and frequencies will vary depending on various factors such as the recipient of the treatment, the disease or disorder being treated and the severity thereof, the composition containing the compound, the time of administration, the route of administration, the duration of treatment, the compound potency, its rate of clearance and whether or not another drug is co-administered.

### Extracts

In one aspect, the present disclosure also provides an extract of certain Traditional Chinese Medicine(s) (TCMs) that have antibacterial activities. The term Traditional Chinese Medicine should be broadly construed as including both herbal and non-herbal Chinese medicinals, for example, as described in the corresponding sections of the Pharmacopoeia of the People's Republic of China (current edition). As detailed in the Examples section, various TCMs were found to have activities against a representative microorganism herein, B. *megaterium.* While some of the isolated components from these TCMs were further identified as active against *B. megaterium,* the extracts can themselves be useful for treating infections with the microorganisms herein and the associated diseases or disorders such as AMD.

Accordingly, in some embodiments, the present disclosure provides a method of treating or preventing AMD in a subject in need thereof, the method comprises administering to the subject an extract from one or more TCMs selected from Licorice *(e.g.*, *Glycyrrhiza uralensis)*, Rhubarb *(e.g., Rheum palmatum),* White Peony Root *(e.g.*, *Cynanchum otophyllum)*, Forsythia *(e.g., Forsythia suspense),* Fructus Aurantii *(e.g.*, *Citrus aurantium L.)*, Rehmannia glutinosa *(e.g.*, *Rehmannia glutinosa Libosch),* Tangerine Peel *(e.g.*, *Citrus reticulata Blanco)*, and Notoginseng *(e.g., Panax notoginseng).* In some embodiments, the AMD can be dry or wet age-related macular degeneration with drusen symptoms, including a hard drusen, a soft drusen, a mixed drusen and/or a degraded drusen, for example, dry or wet age-related macular degeneration with soft drusen symptoms. In some embodiments, the method further comprises identifying, or having identified, the subject as being infected with, e.g., in the intraocular space, a microorganism herein, such as *Bacillus megaterium.* In some embodiments, the subject is infected with, e.g., in the intraocular space, a microorganism herein, such as *Bacillus megaterium.*

In some embodiments, the present disclosure provides a method of killing or inhibiting the growth of a microorganism herein, or a method of treating an infection with a microorganism herein, such as *Bacillus megaterium,* in a subject in need thereof, the method comprises administering to the subject an extract from one or more TCMs selected from Licorice *(e.g.*, *Glycyrrhiza uralensis)*, Rhubarb *(e.g., Rheum palmatum),* White Peony Root *(e.g.*, *Cynanchum otophyllum)*, Forsythia *(e.g., Forsythia suspense),* Fructus Aurantii *(e.g.*, *Citrus aurantium L.)*, Rehmannia glutinosa *(e.g., Rehmannia glutinosa Libosch),* Tangerine Peel *(e.g.*, *Citrus reticulata Blanco)*, and Notoginseng *(e.g., Panax notoginseng).* In some embodiments, the subject suffers from AMD. In some embodiments, the subject does not suffer from AMD. In some embodiments, the subject is at risk of developing AMD. In some embodiments, the subject has ocular infection with the microorganism herein, such as *Bacillus megaterium.* In some embodiments, the method further comprises identifying, or having identified, the subject as being infected with, e.g., in the intraocular space, the microorganism, such as *Bacillus megaterium.* In some embodiments, the subject is further administered an antibiotic and/or an anti-VEGF medication, e.g., as described herein.

In some embodiments, the extract can be an extract of a single TCM. For example, in some embodiments, the method comprises administering to the subject an extract of Licorice *(e.g.*, *Glycyrrhiza uralensis).* In some embodiments, the method comprises administering to the subject an extract of Rhubarb *(e.g., Rheum palmatum).* In some embodiments, the method comprises administering to the subject an extract of White Peony Root *(e.g.*, *Cynanchum otophyllum).* In some embodiments, the method comprises administering to the subject an extract of Forsythia *(e.g., Forsythia suspense).* In some embodiments, the method comprises administering to the subject an extract of Fructus Aurantii *(e.g.*, *Citrus aurantium L.).* In some embodiments, the method comprises administering to the subject an extract of Rehmannia glutinosa *(e.g., Rehmannia glutinosa Libosch),* Tangerine Peel *(e.g.*, *Citrus reticulata Blanco).* In some embodiments, the method comprises administering to the subject an extract of Notoginseng *(e.g., Panax notoginseng*)*.*

In some embodiments, the extract can be an extract of a combination of two or more TCMs. For example, in some embodiments, the method comprises administering to the subject an extract from two or more TCMs selected from Licorice *(e.g.*, *Glycyrrhiza uralensis)*, Rhubarb *(e.g., Rheum palmatum),* White Peony Root *(e.g.*, *Cynanchum otophyllum)*, Forsythia *(e.g., Forsythia suspense),* Fructus Aurantii *(e.g.*, *Citrus aurantium L.)*, Rehmannia glutinosa *(e.g.*, *Rehmannia glutinosa Libosch)*, Tangerine Peel *(e.g.*, *Citrus reticulata Blanco)*, and Notoginseng *(e.g., Panax notoginseng*)*.* In some embodiments, the method comprises administering to the subject an extract from (a) one TCM selected from Licorice *(e.g.*, *Glycyrrhiza uralensis)*, Rhubarb *(e.g.*, *Rheum palmatum)*, White Peony Root *(e.g.*, *Cynanchum otophyllum)*, Forsythia *(e.g.*, *Forsythia suspense)*, Fructus Aurantii *(e.g.*, *Citrus aurantium L.)*, Rehmannia glutinosa *(e.g.*, *Rehmannia glutinosa Libosch),* Tangerine Peel *(e.g., Citrus reticulata Blanco)*, and Notoginseng *(e.g.*, *Panax notoginseng*); and (b) one or more other TCMs. In some embodiments, the method comprises administering to the subject an extract from (a) 1-7, but not all, TCMs, in any combination, each independently selected from *Glycyrrhiza uralensis*, *Rheum palmatum*, *Cynanchum otophyllum*, *Forsythia suspense*, *Citrus aurantium L.*, *Rehmannia glutinosa Libosch, Citrus reticulata Blanco,* and *Panax notoginseng*; and optionally (b) one or more other TCMs.

The extract herein is typically prepared according to common practice of TCMs. *See e.g.*, the Examples section. When two or more TCMs are used, the extract can be prepared by extracting each TCMs individually (or extracting any subgroup of the TCMs) and then combine the extracts; or extracting the two or more TCMs together. Typically, the extract is an aqueous extract. In some embodiments, non-aqueous extract can also be useful. It should also be noted that for some TCMs, various plant parts can be useful, such as leaf, stem, root, fruit, seed, etc. In embodiments herein, the extract is not limited to any specific part of the TCM plant, as applicable.

The extracts herein can exist or be administered in liquid, semisolid, or solid form or any other form. For example, the extracts can be administered as an aqueous solution, suspension, or emulsion. Alternatively, the extracts can also be made into a capsule, a tablet, a powder, etc. and be administered accordingly, typically orally. Administering the extract(s) can follow typical practice regarding TCMs and is not limited to a particular route of administration. Dosing regimen such as amounts and frequencies can be adjusted based on various factors such as the recipient of the treatment, the disease or disorder being treated and the severity thereof, the composition containing the extract, the time of administration, the route of administration, the duration of treatment, potency of the extract, its rate of clearance and whether or not another drug is co-administered. In some embodiments, the extract is administered in an amount effective for killing or inhibiting the growth of a microorganism herein, such as *Bacillus megaterium* in the subject, for example, in the eye (e.g., intraocular space), blood, and/or GI tract, such as intestine of the subject.

### Antibiotics

As discussed herein, the present invention is in part based on the unexpected discovery that the intraocular environment is not sterile and certain intraocular microbiota can be pathogenic causes of AMD. Thus, any antibiotics, such as those known in the art, can be useful for treating infections with the microorganisms herein and can be used for treating or preventing AMD. Accordingly, in some embodiments, the present disclosure also provides of a method of killing or inhibiting growth of a microorganism herein, such as *Bacillus megaterium*, a method of treating an infection (e.g., ocular infection, such as in the intraocular space) with a microorganism herein, and/or a method of treating or preventing a disease or disorder associated with the microorganism or infection, such as AMD, in a subject in need thereof, the method comprises administering to the subject an effective amount of an antibiotic. In some embodiments, any of the commercially available antibiotics, e.g., those approved by the U.S. FDA, can be used. In some embodiments, the antibiotics can be characterized as a broad spectrum antibiotic. In some embodiments, the antibiotics can be an antibiotic against gram-positive bacteria. In some embodiments, the subject suffers from AMD. In some embodiments, the subject does not suffer from AMD. In some embodiments, the subject is at risk of developing AMD. In some embodiments, the subject has ocular infection, e.g., with one of the microorganisms herein, such as *Bacillus megaterium.* In some embodiments, the AMD can be dry or wet age-related macular degeneration with drusen symptoms, including a hard drusen, a soft drusen, a mixed drusen and/or a degraded drusen, for example, dry or wet age-related macular degeneration with soft drusen symptoms. In some embodiments, the method further comprises identifying, or having identified, the subject as being infected with, e.g., in the intraocular space, a microorganism herein, such as *Bacillus megaterium.* In some embodiments, the subject is infected with, e.g., in the intraocular space, a microorganism herein, such as *Bacillus megaterium.* In some embodiments, the subject is further administered an anti-VEGF medication, e.g., as described herein.

Compounds of the present disclosure (see e.g., the Compounds section) typically have antibacterial activity and therefore can be an antibiotic. However, the antibiotics described in this section can be independent of the compounds of the present disclosure (e.g., as defined herein). In some embodiments, the antibiotics are also compounds of the present disclosure. In some embodiments, the antibiotics are not compounds of the present disclosure. In some embodiments, the antibiotics and the compounds of the present disclosure are used together in a combination therapy, which can be administered to a subject in need concurrently (e.g., in a single dosage form) or sequentially in any order.

In some embodiments, the antibiotic can be a β-lactam antibiotic, an aminoglycoside antibiotic, a tetracycline antibiotic, a chloramphenicol antibiotic, a macrolide antibiotic, a glycopeptide antibiotic, a quinolone antibiotic, a nitroimidazole antibiotic, a rifamycin antibiotic, an echinocandins antibiotic, a polyene antibiotic, a pyrimidine antibiotic, an allylamines antibiotic, or an azoles antibiotic, or a combination thereof.

In some embodiments, the antibiotics can include one or more of the following: β-lactam antibiotics, including penicillins (e.g., penicillin V), amoxicillin, ampicillin, bacampicillin, carbenicillin, cloxacillin, dicloxacillin, flucloxacillin, mezlocillin, nafcillin, oxacillin, penicillin G, piperacillin, pivampicillin, pivmecillinam, ticarcillin, cephalosporins such as cefacetrile, cefadroxil, cefalexin, cefaloglycin, cefalonium, cefaloridine, cefalotin, cefapirin, cefatrizine, cefazaflur, cefazedone, cefazolin, cefradine, cefroxadine, ceftezole, cefaclor, cefamandole, cefmetazole, cefonicid, cefotetan, cefoxitin, cefprozil, cefuroxime, cefuzonam, cefcapene, cefdaloxime, cefdinir, cefditoren, cefetamet, cefixime, cefmenoxime, cefodizime, cefotaxime, cefpimizole, cefpodoxime, cefteram, ceftibuten, ceftiofur, ceftiolene, ceftizoxime, ceftriaxone, cefoperazone, ceftazidime, cefclidine, cefepime, cefluprenam, cefoselis, cefozopran, cefpirome, cefquinome, ceftobiprole, ceftaroline, cefaclomezine, cefaloram, cefaparole, cefcanel, cefedrolor, cefempidone, cefetrizole, cefivitril, cefmatilen, cefmepidium, cefovecin, cefoxazole, cefrotil, cefsumide, cefuracetime, ceftioxide, thienamycins, monobactams, β-lactamase inhibitors, methoxypenicillins, etc.; Aminoglycoside antibiotics: including streptomycin, gentamicin, kanamycin (e.g., kanamycin A), tobramycin, amikacin, neomycin (e.g., neomycin B, neomycin C, neomycin E), ribomycin, micronomicin, azithromycin, dibekacin, sisomicin, netilmicin, paramomycin, bramycin, etc.; Tetracycline antibiotics: including tetracycline, oxytetracycline, chlortetracycline and doxycycline; chloramphenicol antibiotics: including chloramphenicol, thiamphenicol, etc.; macrolide antibiotics: including erythromycin, leucomycin, odorless erythromycin, acetylspiramycin, medimycin, josamycin, azithromycin, clarithromycin, dirithromycin, oxithromycin, telithromycin, etc.; glycopeptide antibiotics: including vancomycin, norvancomycin, teicoplanin, etc.; quinolone antibiotics : including norfloxacin, ofloxacin, ciprofloxacin, pefloxacin, gatifloxacin, enoxacin, lomefloxacin, nalidixic acid, levofloxacin, moxifloxacin, besifloxacin; nitroimidazole antibiotics: including metronidazole, tinidazole, ornidazole, etc.; rifamycinoid antibiotics: including rifampicin; echinocandin antibiotics; polyene antibiotics; pyrimidines antibiotics; allylamine antibiotics; azole antibiotics; other antibiotics: fosfomycin, capreomycin, cycloserine, lincomycin, clindamycin, mitomycin, actinomycin D, bleomycin, doxorubicin, isoniazid, pyrazinamide, cyclosporine, polymyxin B combinations such as polymyxin B/trimethoprim, polymyxin B/bacitracin, polymyxin B/neomycin/gramicidin, etc.

In some embodiments, the antibiotic can be selected from Amikacin, Amoxicillin, Ampicillin, Arsphenamine, Azithromycin, Azlocillin, Aztreonam, Bacitracin, Capreomycin, Carbenicillin, Cefaclor, Cefadroxil, Cefalexin, Cefalotin, Cefamandole, Cefazolin, Cefdinir, Cefditoren, Cefixime, Cefoperazone, Cefotaxime, Cefoxitin, Cefpodoxime, Cefprozil, Ceftazidime, Ceftibuten, Ceftizoxime, Ceftriaxone, Cefuroxime, Chloramphenicol, Cilastatin, Clarithromycin, Clavulanate, Clindamycin, Clofazimine, Cloxacillin, Colistin, Cycloserine, Dalfopristin, Dapsone, Daptomycin, Dicloxacillin, Dirithromycin, Doripenem, Doxycycline, Erythromycin, Ethambutol, Ethionamide, Flucloxacillin, Fosfomycin, Furazolidone, Fusidic acid, Gentamicin, Imipenem, Isoniazid, Kanamycin, Lincomycin, Linezolid, Loracarbef, Mafenide, Meropenem, Methicillin, Metronidazole, Mezlocillin, Minocycline, Mupirocin, Nafcillin, Neomycin, Netilmicin, Nitrofurantoin, Oxacillin, Oxytetracycline, Paromomycin, Penicillin G, Penicillin V, Piperacillin, Platensimycin, Polymyxin B, Pyrazinamide, Quinupristin, Rapamycin, Rifabutin, Rifampicin, Rifampin, Rifapentine, Rifaximin, Roxithromycin, Silver sulfadiazine, Spectinomycin, Streptomycin, Sulbactam, Sulfacetamide, Sulfadiazine, Sulfamethizole, Sulfamethoxazole, Sulfanilimide, Sulfasalazine, Sulfisoxazole, Tazobactam, Teicoplanin, Telavancin, Telithromycin, Temocillin, Tetracycline, Thiamphenicol, Ticarcillin, Tigecycline, Tinidazole, Tobramycin, Trimethoprim, Troleandomycin Vancomycin, enoxacin, lomefloxacin, nalidixic acid, ciprofloxacin, levofloxacin, gatifloxacin, moxifloxacin, ofloxacin, norfloxacin, Cefotetan, Cefonicid, Cephradine, Cephapirin, Cephalothin, Cefmetazole, Cefotaxime, Moxalactam, Cefepime, Ceftaroline fosamil, Ceftobiprole, Dalbavancin, Demeclocycline, Metacycline, Ertapenem, Fidaxomicin, geldanamycin, herbimycin, Posizolid, Radezolid, Torezolid, Oritavancin, Spiramycin, Sulfadimethoxine, Sulfonamidochrysoidine, Gemifloxacin Nadifloxacin Trovafloxacin Grepafloxacin Sparfloxacin Temafloxacin, Teixobactin, Malacidins, and combinations thereof.

In some embodiments, the antibiotic is administered in an amount effective for killing or inhibiting the growth of a microorganism herein, such as *Bacillus megaterium* in the subject, for example, in the eye (e.g., intraocular space), blood, and/or GI tract, such as intestine of the subject.

The antibiotics can be in any form such as in the form of or in a mixture with their respective pharmaceutically acceptable salts. The antibiotics can be formulated and administered according to its known route of administration and are not particularly limited. In some embodiments, the administering can be orally, topically, intravitreously, intramuscularly, subcutaneously, or intravenously. In some embodiments, the administering is orally. In some embodiments, the administering is intravitreously.

The dosing regimen such as amounts and frequencies will vary depending on various factors such as the recipient of the treatment, the disease or disorder being treated and the severity thereof, the composition containing the antibiotic, the time of administration, the route of administration, the duration of treatment, the potency of the antibiotic, its rate of clearance and whether or not another drug is co-administered.

### Definitions

It is meant to be understood that proper valences are maintained for all moieties and combinations thereof.

It is also meant to be understood that a specific embodiment of a variable moiety herein may be the same or different as another specific embodiment having the same identifier.

Suitable groups for the variables in compounds of Formula I, II, III, IV-1, IV-2, V, or any sub-formulae thereof, as applicable, are independently selected. The described embodiments of the present invention can be combined. Such combination is contemplated and within the scope of the present invention. For example, definitions of one of the variables can be combined with any of the definitions of any other of the variables in Formula I, II, III, IV-1, IV-2, V, or any sub-formulae thereof.

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987. The disclosure is not intended to be limited in any manner by the exemplary listing of substituents described herein.

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high performance liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The disclosure additionally encompasses compounds described herein as individual isomers substantially free of other isomers, and alternatively, as mixtures of various isomers including racemic mixtures.

As used herein, the term "compound(s) of the present disclosure" refers to any of the compounds described herein in the "Compounds" section, such as those according to Formula I, II, III, IV-1, IV-2, V, any sub-formulae thereof, or any one or more of compounds 1-13, isotopically labeled compound(s) thereof (such as a deuterated analog wherein one of the hydrogen atoms is substituted with a deuterium atom with an abundance above its natural abundance), possible stereoisomers thereof (including diastereoisomers, enantiomers, and racemic mixtures), geometric isomers thereof, tautomers thereof, conformational isomers thereof, possible zwitterions thereof, esters thereof (such as pharmaceutically acceptable esters), and/or pharmaceutically acceptable salts thereof (e.g., acid addition salt such as HCl salt or base addition salt such as Na salt). Compound(s) of the present disclosure is not limited to any particular solid state forms, for example, it can be in an amorphous form or a polymorphic form. Hydrates and solvates of the compounds of the present disclosure are considered compositions of the present disclosure, wherein the compound(s) is in association with water or solvent, respectively.

As used herein, the phrase "administration" of a compound, "administering" a compound, or other variants thereof means providing the compound or a prodrug (e.g., an ester prodrug) of the compound to the individual in need of treatment.

As used herein, the term "alkyl" as used by itself or as part of another group refers to a straight- or branched-chain aliphatic hydrocarbon. In some embodiments, the alkyl which can include one to twelve carbon atoms (i.e., C₁₋₁₂ alkyl) or the number of carbon atoms designated (i.e., a C₁ alkyl such as methyl, a C₂ alkyl such as ethyl, a C₃ alkyl such as propyl or isopropyl, etc.). In one embodiment, the alkyl group is a straight chain C₁₋₁₀ alkyl group. In another embodiment, the alkyl group is a branched chain C₃₋₁₀ alkyl group. In another embodiment, the alkyl group is a straight chain C₁₋₆ alkyl group. In another embodiment, the alkyl group is a branched chain C₃₋₆ alkyl group. In another embodiment, the alkyl group is a straight chain C₁₋₄ alkyl group. Non-limiting exemplary C₁₋₄ alkyl groups include methyl, ethyl, propyl, isopropyl, butyl, sec-butyl, tert-butyl, and iso-butyl.

As used herein, the term "cycloalkyl" as used by itself or as part of another group refers to saturated and partially unsaturated (containing one or two double bonds) cyclic aliphatic hydrocarbons containing one to three rings having from three to twelve carbon atoms (i.e., C₃₋₁₂ cycloalkyl) or the number of carbons designated. In one embodiment, the cycloalkyl group has two rings. In one embodiment, the cycloalkyl group has one ring. In another embodiment, the cycloalkyl group is a C₃₋₈ cycloalkyl group. In another embodiment, the cycloalkyl group is a C₃₋₆ cycloalkyl group. "Cycloalkyl" also includes ring systems wherein the cycloalkyl ring, as defined above, is fused with one or more aryl or heteroaryl groups wherein the point of attachment is on the cycloalkyl ring, and in such instances, the number of carbons continue to designate the number of carbons in the cycloalkyl ring system. Non-limiting exemplary cycloalkyl groups include cyclopropyl, cyclobutyl, cyclopentyl, cyclohexyl, cycloheptyl, cyclooctyl, norbornyl, decalin, adamantyl, cyclopentenyl, and cyclohexenyl.

As used herein, the term "alkenyl" as used by itself or as part of another group refers to an alkyl group as defined above containing one, two or three carbon-to-carbon double bonds. In one embodiment, the alkenyl group is a C₂₋₆ alkenyl group. In another embodiment, the alkenyl group is a C₂₋₄ alkenyl group. Non-limiting exemplary alkenyl groups include ethenyl, propenyl, isopropenyl, butenyl, sec-butenyl, pentenyl, and hexenyl.

As used herein, the term "alkynyl" as used by itself or as part of another group refers to an alkyl group as defined above containing one to three carbon-to-carbon triple bonds. In one embodiment, the alkynyl has one carbon-carbon triple bond. In one embodiment, the alkynyl group is a C₂₋₆ alkynyl group. In another embodiment, the alkynyl group is a C₂₋₄ alkynyl group. Non-limiting exemplary alkynyl groups include ethynyl, propynyl, butynyl, 2-butynyl, pentynyl, and hexynyl groups.

As used herein, the term "heteroalkyl," by itself or in combination with another term, means, unless otherwise stated, a stable straight or branched-chain alkyl group, preferably having from 2 to 14 carbons, more preferably 2 to 10 carbons in the chain, one or more of which has been replaced by a heteroatom selected from S, O, P and N, and wherein the nitrogen, phosphine, and sulfur atoms can optionally be oxidized and the nitrogen heteroatom can optionally be quaternized. The heteroatom(s) S, O, P and N may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, O-CH₃, and -O-CH₂-CH₃. Similarly, the term "heteroalkylene" by itself or as part of another substituent means a divalent radical derived from heteroalkyl, as exemplified, but not limited by, -CH₂-CH₂-O-CH₂-CH₂- and -O-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R^{"} or the like.

As used herein, the term "alkoxy" as used by itself or as part of another group refers to a radical of the formula OR^{a1}, wherein R^{a1} is an alkyl.

As used herein, the term "cycloalkoxy" as used by itself or as part of another group refers to a radical of the formula OR^{a1}, wherein R^{a1} is a cycloalkyl.

As used herein, the term "alkanoyl" as used by itself or as part of another group refers to - C(O)R^{a1}, wherein R^{a1} is hydrogen or an alkyl. For example, C₁ alkanoyl refers to -C(O)H, C₂ alkanoyl refers to -C(O)CH₃.

As used herein, the term "aryl" as used by itself or as part of another group refers to a monocyclic, bicyclic or tricyclic aromatic ring system having from six to fourteen carbon atoms (i.e., C₆₋₁₄ aryl). "Aryl" also includes ring systems wherein the aryl ring, as defined above, is fused with one or more cycloalkyl or heterocyclyl groups wherein the radical or point of attachment is on the aryl ring, and in such instances, the number of carbon atoms continue to designate the number of carbon atoms in the aryl ring system. In embodiments herein, an aryl ring can be designated as connecting to two groups, or an arylene, such as in A-Aryl-B. In such cases, the two points of attachments can be independently selected from any of the available positions.

As used herein, the term "heteroaryl" or "heteroaromatic" refers to monocyclic, bicyclic or tricyclic aromatic ring systems having 5 to 14 ring atoms (i.e., a 5- to 14-membered heteroaryl) and 1, 2, 3, or 4 heteroatoms independently chosen from oxygen, nitrogen and sulfur. In one embodiment, the heteroaryl has one heteroatom, e.g., one nitrogen. In another embodiment, the heteroaryl has 6 ring atoms, e.g., pyridyl. In one embodiment, the heteroaryl is a bicyclic heteroaryl having 8 to 10 ring atoms, e.g., a bicyclic heteroaryl having 1, 2, or 3 nitrogen ring atoms, such as quinolyl. As used herein, the term "heteroaryl" is also meant to include possible N-oxides. "Heteroaryl" includes ring systems wherein the heteroaryl ring, as defined above, is fused with one or more cycloalkyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. In embodiments herein, a heteroaryl ring can be designated as connecting to two groups, or a heteroarylene, such as in A-Heteroaryl-B. In such cases, the two points of attachments can be independently selected from any of the available positions.

As used herein, the term "heterocycle" or "heterocyclyl" as used by itself or as part of another group refers to saturated and partially unsaturated (e.g., containing one or two double bonds) cyclic groups containing one, two, or three rings having from three to fourteen ring members (i.e., a 3- to 14-membered heterocycle) and at least one heteroatom. Each heteroatom is independently selected from the group consisting of oxygen, sulfur, including sulfoxide and sulfone, and/or nitrogen atoms, which can be quaternized. The term "heterocyclyl" is meant to include cyclic ureido groups such as imidazolidinyl-2-one, cyclic amide groups such as β-lactam, γ-lactam, δ-lactam and ε-lactam, and cyclic carbamate groups such as oxazolidinyl-2-one. In one embodiment, the heterocyclyl group is a 4-, 5-, 6-, 7- or 8-membered cyclic group containing one ring and one or two oxygen and/or nitrogen atoms. The heterocyclyl can be optionally linked to the rest of the molecule through a carbon or nitrogen atom. A heterocyclyl group can either be monocyclic ("monocyclic heterocyclyl") or a fused, bridged, or spiro ring system, such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclic ring, as defined above, is fused with one or more cycloalkyl groups wherein the point of attachment is either on the cycloalkyl or heterocyclic ring, or ring systems wherein the heterocyclic ring, as defined above, is fused with one or more aryl or heteroaryl groups, wherein the point of attachment is on the heterocyclic ring, and in such instances, the number of ring members continue to designate the number of ring members in the heterocyclic ring system.

An "optionally substituted" group, such as an optionally substituted alkyl, an optionally substituted alkenyl, an optionally substituted alkynyl, an optionally substituted cycloalkyl, an optionally substituted heterocyclyl, an optionally substituted aryl, and an optionally substituted heteroaryl groups, refers to the respective group that is unsubstituted or substituted. In general, the term "substituted", means that at least one hydrogen present on a group (*e.g.*, a carbon or nitrogen atom) is replaced with a permissible substituent, e.g., a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent can be the same or different at each position. Typically, when substituted, the optionally substituted groups herein can be substituted with 1-5 substituents. Substituents can be a carbon atom substituent, a nitrogen atom substituent, an oxygen atom substituent or a sulfur atom substituent, as applicable. Two of the optional substituents can join to form an optionally substituted cycloalkyl, heterocylyl, aryl, or heteroaryl ring. Substitution can occur on any available carbon, oxygen, or nitrogen atom, and can form a spirocycle. When a bicyclic or polycyclic ring structure is designated as connected to two groups, each point of attachment can be independently selected from any available positions on any of the rings. Typically, substitution herein does not result in an O-O, O-N, S-S, S-N (except SO₂-N bond), heteroatom-halogen, heteroatom-CN bond, or -C(O)-S bond or three or more consecutive heteroatoms, with the exception of O-SO₂-O, O-SO₂-N, and N-SO₂-N, except that some of such bonds or connections may be allowed if in a stable aromatic system.

In any of the embodiments described herein, unless otherwise indicated, the "optionally substituted" non-aromatic group can be unsubstituted or substituted with 1, 2, or 3 substituents independently selected from F, Cl, -OH, oxo (as applicable), C₁₋₄ alkyl, C₁₋₄ alkoxy, C₃₋₆ cycloalkyl, C₃₋₆ cycloalkoxy, phenyl, 5 or 6 membered heteroaryl containing 1 or 2 ring heteroatoms independently selected from O, S, and N, 4-7 membered heterocyclyl containing 1 or 2 ring heteroatoms independently selected from O, S, and N, wherein each of the alkyl, alkoxy, cycloalkyl, cycloalkoxy phenyl, heteroaryl, and heterocyclyl, is optionally substituted with 1, 2, or 3 substituents independently selected from F, -OH, oxo (as applicable), C₁₋₄ alkyl and C₁₋₄ alkoxy. In any of the embodiments described herein, unless otherwise indicated, the "optionally substituted" aromatic group (including aryl and heteroaryl groups) can be unsubstituted or substituted with 1, 2, or 3 substituents independently selected from F, Cl, -OH, -CN, C₁₋₄ alkyl, C₁₋₄ alkoxy, C₃₋₆ cycloalkyl, C₃₋₆ cycloalkoxy, phenyl, 5 or 6 membered heteroaryl containing 1 or 2 ring heteroatoms independently selected from O, S, and N, 4-7 membered heterocyclyl containing 1 or 2 ring heteroatoms independently selected from O, S, and N, wherein each of the alkyl, alkoxy, cycloalkyl, cycloalkoxy phenyl, heteroaryl, and heterocyclyl, is optionally substituted with 1, 2, or 3 substituents independently selected from F, -OH, oxo (as applicable), C₁₋₄ alkyl and C₁₋₄ alkoxy.

Exemplary carbon atom substituents include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, - SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, - C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, - C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, - OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, - SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, - SC(=O)OR^{aa}, -SC(=O)R^{aa},-P(=O)(R^{aa})₂, -P(=O)(OR^{cc})₂, -OP(=O)(R^{aa})₂, -OP(=O)(OR^{cc})₂, - P(=O)(N(R^{bb})₂)₂, -OP(=O)(N(R^{bb})₂)₂, -NR^{bb}P(=O)(R^{aa})₂, -NR^{bb}P(=O)(OR^{cc})₂, - NR^{bb}P(=O)(N(R^{bb})₂)₂, -P(R^{cc})₂, -P(OR^{cc})₂, -P(R^{cc})₃⁺X⁻, -P(OR^{cc})₃⁺X⁻, -P(R^{cc})₄, -P(OR^{cc})₄, -OP(R^{cc})₂, -OP(R^{cc})₃⁺X⁻, -OP(OR^{cc})₂, -OP(OR^{cc})₃⁺X⁻, -OP(R^{cc})₄, -OP(OR^{cc})₄, -B(R^{aa})₂, - B(OR^{cc})₂, -BR^{aa}(OR^{cc}), C₁₋₁₀ alkyl, C₁₋₁₀ haloalkyl, C₂₋₁₀ alkenyl, C₂₋₁₀ alkynyl, C₃₋₁₀ cycloalkyl, 3-14 membered heterocyclyl, C₆₋₁₄ aryl, and 5-14 membered heteroaryl, wherein each alkyl, alkenyl, alkynyl, cycloalkyl, heterocyclyl, aryl, and heteroaryl is independently substituted with 0, 1, 2, 3, 4, or 5 R^{dd} groups; wherein X⁻ is a counterion;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from C₁₋₁₀ alkyl, C₁₋₁₀ haloalkyl, C₂₋₁₀ alkenyl, C₂₋₁₀ alkynyl, C₃₋₁₀ cycloalkyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, cycloalkyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, - SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, - P(=O)(R^{aa})₂, -P(=O)(OR^{cc})₂, -P(=O)(N(R^{cc})₂)₂, C₁₋₁₀ alkyl, C₁₋₁₀ haloalkyl, C₂₋₁₀ alkenyl, C₂₋₁₀ alkynyl, C₃₋₁₀ cycloalkyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, cycloalkyl, heterocyclyl, aryl, and heteroaryl is independently substituted with 0, 1, 2, 3, 4, or 5 R^{dd} groups; wherein X⁻ is a counterion;
each instance of R^{cc} is, independently, selected from hydrogen, C₁₋₁₀ alkyl, C₁₋₁₀ haloalkyl, C₂₋₁₀ alkenyl, C₂₋₁₀ alkynyl, C₃₋₁₀ cycloalkyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, cycloalkyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, -SO₂H, - SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, - C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, - NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, - OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, - C(=S)N(R^{ff})₂, -C(=O)SR^{ee}, -C(=S)SR^{ee}, -SC(=S)SR^{ee}, -P(=O)(OR^{ee})₂, -P(=O)(R^{ee})₂, - OP(=O)(R^{ee})₂, -OP(=O)(OR^{ee})₂, C₁₋₆ alkyl, C₁₋₆ haloalkyl, C₂₋₆ alkenyl, C₂₋₆ alkynyl, C₃₋₁₀ cycloalkyl, 3-10 membered heterocyclyl, C₆₋₁₀ aryl, 5-10 membered heteroaryl, wherein each alkyl, alkenyl, alkynyl, cycloalkyl, heterocyclyl, aryl, and heteroaryl is independently substituted with 0, 1, 2, 3, 4, or 5 R^{gg} groups, or two geminal R^{dd} substituents can be joined to form =O or =S; wherein X⁻ is a counterion;
each instance of R^{ee} is, independently, selected from C₁₋₆ alkyl, C₁₋₆ haloalkyl, C₂₋₆ alkenyl, C₂₋₆ alkynyl, C₃₋₁₀ cycloalkyl, C₆₋₁₀ aryl, 3-10 membered heterocyclyl, and 3-10 membered heteroaryl, wherein each alkyl, alkenyl, alkynyl, cycloalkyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ haloalkyl, C₂₋₆ alkenyl, C₂₋₆ alkynyl, C₃₋₁₀ cycloalkyl, 3-10 membered heterocyclyl, C₆₋₁₀ aryl and 5-10 membered heteroaryl, or two R^{ff} groups are joined to form a 3-14 membered heterocyclyl or 5-14 membered heteroaryl ring, wherein each alkyl, alkenyl, alkynyl, cycloalkyl, heterocyclyl, aryl, and heteroaryl is independently substituted with 0, 1, 2, 3, 4, or 5 R^{gg} groups; and
each instance of R^{gg} is, independently, halogen, -CN, -NO₂, -N₃, -SO₂H, -SO₃H, -OH, - OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, - NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), - OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)( C₁₋₆ alkyl), -N(C₁₋₆ alkyl)C(=O)( C₁₋₆ alkyl), - NHCO₂(C₁₋₆ alkyl), -NHC(=O)N(C₁₋₆ alkyl)₂, -NHC(=O)NH(C₁₋₆ alkyl), -NHC(=O)NH₂, - C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), - SO₂N(C₁₋₆ alkyl)₂, -SO₂NH(C₁₋₆ alkyl), -SO₂NH₂,-SO₂C₁₋₆, alkyl, -SO₂OC₁₋₆ alkyl, - OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)(OC₁₋₆ alkyl)₂, -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂₋₆ alkenyl, C₂₋₆ alkynyl, C₃₋₁₀ cycloalkyl, C₆₋₁₀ aryl, 3-10 membered heterocyclyl, 5-10 membered heteroaryl; or two geminal R^{gg} substituents can be joined to form =O or =S; wherein X⁻ is a counterion.

A "counterion" or "anionic counterion" is a negatively charged group associated with a positively charged group in order to maintain electronic neutrality.

"Halo" or "halogen" refers to fluorine (fluoro, -F), chlorine (chloro, -Cl), bromine (bromo, -Br), or iodine (iodo, -I).

"Acyl" refers to a moiety selected from the group consisting of -C(=O)R^{aa},-CHO, - CO₂R^{aa}, -C(=O)N(R^{bb})₂, -C(=NR^{bb})R^{aa}, -C(=NR^{bb})OR^{aa}, -C(=NR^{bb})N(R^{bb})₂, - C(=O)N^{Rbb}SO₂R^{aa}, -C(=S)N(R^{bb})₂, -C(=O)SR^{aa}, or -C(=S)SR^{aa}, wherein R^{aa} and R^{bb} are as defined herein.

Nitrogen atoms can be substituted or unsubstituted as valency permits, and include primary, secondary, tertiary, and quaternary nitrogen atoms. Exemplary nitrogen atom substituents include, but are not limited to, hydrogen, -OH, -OR^{aa}, -N(R^{cc})₂, -CN, - C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)(OR^{cc})₂, -P(=O)(R^{aa})₂,-P(=O)(N(R^{cc})₂)₂, C₁₋₁₀ alkyl, C₁₋₁₀ haloalkyl, C₂₋₁₀ alkenyl, C₂₋₁₀ alkynyl, C₃₋₁₀ cycloalkyl, 3-14 membered heterocyclyl, C₆₋₁₄ aryl, and 5-14 membered heteroaryl, or two R^{cc} groups attached to a nitrogen atom are joined to form a 3-14 membered heterocyclyl or 5-14 membered heteroaryl ring, wherein each alkyl, alkenyl, alkynyl, cycloalkyl, heterocyclyl, aryl, and heteroaryl is independently substituted with 0, 1, 2, 3, 4, or 5 R^{dd} groups, and wherein R^{aa}, R^{bb}, R^{cc}, and R^{dd} are as defined above.

In certain embodiments, the substituent present on a nitrogen atom is a nitrogen protecting group. Nitrogen protecting groups include, but are not limited to, -OH, -OR^{aa}, - N(R^{cc})₂, -C(=O)R^{aa}, -C(=O)N(R^{cc})₂, -CO₂R^{aa}, -SO₂R^{aa}, -C(=NR^{cc})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, C₁₋₁₀ alkyl, ar-C₁₋₁₀ alkyl, heteroar-C₁₋₁₀ alkyl, C₂₋₁₀ alkenyl, C₂₋₁₀ alkynyl, C₃₋₁₀ cycloalkyl, 3-14 membered heterocyclyl, C₆₋₁₄ aryl, and 5-14 membered heteroaryl groups, wherein each alkyl, alkenyl, alkynyl, cycloalkyl, heterocyclyl, aralkyl, aryl, and heteroaryl is independently substituted with 0, 1, 2, 3, 4, or 5 R^{dd} groups, and wherein R^{aa}, R^{bb}, R^{cc} and R^{dd} are as defined herein. Nitrogen protecting groups are well known in the art and include those described in detail in Protective Groups in Organic Synthesis, T. W. Greene and P. G. M. Wuts, 3rd edition, John Wiley & Sons, 1999.

Exemplary oxygen atom substituents include, but are not limited to, -R^{aa}, -C(=O)SR^{aa}, -C(=O)R^{aa}, -CO₂R^{aa}, -C(=O)N(R^{bb})₂, -C(=NR^{bb})R^{aa}, -C(=NR^{bb})OR^{aa}, - C(=NR^{bb})N(R^{bb})₂, -S(=O)R^{aa}, -SO₂R^{aa}, -Si(R^{aa})₃,-P(R^{cc})₂, -P(R^{cc})₃⁺X⁻, -P(OR^{cc})₂, -P(OR^{cc})₃⁺X⁻, -P(=O)(R^{aa})₂,-P(=O)(OR^{cc})₂, and -P(=O)(N(R^{bb})₂)₂, wherein X⁻, R^{aa}, R^{bb}, and R^{cc} are as defined herein. In certain embodiments, the oxygen atom substituent present on an oxygen atom is an oxygen protecting group. Oxygen protecting groups are well known in the art and include those described in detail in Protective Groups in Organic Synthesis, T. W. Greene and P. G. M. Wuts, 3rd edition, John Wiley & Sons, 1999.

The term "pharmaceutically acceptable salt" refers to those salts which are, within the scope of sound medical judgment, suitable for use in contact with the tissues of humans and lower animals without undue toxicity, irritation, allergic response, and the like, and are commensurate with a reasonable benefit/risk ratio. Pharmaceutically acceptable salts are well known in the art. The term "pharmaceutically acceptable ester" should be understood similarly.

As used herein, the terms "treat," "treating," "treatment," and the like refer to eliminating, reducing, or ameliorating a disease or condition, and/or symptoms associated therewith. Although not precluded, treating a disease or condition does not require that the disease, condition, or symptoms associated therewith be completely eliminated. As used herein, the terms "treat," "treating," "treatment," and the like may include "prophylactic treatment," which refers to reducing the probability of redeveloping a disease or condition, or of a recurrence of a previously-controlled disease or condition, in a subject who does not have, but is at risk of or is susceptible to, redeveloping a disease or condition or a recurrence of the disease or condition. The term "treat" and synonyms contemplate administering a therapeutically effective amount of a compound described herein to a subject in need of such treatment.

The term "inhibition", "inhibiting", or "inhibit," refer to the ability of a compound to reduce, slow, halt or prevent activity of a particular biological process (e.g., growth of a bacteria relative to vehicle).

The term "subject" (alternatively referred to herein as "patient") as used herein, refers to an animal, preferably a mammal, most preferably a human, who has been the object of treatment, observation or experiment. In some embodiments, the subject can be a vertebrate such as a dog, a cat, a horse or a monkey.

Compounds of the present disclosure can exist in isotope-labeled or - enriched form containing one or more atoms having an atomic mass or mass number different from the atomic mass or mass number most abundantly found in nature. Isotopes can be radioactive or non-radioactive isotopes. Isotopes of atoms such as hydrogen, carbon, phosphorous, sulfur, fluorine, chlorine, and iodine include, but are not limited to 2H, 3H, 13C, 14C, 15N, 180, 32P, 35S, 18F, 36Cl, and 125I. Compounds that contain other isotopes of these and/or other atoms are within the scope of this invention.

Unless expressly stated to the contrary, combinations of substituents and/or variables are allowable only if such combinations are chemically allowed and result in a stable compound. A "stable" compound is a compound that can be prepared and isolated and whose structure and properties remain or can be caused to remain essentially unchanged for a period of time sufficient to allow use of the compound for the purposes described herein (e.g., therapeutic administration to a subject).

### Examples

### Example 1. Bacillus megaterium is enriched in soft drusen from AMD patients

As detailed in WO2019080916 filed as PCT Application No. PCT/CN2018/112022, metagenomic sequencing analysis were carried out on aqueous humor specimens from 41 cataract (Cat), 20 AMD, 18 glaucoma (GLA), 9 Betch's disease (BD), 9 Vogt-Koyanagi-Harada Syndrome (VKH), and 8 endophthalmitis (EOS) patients. The results are briefly discussed below.

In brief, 14 bacterial species were identified as highly enriched in the AH of AMD patients using metagenomic analysis. See Table 1 below:

**Table 1**

| | **Bacteria Name** | **Fold change (AMD vs Cat)** | **PValue (AMD vs Cat)** | **QValue (AMD vs Cat)** | **AMD (Ave)** | **Cat (Ave)** | **Gla (Ave)** | **BD (Ave)** | **VKH (Ave)** | **EOS (Ave)** |
|---|---|---|---|---|---|---|---|---|---|---|
| High Abandance | Bacilluslicheniformis | 324.1 | 1.7E-07 | 1.7E-06 | 0.187 | 0.000 | 0.002 | 0.000 | 0.000 | 0.000 |
| | Bacillusmegaterium | 11.2 | 1.2E-05 | 6.2E-05 | 0.159 | 0.014 | 0.124 | 0.004 | 0.004 | 0.000 |
| | Pseudomonasputida | 8.3 | 2.1E-05 | 3.5E-05 | 0.530 | 0.064 | 0.087 | 0.053 | 0.053 | 0.001 |
| | Stenotrophomonasmaltophilia | 5.4 | 7.4E-08 | 2.5E-07 | 1. 159 | 0.213 | 0.537 | 0.078 | 0.071 | 0.001 |
| | Bacilluscereus | 4.6 | 4.5E-07 | 8.9E-07 | 0. 122 | 0.027 | 0.047 | 0.012 | 0.018 | 0.000 |
| | Pseudomonasaeruginosa | 1.9 | 1.3E-02 | 1.4E-02 | 0.696 | 0.375 | 0.678 | 0.059 | 0.068 | 0.000 |
| | StaphylococcusepidermidisS | 1.7 | 1.4E-01 | 1.0E+00 | 3. 130 | 1.801 | 1.054 | 1.000 | 1. 263 | 20. 668 |
| | taphylococcusaureus | 1.6 | 1.7E-01 | 1.0E+00 | 0.610 | 0.388 | 0.302 | 0.064 | 0.067 | 0.256 |
| | Staphylococcushaemolyticus | 1.5 | 1.9E-01 | 1.0E+00 | 0.149 | 0.100 | 0.090 | 0.050 | 0.042 | 0.006 |
| | | | | | | | | | | |
| Low Abandance | Xanthomonasoryzae | 73.1 | 9.2E-08 | 2.3E-07 | 0.054 | 0.001 | 0.000 | 0.000 | 0.000 | 0.000 |
| | Cytophagahutchinsonii | 18.5 | 1.4E-04 | 1.9E-04 | 0.032 | 0.002 | 0.001 | 0.000 | 0.000 | 0.000 |
| | EnterococcusfaeciumLa | 11. 4 | 1. 3E-02 | 1.3E-02 | 0.039 | 0.003 | 0.022 | 0.002 | 0.000 | 0.000 |
| | ctobacillusreuteriGar | 3.5 | 6.6E-02 | 1.0E+00 | 0.051 | 0.014 | 0.011 | 0.001 | 0.003 | |
| | dnerellavaginalis | 2.8 | 1. 3E-02 | 1.6E-02 | 0.041 | 0.015 | 0.015 | 0.019 | 0.044 | |
| | | | | | | | | | 0.013 | |
| | | | | | | | | | 0.000 | |

While *P. acnes* was found to be the most abundant microorganism in the AH of AMD patients, *Bacillus licheniformis* (*B. licheniformis*) and *Bacillus megaterium* (*B. megaterium*) were the most enriched species, among the 14 AMD-specific ones, in AMD AH specimens (Table 1). We then carried out PCR analysis to investigate whether the 14 AMD-specific bacteria could be detected in the hard or soft drusen tissues, as compared to the non-drusen retinal tissues from 6 archived ocular slides of AMD patients. Our results showed only 8 bacteria could be detected, among which *P. acnes* was the most abundant species and *B. megaterium* was the only species enriched in soft drusen. Intriguingly, the relative abundance of *P. acnes* was comparable in hard drusen, soft drusen, and dry AMD lesion tissues as compared to the non-drusen non-lesion retinal tissues. The relative abundance of *B. megaterium* was elevated by ~18 fold in soft drusen when compared to the non-drusen/non-lesion tissues. These data suggest a possible role *of B. megaterium* in drusen formation and AMD pathogenesis.

### Example 2. Bacillus megaterium induces activation of complement, pyroptosis of RPE cells in vitro and induces drusenoid lesions in macaque

Also detailed in WO2019080916 filed as PCT Application No. PCT/CN2018/112022, the present inventors have shown that *Bacillus megaterium* can induce activation of complement, pyroptosis of RPE cells *in vitro* and can induces drusenoid lesions in macaque. Briefly, the inventors found that *in vitro* infection *of B. megaterium,* but not *P. acnes,* led to secretion of active IL-1β and IL-18 by RPE cells, which suggests that infection *of B. megaterium* can lead to inflammation mediated by RPE.

Further, it was demonstrated that *B. megaterium* exists in both AH and retinal tissues. The inventors collected both AH and vitreous humor (VH) specimens from AMD patients and were able to detect *B. megaterium* DNA in both uncultured and cultured samples. The inventors further examined whether subretinal inoculation of AH and VH cultures which had *B. megaterium,* as well as the cultured single species of *B. megaterium* led to AMD like pathology in macaque. Briefly, about 20 CFU of bacteria (in 20 µl PBS) from AH, VH, and *B. megaterium* cultures were injected subretinally and PBS was used as a control. The fundus examination of macaque eye was performed before (Day 0) and after bacterial inoculation on Day 1, Day 3, Day 35 as well as Day 47. The PBS injection left only visible scar on the retina, while all bacterial inoculations led to drusenoid lesions on retinal tissues. Drusen-like nodules were also visible under the RPE layer. Fluorescence *in situ* hybridization results also located *B. megaterium* in drusenoid but not in the normal tissues post inoculation. An elevation in the expression of C5A, CFH, CASPASE1, and NLRP3 proteins was also detected in the *B. megaterium* infected drusenoid lesion and para-lesion tissues as compared to the uninfected normal retina in macaque. Taken together, the data demonstrate that infection *of B. megaterium* can activate complement system and induce drusenoid pathology *in vivo.*

### Example 3. In vitro Screening of Compounds that Can Inhibit the growth ofmicrobiota

To test whether certain Traditional Chinese Medicine (TCM) can control the growth of *Bacillus megateriumin vitro* and *in vivo,* an antibiotic sensitivity screening test in petri dishes was carried out. The sensitivity of *Bacillus megaterium* to various components from 8 different TCMs, including Licorice *(Glycyrrhiza uralensis),* Rhubarb *(Rheum palmatum),* White Peony Root *(Cynanchumotophyllum),* Forsythia *(Forsythia suspense),* Fructus *Aurantii(Citrus aurantium L.),* Rehmanniaglutinosa *(RehmanniaglutinosaLibosch),* Tangerine Peel *(Citrus reticulata Blanco),* and Notoginseng*(Panax notoginseng)* were tested. The extract from these TCMs were tested to be positive in killing or inhibiting the growth of *Bacillus megaterium.*

The screening procedure for the TCMs is shown below.

100g of TCMs were soaked in 300ml of water for approximately 30min, then boiled on fire, simmered for 20~40min, and concentrated to about 100ml.

Preparation of the bacterial growth buffer (Sigma-Aldrich, USA):Peptone 5.0 g, beef extract 3.0 g, NaCl 5.0 g, agar 15.0 g, and distilled water 1.0 L, at pH 7.0. Five miligram of MnSO4·H2O was added to the culture of Bacillus to facilitate spore formation. The buffer was placed in a pressure cooker (HIRAYAMA, HEV-50, Japan) for 30 minutes at 120°C, then cooled down to 40-50 °C .

One milliliter of TCMs solution was added to 15ml of the growth buffer, mixed and introduced into the culture dish, and let it stand in the clean bench to solidify.

100 ul of the suspension of *Bacillus* megaterium (concentration : 1 × 10⁶/ml) was added to the plate and evenly spreaded using a sterilized spreader, then placed in a 37 ° C incubator (HettCube 200, Germany) for 24h.

The growth of the flora on the plate were observed.

Eight TCMs with antibacterial function were screened by pre-experimental experiments, including Licorice, Rhubarb, White Peony Root, Forsythia, Fructus Aurantii, Rehmanniaglutinosa, Tangerine Peel and Notoginseng.

Following similar screening procedures, the various components (each contains a single chemical compound) from the 8 different TCMs were also screened. Compounds 1-13 below were found to be the most active compounds in killing or inhibiting the growth of *Bacillus megaterium.* At the tested concentration, each of Compounds 1-13 effectively killed and inhibited the growth of *Bacillus megaterium* in petri dishes. Other tested compounds did not kill or inhibit the growth of *Bacillus megaterium* in petri dishes at the tested concentration. Compounds 1-8, 10-13 are not according to the invention and are present for illustration purposes only. 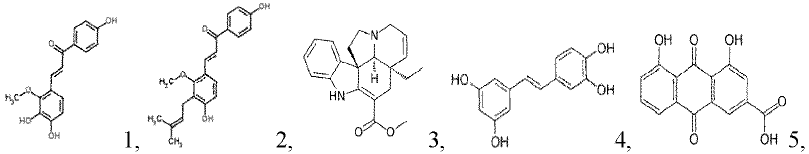 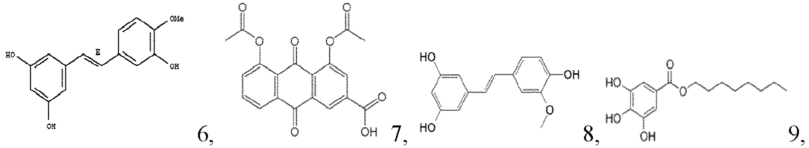 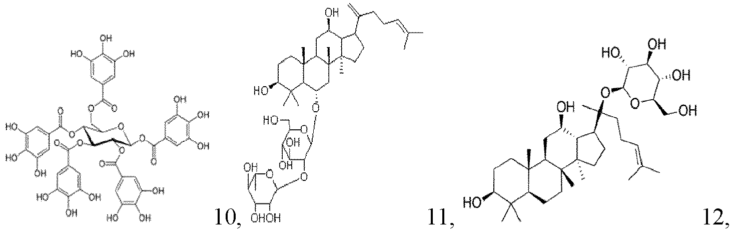 and

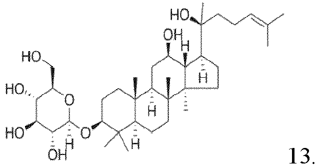

### Experimental procedure:

Dissolving components: In a clean bench, TCM components (each contains a single chemical compound) were dissolved by shaking in distilled water to a concentration of 20 g/L and stood overnight at room temperature.

Preparation of the bacterial growth buffer (Sigma-Aldrich, USA)

15ml of the buffer was plated and autoclaved for 30 minutes at 120 °C.

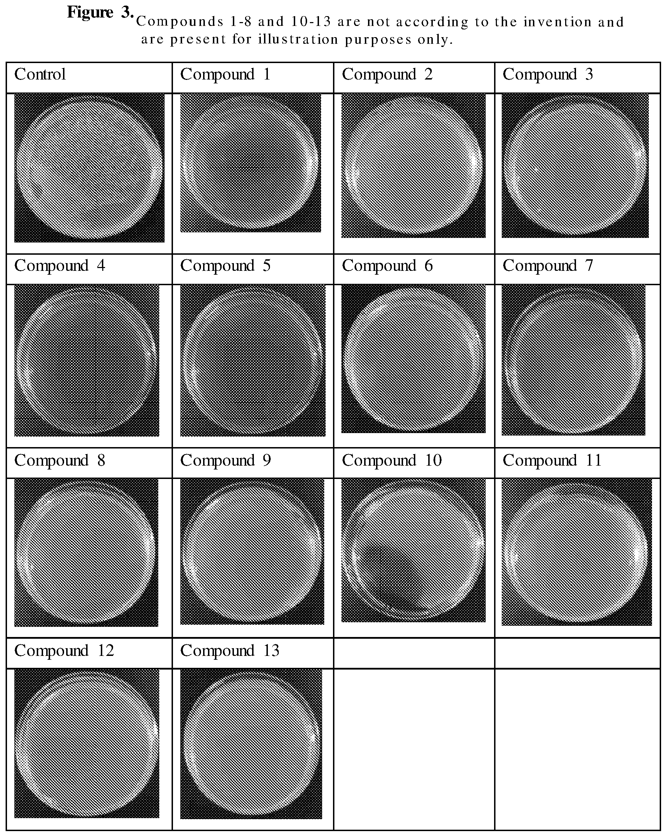

After the culture plate was sterilized and cooled to approximately 50°C, 10 µl of each component was added, and the mixture was stood and solidified. The bacteria were plated in the center and incubated at 37 ° C overnight.
The growth of the flora on the plate were observed. Figure 3 shows pictures of the plates for compounds 1-13.

## Claims

1. A therapeutically effective amount of a compound 9
or a pharmaceutically acceptable salt or ester thereof, or a pharmaceutical compositio n comprising the compound or pharmaceutically acceptable salt or ester thereof for the use in treating or preventing age-related macular degeneration (AMD), further comprising identifying or having identified the subject as being infected with a microorganism in the intraocular space, wherein the microorganism comprises Bacillus megaterium,

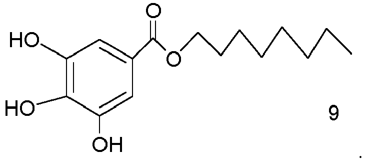

## Patentansprüche

1. Eine therapeutisch wirksame Menge einer Verbindung 9
oder eines pharmazeutisch akzeptablen Salzes oder Esters davon oder einer pharmazeutischen Zusammensetzung, die die Verbindung oder das pharmazeutisch akzeptable Salz oder den pharmazeutisch akzeptablen Ester davon beinhaltet, zur Verwendung bei der Behandlung oder Vorbeugung von altersbedingter Makuladegeneration (AMD), ferner beinhaltend das Identifizieren oder das Identifizierenlassen des Individuums als mit einem Mikroorganismus in dem intraokularen Raum infiziert, wobei der Mikroorganismus Bacillus megaterium beinhaltet,

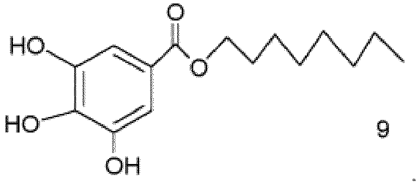

## Revendications

1. Une quantité thérapeutiquement efficace d'un composé 9
ou d'un sel ou ester pharmaceutiquement acceptables de celui-ci, ou d'une composition pharmaceutique comprenant le composé ou sel ou ester pharmaceutiquement acceptables de celui-ci pour son utilisation dans le traitement ou la prévention de la dégénérescence maculaire liée à l'âge (DMLA), comprenant en sus le fait d'identifier ou d'avoir identifié que le sujet souffre ou souffrait d'une infection par un microorganisme dans l'espace intra-oculaire, où le microorganisme comprend le Bacillus megaterium,
